# EUROPEAN PATENT SPECIFICATION

(11) **EP 3 947 344 B1**
(45) Date of publication and mention of the grant of the patent: **01.10.2025**
(21) Application number: 20714233.2
(22) Date of filing: 01.04.2020
(51) Int. Cl.: C07C 281/18, C07C 311/08, C07C 311/21, C07D 233/52, C07D 235/30, A61K 31/155, A61P 25/00

(54) **2,2'-(1,3-PHENYLENEDIMETHYLIDYNE)-BIS-HYDRAZINECARBOXIMIDAMIDE DERIVATIVES AS RETROMER STABILIZERS FOR THE TREATMENT OF NEUROLOGICAL DISEASES**
2,2'-(1,3-PHENYLENDIMETHYLIDYN)-BIS-HYDRAZINCARBOXIMIDAMIDE- DERIVATE ALS RETROMER-STABILISATOREN ZUR BEHANDLUNG VON NEUROLOGISCHEN ERKRANKUNGEN
DÉRIVÉS DE 2,2'-(1,3-PHÉNYLÈNEDIMÉTHYLIDYNE)-BIS-HYDRAZINECARBOXIMIDAMIDE EN TANT QUE STABILISATEURS DE RÉTROMÈRES POUR LE TRAITEMENT DE MALADIES NEUROLOGIQUES

(30) Priority: 01.04.2019 EP 19166534
(43) Date of publication of application: 09.02.2022
(73) Proprietor: Ospedale San Raffaele Srl, 200132 Milano (MI) (IT)
(72) Inventor: MARTINO, Gianvito, 20132 Milano MI (IT); MUZIO, Luca, 200132 Milano MI (IT); RIVA, Nilo, 200132 Milano MI (IT); GORNATI, Davide, 20010 Arluno (MI) (IT); SENECI, Pierfausto, 25015 Desenzano del Garda (BS) (IT); ELEUTERI, Simona, Carmen, 20132 Milano MI (IT)
(74) Representative: Trupiano, Federica
(86) International application number: PCT/EP2020/059207
(87) International publication number: WO 2020/201326

(56) References cited:
- WO-A1-2013/085877
- WO-A1-2018/164994
- WO-A2-96/21450
- US-A1- 2003 186 993
- YAN ZHANG ET AL: "Multivalent Dendrimers and their Differential Recognition of Short Single-Stranded DNAs of Various Length and Sequence", CHEMPLUSCHEM, vol. 83, no. 5, 2018, pages 354 - 360, XP055707535, ISSN: 2192-6506, DOI: 10.1002/cplu.201800081
- JING DENG ET AL: "Discovery of Novel Small Molecule Inhibitors of Dengue Viral NS2B-NS3 Protease Using Virtual Screening and Scaffold Hopping", JOURNAL OF MEDICINAL CHEMISTRY, vol. 55, no. 14, 2012, pages 6278 - 6293, XP055707747, ISSN: 0022-2623, DOI: 10.1021/jm300146f
- VINCENT J MECOZZI ET AL: "Pharmacological chaperones stabilize retromer to limit APP processing", NATURE CHEMICAL BIOLOGY, vol. 10, no. 6, 2014, pages 443 - 449, XP055707418, ISSN: 1552-4450, DOI: 10.1038/nchembio.1508

## Description

### Summary of the Invention

The present invention relates to novel aminoguanidine hydrazone-derivatives which are effective as retromer stabilizers and useful as neuroprotecting drugs. The invention also relates to pharmaceutical compositions comprising thereof and pharmaceutical compositions for use in therapy and diagnostic.

### Technical Background

The endocytic pathway (Huotari and Helenius 2011) includes a predominant recycling circuit for plasma membrane receptors and ligands, a degradative system for digestion of (macro) molecules and exogenous large particles, and a late endosome (LEs)-mediated feeder pathway for transport of non-sorted fluid and membrane components from the former to the latter circuit. Dysfunctional endocytic membrane trafficking in neurons is common in neurodegenerative diseases (NDDs), leading to the accumulation of dysfunctional proteins and organelles, to neuronal vulnerability and cell death (Schreij, Fon et al. 2016).

The retromer complex (Seaman 2005) is a multi-modular protein assembly involved in the retrieval of cargos from endosomes and in their delivery to the trans-Golgi network (TGN) (Seaman 2004); and in the recycling of cargos from endosomes back to the cell surface (Temkin, Lauffer et al. 2011). Its trimeric cargo recognition core (CRC) (Hierro, Rojas et al. 2007) binds to the trans-membrane proteins to be transported. CRC is made by vacuolar protein sorting-associated proteins 26 (VPS26) and VPS29, which assemble onto the VPS35 backbone.

Retromer dysfunctions in NDDs may cause abnormal processing of cargos into neurotoxic fragments (Bhalla, Vetanovetz et al. 2012), reduced protease-driven degradation of protein oligomers and aggregates in the endosomal-lysosomal system (Futerman and van Meer 2004), impaired autophagosome formation and functionality (Zavodszky, Seaman et al. 2014), increased seeding and cell-to-cell spread of intracellular neurotoxic aggregates (Walker, Diamond et al. 2013) and reduction of microglia phagocytic receptors (Lucin, O'Brien et al. 2013).

Increased retromer levels enhance cargo trafficking and transport in cells (Small, Kent et al. 2005). A stronger interaction among CRC proteins stabilizes the retromer (Norwood, Shaw et al. 2011) and should increase its functionality. An in silico screen recently targeted retromer stabilizers (Mecozzi, Berman et al. 2014) binding at the interface between VPS35 and VPS29. The thiophene-connected bis-isothiourea R55 (1) was identified as a pharmacological chaperone (Convertino, Das et al. 2016) being able to bind at the VPS35-VPS29 interface. R55 stabilizes CMC in vitro (≈10°C increase in denaturation temperature of CMC), increases VPS35 (≈180%) and VPS26 levels (≈150%) in neuronal cultures, and is not toxic up to 50µM (Mecozzi, Berman et al. 2014). R55 reduces the levels of Aβ40 (up to 44%) and Aβ42 (up to 39%) in wild type and APP-mutated hippocampal neurons (Mecozzi, Berman et al. 2014).

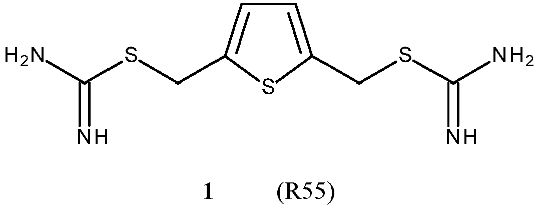

US 2003/186993 discloses a method for treating malaria by using i.a. guanylhydrazones.

WO 2018/164994 discloses a self-assembled active agent comprising i.a. combination of aminoguanidines and aldehydes, as well as a method for treating pathogens or microbes comprising administering said agents.

ChemPlusChem (2018), vol. 83, p.354-360 discloses polycationic dendrimers and their interaction with DNA and RNA leading to possible transfection and medical application, such as gene therapy.

WO 96/21450A discloses bis-guanylhydrazones and methods for reducing advanced glycosylation of proteins, therefore compounds generally useful to prevent aging.

J. Med. Chem. (2012), vol. 55, p. 6278 -6293 discloses guanylhydrazones active against Dengue Viruses.

WO 2013/085877 A discloses retromer stabilizers having a thiophene nucleus. There is an existing need for novel retromer stabilizers which are effective and which can be valuably used in the treatment and prevention of several neurological diseases, such as for instance amyotrophic lateral sclerosis (ALS), Charcot-Marie Tooth disease, Azheimer's disease (AD), Parkinson's disease (PD) and the like.

### Objects of the invention

It an object of the invention to provide novel compounds which are effective as retromer stabilizers.

It is a further object of the invention to provide the novel compounds of the invention for use in diagnostic and in therapy, particularly in the treatment of neurological diseases such as amyotrophic lateral sclerosis (ALS), Charcot-Marie Tooth disease, Azheimer's disease (AD), Parkinson's disease (PD) and the like.

It is a further object of the invention to provide pharmaceutical compositions comprising the novel compounds of the invention.

It is another object of the invention to provide a process for the preparation of the novel compounds of the invention.

### Brief description of the Figures

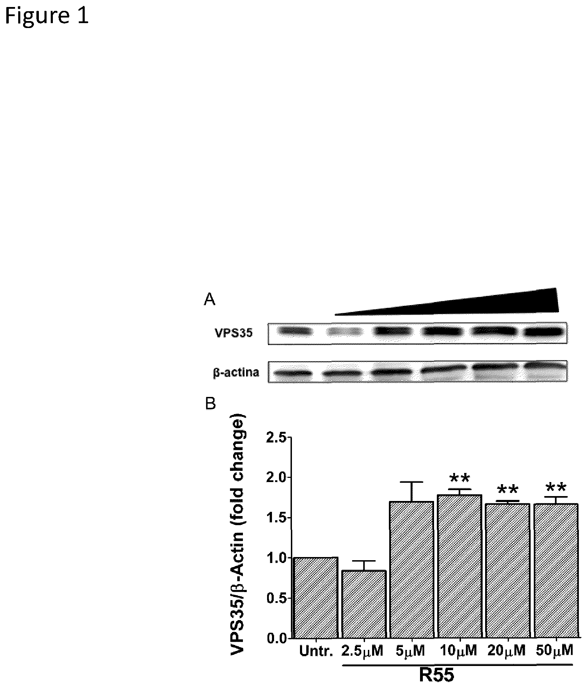

Figure 1 shows biochemical effects of R55 on VPS35 in Neuro2a cells. Histogram shows mean (± s.d.) values (n=3 for each group). **P < 0.01 determined using Student's t-test.

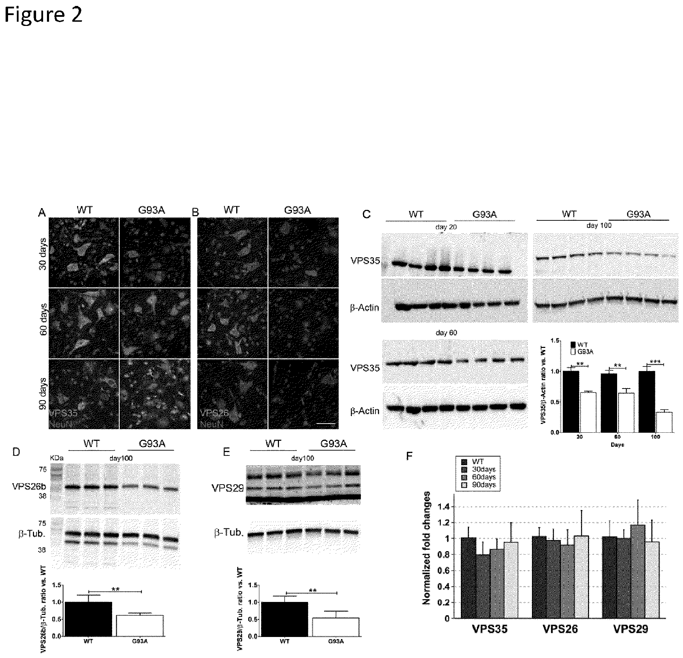

Figure 2 shows VPS35, VPS29 and VPS26 levels in G93A mice. A, maximal projections of confocal stacks of VPS35⁺NeuN⁺ putative motor neurons (MNs) in the ventral horn of lumbar SCs from WT and G93A mice (30, 60 and 90 days, n=4/time point). Adjacent sections labelled for VPS26 and NeuN are shown in B, (30, 60 and 90 days, n=4/time point). Panel C shows WBs for VPS35 and β-Actin in lumbar SCs extracts from WT and G93A mice at 20, 60 and 100 days (n=4/time point). Quantifications (means ± s.d.) of normalized VPS35 levels (ratio versus WT) are shown in the histogram of panel C. D shows a representative WB for VPS26b and β-Tubulin in SCs from WT and G93A mice at day 100. Quantification (means ± s.d.) of normalized VPS26b levels (ratio versus WT) is shown in the histogram of the panel (n=5 WT and n=6 G93A). E, WB for VPS29 levels in SCs from WT and G93A mice at day 100. Quantification (means ± s.d.) of normalized VPS29 levels (ratio versus WT) is shown in the histogram of the panel (n=6 for each group). Panels F show quantification of *Vps35, Vps26* and *Vps29* mRNAs by real time PCR (ratio versus WT) in lumbar SCs from WT (sampled at day 60, n=4) and G93A mice (30, 60 and 90 days, n=3/time point). Two way ANOVA followed by Bonferroni multiple comparisons test was used to analyze data of panel C. Two-tailed Student's test was used to determine the statistical significance in panels D and E. One way ANOVA followed by Tukey's Multiple Comparison test was used to analyze data of panels F. ** p<0.01; *** p<0.001. Scale bar in panel B, 80µm.

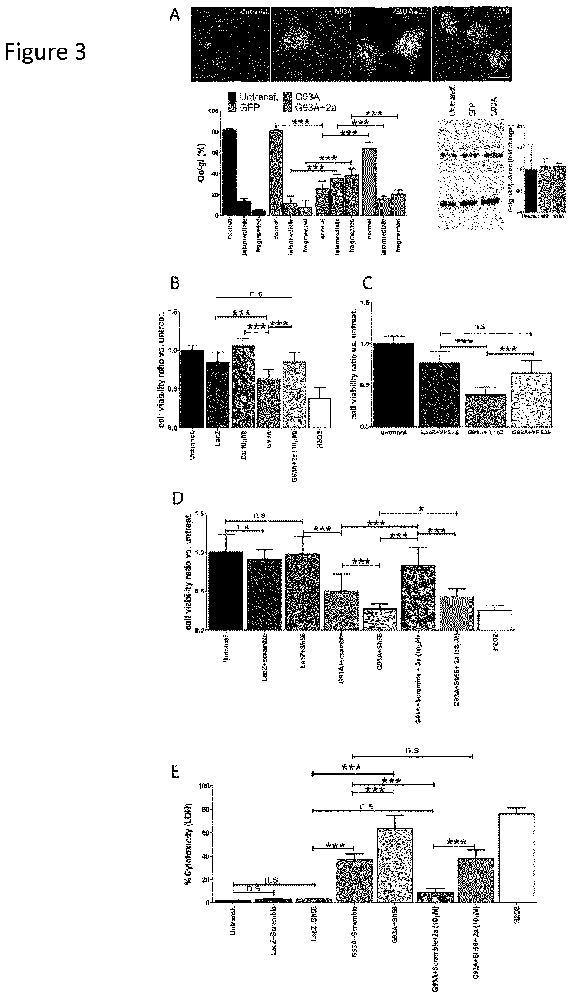

Figure 3 shows merged confocal stacks for GFP and Golgin97 in untransfected-; pcDNA3.1(+)SOD1G93A (1µg/well)-pCAAG-GFP (0.2 µg/well)-; pcDNA3.1(+)SOD1G93A (1 µg/well)-pCAAG-GFP (0.2 µg/well) + 2a (10 µM)- and pCAAG-GFP (1.2 µg/well)-transfected Neuro2a (4x10⁴ cells/well). Morphological analysis of Golgi in normal, intermediate or fragmented was done in untransfected cells (n=83), G93A-GFP (n=88), G93A-GFP+ 2a (n=84) and GFP (n=109). The histogram of panel A shows Golgi sub-classes expressed as a percent (mean ± s.d.) of the total number of Golgi constructs per condition from 4 independent experiments. Equal amounts of cell lysates from untreated Neuro2a cells (3x10⁵ cells/well) and cells transfected with pcDNA3.1(+)SOD1G93A (2 µg/well)-pCAAG-GFP (0.5 µg/well) or with pCAAG-GFP (2 µg/well) were subjected to SDS-PAGE and immunoblotted with antibodies against Golgin97 and β-Actin. Quantifications (mean fold changes± s.d. versus untreated cells) are shown in left side panel (n=3/group). B shows cell viabilities expressed as mean ratio compared to untransfected Neuro2a cells (n=19 wells) of cells treated with: pCMV-LacZ (1.25µg/well, n=19 wells), pcDNA3.1(+)SOD1G93A (1.25 µg/well, n=19 wells), pcDNA3.1(+)SOD1G93A (1.25 µg/well) + 2a (10 µM, n=24 wells), and compound 2a (10 µM, n=17 wells), (4x10⁴cells/well). Positive controls were obtained treating Neuro2a cells with H₂O₂ (200 µM, 16 wells, 4x10⁴cells/well). Data (means ± s.d.) derive from 3 independent experiments. Panel C shows cell viability (ratio versus untreated Neuro2a) of cells transfected with: pCMV-LacZ+pcCDNA3.1-VPS35 (1 µg/well + 0.25 µg/well); pcDNA3.1(+)SOD1G93A+ pCMV-LacZ (1 µg/well + 0.25µg/well) and pcDNA3.1(+)SOD1G93A+ pcCDNA3.1(+)VPS35 (1 µg/well + 0.25 µg/well), (4x10⁴ cells/well). The histogram shows means (± s.d.) derived from 3 independent experiments (n=17/group). Panel D shows cell viability (ratio versus untreated Neuro2a) of cells receiving the following conditions: pCMV-LacZ (1 µg/well) with scramble or with Sh56 plasmids (0.25 µg/well each); pcDNA3.1(+)SOD1G93A (1µg/well) with scramble or with Sh56 plasmids (0.25 µg/well) and with or without lead 2a (10 µM); (n=20/group from 3 independent experiments, 4x10⁴ cells/well). Panel E shows LDH assay in Neuro2a (4x10⁴ cells/well) receiving treatments and conditions as in experiments plotted in panel D. Cytotoxicity (mean %± s.d.) was calculated by measuring amounts of LDH released in the supernatant (n≥9 wells/group from 3 independent experiments). Two way ANOVA followed by Bonferroni multiple comparisons test was used to analyze data of panel A. One way ANOVA followed by Tukey's Multiple Comparison test was used to analyze data of panels B-E. * p<0.05, *** p<0.001, n.s. not significant. Scale bar in A, 10 µm.

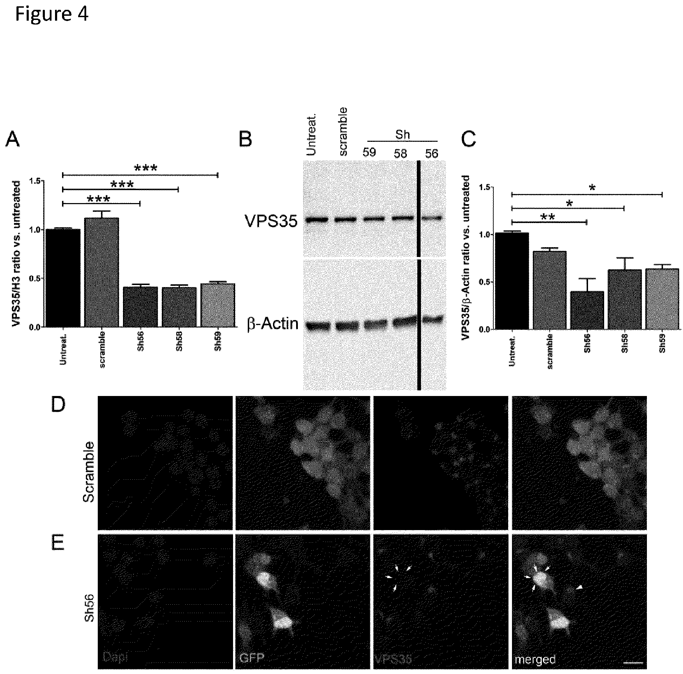

Figure 4 shows VPS35 levels in cells transfected with short interfering plasmids for VPS35. Neuro2a cells (3x10⁵cells/well) were transfected with scramble or with commercial VPS35 RNAi plasmids (Sh56, Sh58 and Sh59, 2µg/well). Knock down was assessed by real time PCR analysis of RNAs sampled 48 h after the transfection. The histogram in A shows normalized *Vps35* mRNA levels (means± s.d.) reported as ratio versus untreated cells (n=3/group). WBs were carried out to verify knockdown of VPS35 in cells receiving scramble or VPS35 RNAi plasmids. Dividing black lines in panel B indicate cropped lanes from the same filter. Quantifications of normalized VPS35 protein levels (means ±s.d.) reported as ratio versus untreated cells are shown in C (n=3/group). Parallel cultures (3x10⁵ cells/well) were transfected with pCAAG-GFP plasmids, along with scramble (D) or with Sh56 (E) plasmids, and labelled for GFP and VPS35. Arrows in E indicate VPS35 immunoreactivity in a GFP⁺ cells receiving Sh56 plasmids. The arrowhead in E indicates a GFP⁻ from the same field that express VPS35 at normal levels (n=3/group). One way ANOVA followed by Tukey's Multiple Comparison test was used to analyze data. * p<0.05, ** p<0.01, *** p<0.001.

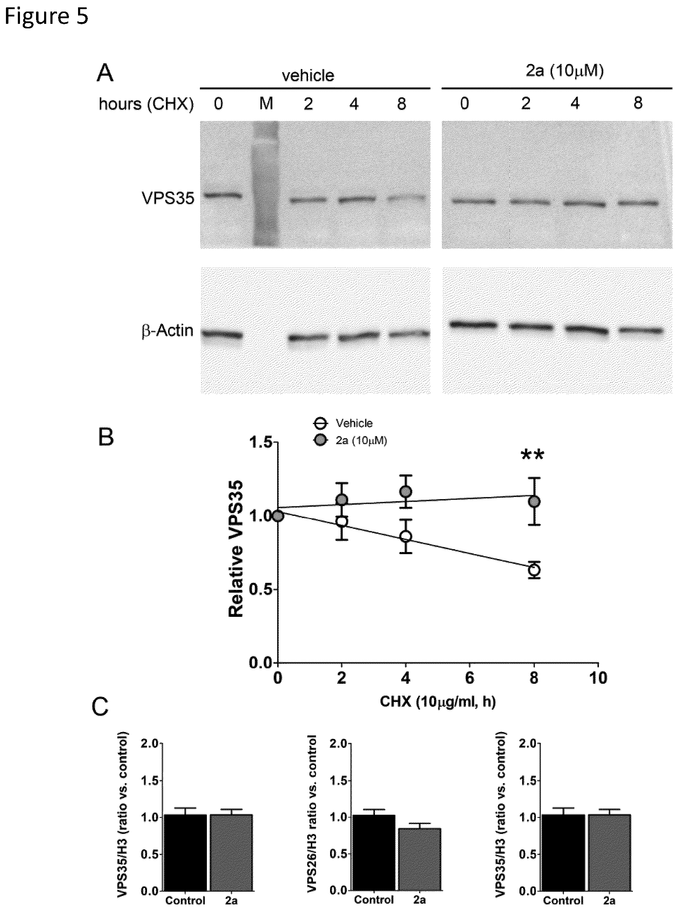

Figure 5. Compond 2a stabilizes VPS35 levels in Neuro2a cells. Panel A shows a cycloheximide (CHX) chase assay that was performed to determine rates of degradation for the VPS35 in Neuro2a cells (3x10⁵ cells/well). Cells were treated with vehicle or 2a (10 µM) for 24 h, and then chased with CHX (10 µg/mL) for 2, 4 and 8h. A shows representative WBs for VPS35 and β-Actin levels in vehicle- and compound 2a-treated Neuro2a cells. Quantifications of normalized VPS35 levels (means normalized ratios ± s.e.m. established at time 0) are shown in panel B (n=8 for each group). C, quantitative real time PCR for *Vps35, Vps26* and *Vps29* mRNA in Neuro2a cells receiving compound 2a (n=8 for each group from 3 independent experiments, 10 µM for 48h, 3x10⁵ cells/well), histograms reported ratio versus vehicle-treated cells. Two way ANOVA followed by Bonferroni multiple comparisons test was used to analyze data plotted in panel B. Two-tailed Student's test was used to determine the statistical significance in C. ** p<0.01.

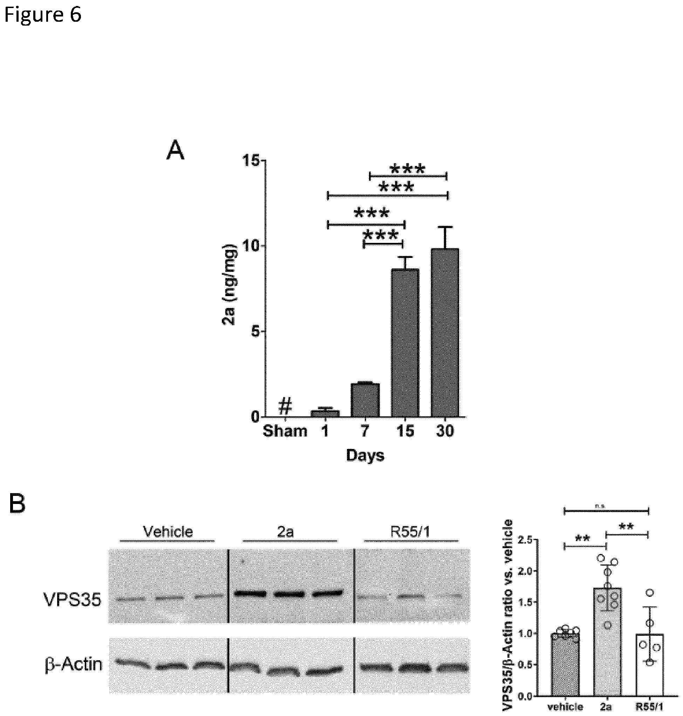

Figure 6 shows the permeability of 2a in the central nervous system. Panel_A shows a time-course of brain uptake in C57BL/6J mice injected daily with 10 mg/kg of 2a and sampled at 1, 7, 15 and 30 days (n=4 for each time point). Data are expressed as means (± s.d.) B shows a representative WB for VPS35 and β-Actin in SCs extracts from C57BL/6J mice, daily injected with: vehicle, R55/1 and 2a (10 mg/kg for 7 days). Dividing black lines mark lanes cropped from parallel filters. Quantification of normalized VPS35 levels (ratio versus vehicle-treated mice) is shown in the panel histogram (each dot represents individual mice). One way ANOVA followed by Tukey's Multiple Comparison test was used to analyze data

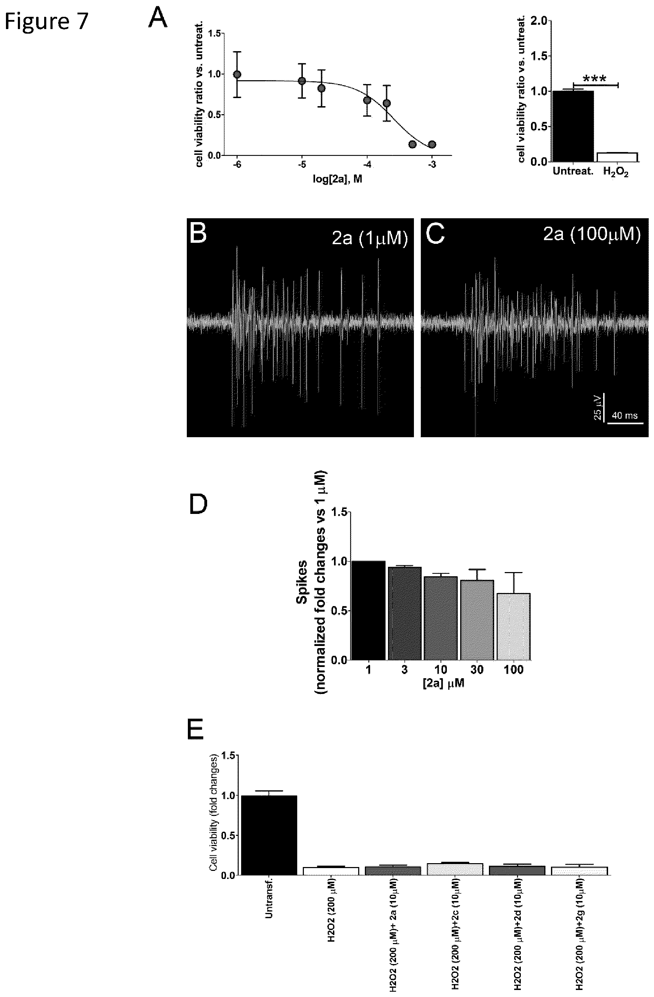

Figure 7 A shows the toxicity of 2a in Neuro2a cells and in primary neuronal cultures. Non-linear fitting analysis of cell survival indicates an LD₅₀ of 257.9 µM for the compound 2a. Data (means ± s.d.) derive from n=24 wells/group from 3 independent experiments in which Neuro2a cells received increasing amount of 2a for 48 hours. Positive controls, established treating Neuro2a cells with H₂O₂ (200 µM for 48 h, 4x10⁴ cells/well, n=24 wells from 3 independent experiments), are shown in the histogram of panel. Panels B and C show recordings of the neuronal activity from the same electrode of a representative Micro Electrode Array biochip plated with primary cortical neurons (3x10⁵ cortical neurons/chip, n=4 chips) treated with 2a, at the concentration of 1 µM (B) and 100 µM (C). Neurons received increasing amounts of 2a (1, 3, 10, 30 and 100 µM) and were recorded every 5 min. D, quantifications of the firing activity reported as fold changes of spike numbers (means ± s. e. m.) measured in neurons exposed to the low concetration of 2a (1 µM). Panel E shows that lead compounds do not revert H₂O₂-induced cell death. Histogram in the panel shows quantification of cell survival (fold changes over untreated, mean values ± s.d.) established in Neuro2a receiving H₂O₂(200µM) with or without the following compounds: 2a, 2c, 2d and 2g (10µM). Upon 48 hours cells were harvested and cell survival established (n=3 independent experiments each consisting of 6 replicates). Differences among cells receiving H₂O₂and cells receiving H₂O₂+leads are not significant. One way ANOVA followed by Tukey's Multiple Comparison test was used to analyse data.

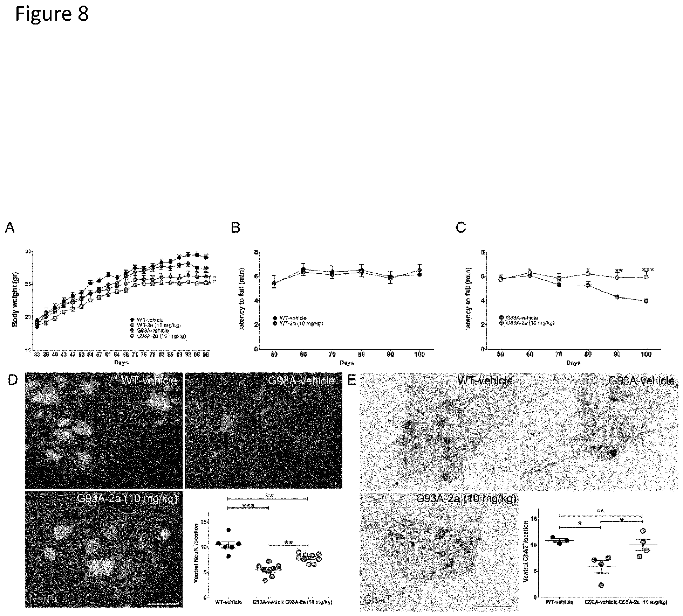

Figure 8 shows the ability of 2a to counteract motor neurons degeneration in G93A mice. Panel A shows body weight curves measured every 3 days in vehicle-treated WT mice (n=10); vehicle-treated G93A mice (n=15); 2a-treated WT mice (n=10) and 2a-treated G93A mice (n=16), (10 mg/kg, daily). Data show means ± s.e.m.. B, latency to fall on the rotating bar of the rotarod apparatus in vehicle- and lead 2a-treated WT mice (n=10 for each group). C, latency to fall in vehicle- and in lead 2a-treated G93A mice (n=15 for each group). Data are reported as means ± s.e.m.. D, lumbar SCs sections from vehicle-treated WT mice, vehicle-treated G93A mice and lead 2a treated-G93A mice (10 mg/kg, sampled at day 100) labelled for NeuN. Quantifications were done in the ventral horn of the SC scoring putative motor neurons (dots represent individual animal; lines show the mean and the s.e.m.). E, IHC for ChAT on lumbar SCs sections. Quantifications of ChAT⁺/section in the ventral horn of the SC are shown in the histogram of panel E (each dot represents a single animal; lines show the mean and the s.e.m.). Two-tailed Student's test was used to determine the statistical significance of data plotted in panel A. Two way ANOVA followed by Bonferroni multiple comparisons test was used to analyze data of panels B and C. One way ANOVA followed by Tukey's Multiple Comparison test was used to analyze data of panels D and E. * p<0.05, ** p<0.01, *** p<0.001, n.s. not significant. Scale bar 50µm.

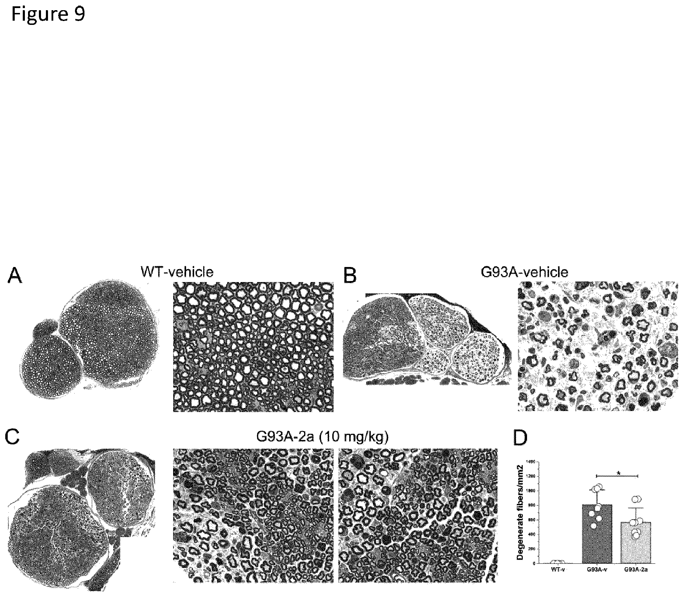

Figure 9 shows that 2a protects G93A mice from nerve degeneration. Panel A shows a representative transverse semi-thin sciatic sections that shows normal fibers in WT mice receiving vehicle (low magnification is shown on the left high magnification is shown on the right). Panel B shows images from a representative G93A mouse receiving the vehicle (arrow in high magnification picture shows a degenerating fiber). Panel C shows representative images form a G93a mouse receiving compound 2a (10 mg/kg) displaying a significant reduction of fiber degeneration. Quantifications of degenerating fibers are plotted in the histogram of panel D. Each symbol represent a single animal while the histogram shows the mean value ± s.d. (*P < 0.05 determined using one way ANOVA followed by Tukey's Multiple Comparison).

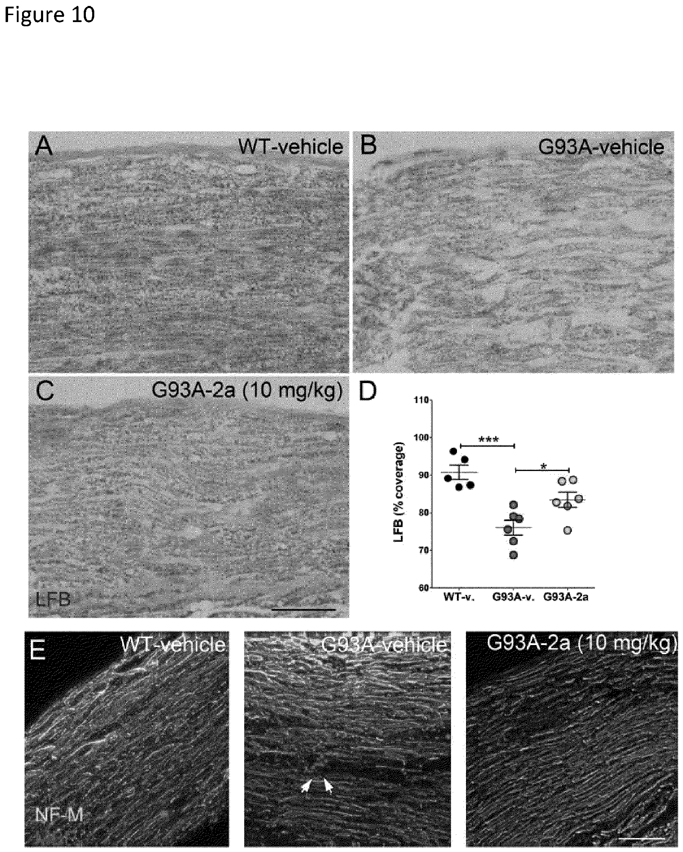

Figure 10 shows a reduction of demyelination in nerves deriving from 2a-treated G93A mice. Panels A-C shows Luxol Fast Blue (LFB) staining on sagittal sections of sciatic nerves from vehicle-treated WT (A), vehicle-treated G93A mice (B) and 2a-treated G93A mice (C) sampled at day 100 (treatment regimen: 10mg/kg daily). Quantifications of (means± s.d.) LFB covered areas are reported as ratio of vehicle-treated WT mice and shown in panel D (each dot represents an individual animal). Parallel sections labelled for NF-M and MBP are shown in panel E (≥ 5 for each group.) Arrows in vehicle-treated G93A mice show damaged myelin in a fiber from vehicle treated G93A mouse. One way ANOVA followed by Tukey's Multiple Comparison test was used to analyze data of panels D. * p<0.05, *** p<0.001. Scale bar 50µm

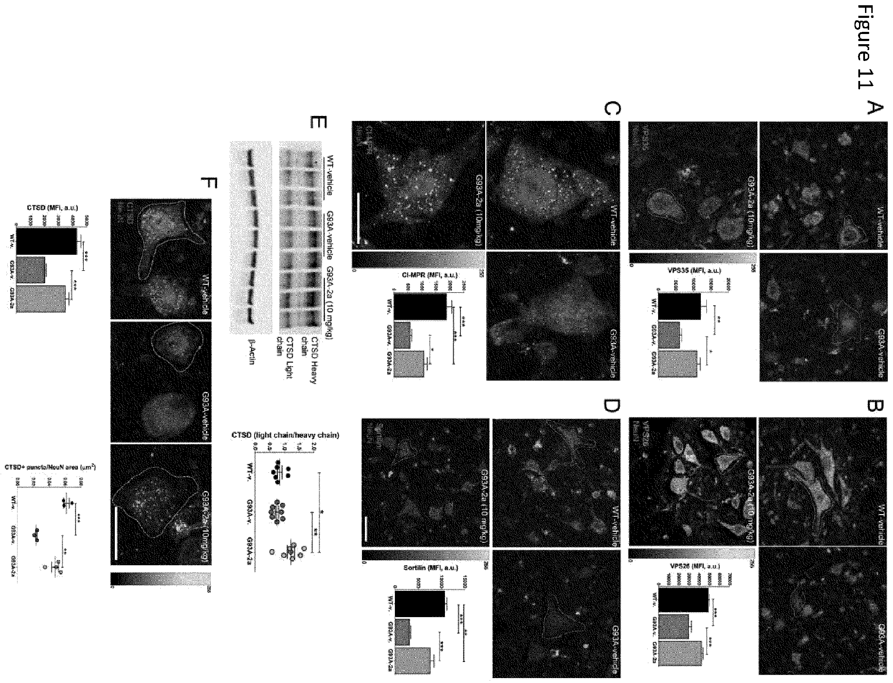

Figure 11 Panels A, B, C, D and F show maximum projections of confocal stacks from lumbar SCs (ventral horn) of vehicle-treated WT, vehicle treated-G93A and 2a (10 mg/kg, day 100)-treated G93A mice (n=3/group) labelled for: VPS35/NeuN (A), VPS26/NeuN (B), CI-MPR/NeuN (C), Sortilin/NeuN (D) and CTSD/NeuN (F). Averaged fluorescence intensities (MFI) in gated NeuN⁺ MNs (dotted lines indicate representative cells, n≥38/group), of VPS35 (Alexafluor 488) are reported as mean arbitrary units (a.u.± s.e.m.) in A. The epi-fluorescence scale is shown in the panel. Averaged MFI (a.u.± s.e.m.) of VPS26 (Alexafluor 488) quantified in gated NeuN⁺ MNs (dotted lines indicate representative cells, n≥40/group) are shown in B. The epi-fluorescence scale is shown in the panel. Averaged MFI of CI-MPR MFI (Alexafluor 488, a.u.± s.e.m.) in NeuN+ MNs (n≥35 cells/group) are shown in C. The epi-fluorescence scale is shown in the panel. Averaged Sortilin MFI levels (Alexafluor 488 a.u.± s.e.m.) quantified in gated NeuN⁺ MNs (dotted lines indicate representative cells, n≥32 cells/group) are shown in D. The epi-fluorescence scale is shown in the panel. E shows a representative WB for CTSD (heavy (46-50 KDa) and light (28-30 KDa chains). Normalization of proteins load was done by β-Actin. Histogram in E shows quantifications, dots indicate individual mice (lines show means + s.e.m.). Averaged CTSD MFI levels (Alexafluor 488, a.u.± s.e.m.) in gated NeuN⁺ MNs (dotted lines indicate representative cells) are shown in F. The epi-fluorescence scale is shown in the panel. Numbers of CTSD puncta normalized for the area of NeuN⁺ MNs are shown in the histogram of F (dots indicates single animal, n≥16 cells/group). One way ANOVA followed by Tukey's Multiple Comparison test was used to analyze data in panels A, B, C, D, E and F. * p<0.05, ** p<0.01, *** p<0.001. Scale bars 30 µm for A, B, D and F; 25 µm for C.

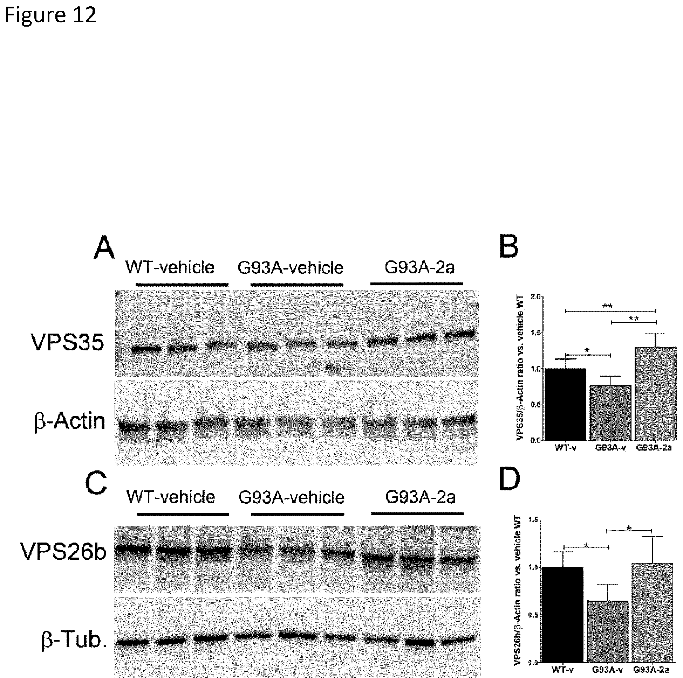

Figure 12 A shows a representative WB for VPS35 and β-Actin in SC from vehicle-treated WT mice, vehicle-treated G93A mice and 2a (10 mg/kg, sampled at day 100)-treated G93A mice (n=6/group). Quantifications of normalized VPS35 levels reported as ratio versus vehicle-treated WT (means ± s.d.) are shown in panel B. C shows a representative WB for VPS26b and β-Actin in SC lysates obtained from vehicle-treated WT mice, vehicle-treated G93A mice and 2a (10 mg/kg, sampled at day 100)-treated G93A mice. Quantifications of normalized VPS26b levels reported as ratio versus vehicle-treated WT (means ±s.d.) are shown in panel D (n=6/group). One way ANOVA followed by Tukey's Multiple Comparison test was used to analyze these data.

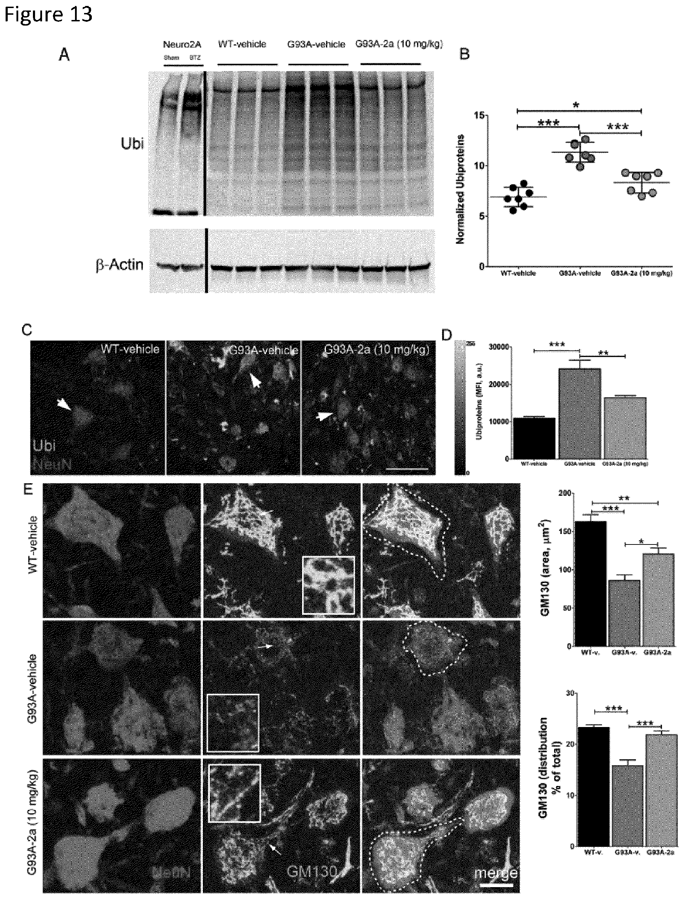

Figure 13. Panel A shows a representative WB for poly-ubiquitinated proteins in experimental controls represented by sham- and Bortezomib (10 nM, BTZ)-treated Neuro2a cells (3x10⁵ cells/well) and in lumbar SC extracts from vehicle-treated WT mice, vehicle-treated G93A mice and 2a-treated G93A mice, (10 mg/kg, day100). Normalization of proteins load was done by β-Actin. Dividing black lines in panel A show lanes cropped from independent filters. B, the collective densities of lanes, normalized for β-Actin, were used for the quantification (means ± s.d., dots represent individual animals, and data are sampled from independent filters). C, maximum projections of confocal stacks from lumbar SCs of vehicle-treated WT, vehicle treated-G93A and compound 2a-treated G93A mice (10 mg/kg, sampled at day 100) labelled for ubiquitin (Ubi) and NeuN (n=3/group). Averaged fluorescence intensities (MFI) in gated Neun⁺ MNs of Ubi (Alexafluor 488) are reported in D, (arrows indicate representative cells, ≥30 cells/group). The epi-fluorescence scale is shown in right side panel D. E, maximum projections of confocal stacks of ventral MNs labelled for NeuN and GM130 (acquisition step at the confocal microscopy: 0.4µm). Arrows in each panel indicate regions showed at high magnification in insets. Quantifications of GM130 area and distribution are shown in panel E, (n=4/group, ≥30 cells/group). One way ANOVA followed by Tukey's Multiple Comparison test was used to analyze data of panels B, D and E. * p<0.05, ** p<0.01, *** p<0.001. Scale bar 50µm in C and 20 µm in E.

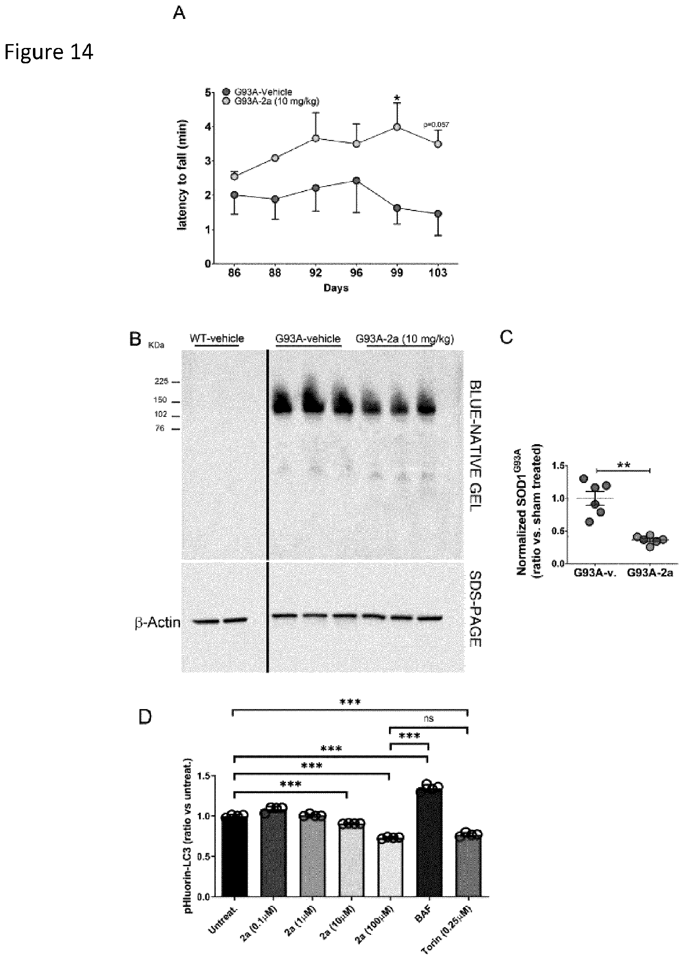

Figure 14. Panel A shows the latency to fall in vehicle- and 2a-treated G93A mice (10 mg/kg, n=6/group. Treated from day 83 to day 103). Data are reported as means ± s.d.. A representative immunoblot of a blue-native polyacrylamide gel loaded with lumbar SC extracts from vehicle- and lead 2a-treated mice is shown in B. Membranes were incubated in acetic acid to fix the proteins and probed with an anti-huSOD1 antibody. Equal amounts of protein extracts were loaded on parallel SDS-PAGE, blotted on nitrocellulose filters and probed with an anti β-Actin antibody. Dividing black lines mark lanes cropped from different filters. C, quantifications of the mutant forms of the SOD1 from mice treated with sham or with compound 2a (each dot indicates individual animals). Histogram of panel D shows normalized pHfluorin-LC3 measured in a stable Neuro2a clone (clone 7B11). Cells were treated with increasing concentration of compound 2a (0.1-100µM) for 48 hours, while controls were established using BafilomycinA1 (200nM) and Torin-1 (250nM). Cells were assayed by flow cytometry and pHfluorin LC3 fold changes were calculated over untreated cells (n=4 for each group, dots indicate single well). Two-tailed Student's test was used to determine the statistical significance in A; the Mann Whitney test was used to determine statistical significance in C. One way ANOVA followed by Tukey's Multiple Comparison test was used to to determine statistical significance in D.

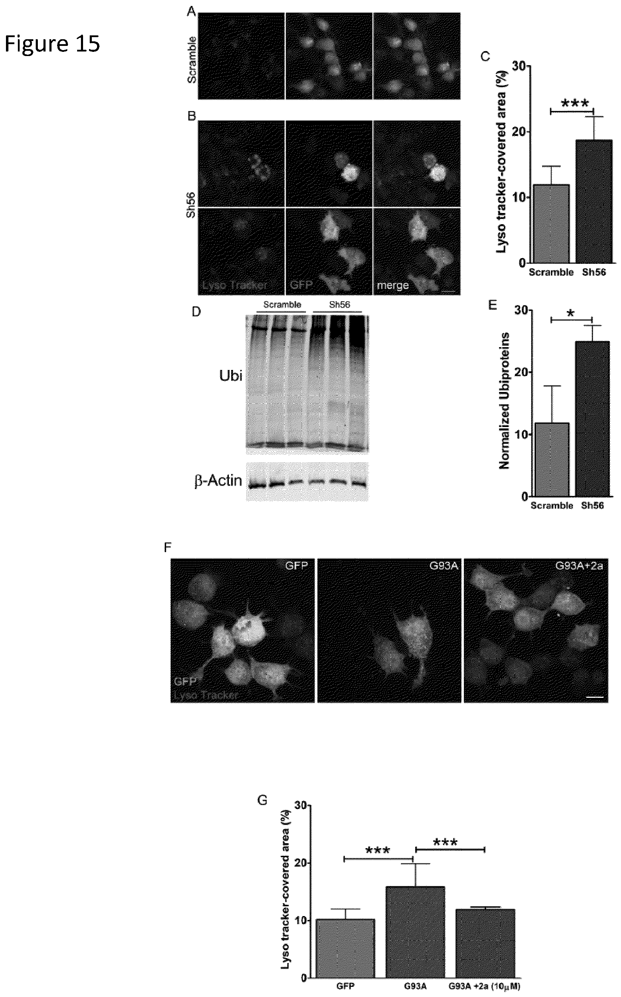

Figure 15 Neuro2a cells (4x10⁴cells/well) were transfected with the scramble plasmid (0.5 µg/well, A) or with the VPS35 RNAi plasmids (Sh56, 0.5 µg/well, B) along with plasmid encoding the GFP to identify transfected cells (0.1 µg/well). Growth medium was replaced after 24 h to induce starvation and cells were kept in FBS-free-medium for additional 24 h. Cells were incubated with Lyso-tracker (75 nM) for 30 min before imaging. Quantifications of Lyso Tracker-covered areas (mean percentages ±s.d.) in individual GFP⁺ cells were done on merged confocal images and data are shown in C (n=15 cells/group, 3 independent experiments). Protein lysates from Neuro2a cells (3x10⁵ cells/well) receiving scramble or Sh56 plasmids (2 µg/well for 48h) were blotted 48 hours after the transfection and labelled for Ubiquitin and β-Actin (D). The collective density of each Ubi-protein lane was expressed relative to β-Actin levels and mean values ± s.d. are plotted in the histogram of panel E (n=3/group). Neuro2a cells (4x10⁴ cells/well) were transfected with plasmid encoding the GFP (0.6µg/well) or with a cocktail of plasmids containing the GFP (0.1 µg/well) and pcDNA3.1(+)SOD1G93A (0.5µg/well). Upon transfection cells received 2a (10µM) or the vehicle and 24 hours later they were starved and kept in FBS-free-medium with or without 2a for additional 24 h. Cells were incubated with Lyso-tracker (75 nM) for 30 min before imaging. F, representative merged confocal images of cells labelled for the GFP and the Lyso-tracker. Quantifications of Lyso-tracker-covered areas (mean percentages ±s.d.) in each group are shown in panel G (n≥15 cells, 3 independent experiments). Two-tailed Student's test was used to determine the statistical significance in panels C and E. One way ANOVA followed by Tukey's Multiple Comparison test was used to analyze data plotted on panel G. * p<0.05; *** p<0.001. Scale bar 10µm.

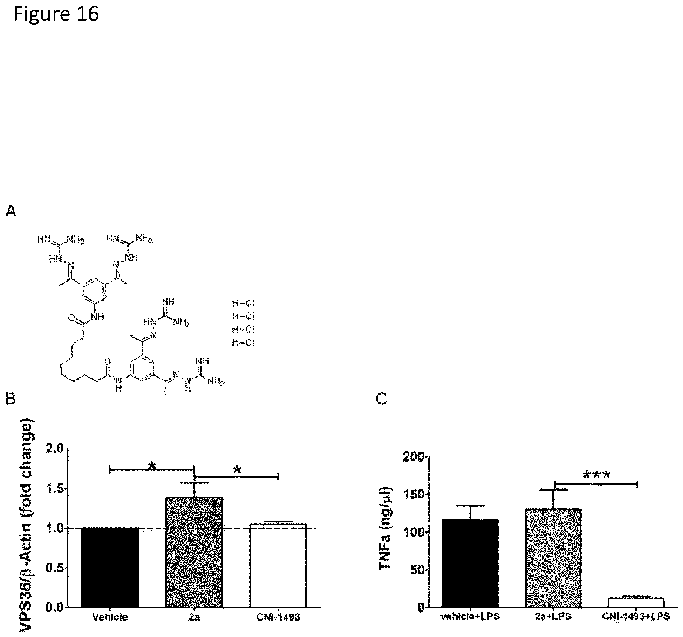

Figure 16 shows that CNI-1494 does not modulate VPS35. Panel A shows the molecular feature of CNI-1493. Panel B shows fold changes of VPS35 measured in Neuro2a were treated with CNI-1493 or DG004 for 48 hours (10µM each). C, Raw 264.7 cells were treated with CNI-1493 or 2a (10µM), 1 hour before receiving LPS (100 ng/ml). Supernatants were collected 4 hours after LPS delivery and standard ELISA was used to detect the released TNFα. Compound 2a did not inhibit TNFα release. Conversely, CNI-1493 significantly blunted TNFα expression. Data are represented as mean values (± s.d.) (n=3 for each experiment). From independent experiments and are analyzed by One way ANOVA following Tukey's multiple comparison test. * p<0.05, *** p<0.001.

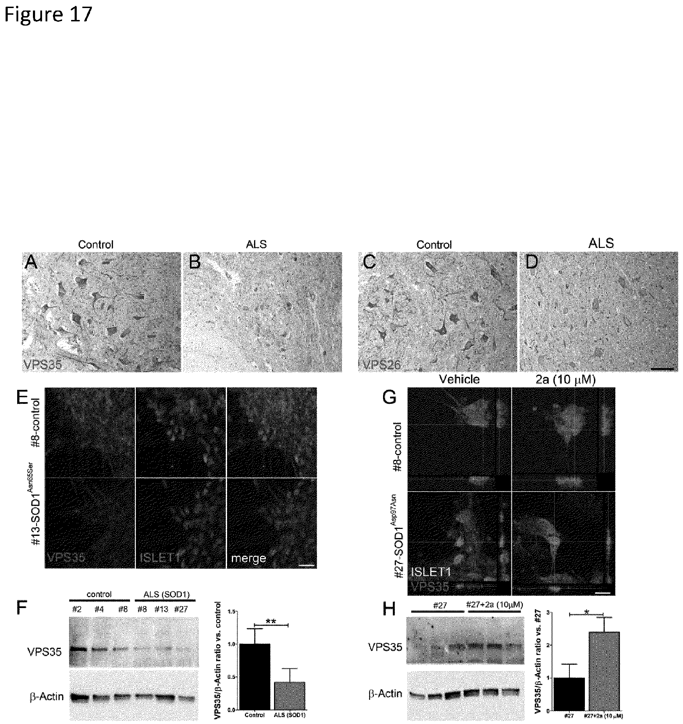

Figure 17. Panels A and B show VPS35 immunoreactivity in alpha-MNs in representative sections from ventral horns of SCs biopsies from control (A) and ALS (B). C and D show VPS26 in alpha-MNs in adjacent sections from control (C) and ALS patients (D). Immunohistochemistry for both CRC proteins was done on biopsies derived from 5 ALS patients and 4 non neurological controls. Panel E shows maximum projections of confocal stacks for VPS35 and ISLET1 in representative cultures of iPSC-derived MNs from a healthy volunteer (#8) and an ALS patient (#13, carrying the SOD1Leu144Phe mutation). WB from VPS35 and β-Actin from iPSCs-derived MN cultures (#8 SOD1Asn65Ser, #13 SOD1Leu144Phe, #27 SOD1Asp97Asn and age- and sex-matched healthy volunteers #2, #4 and #8) are shown in F. Panel G show representative cross sections from confocal stacks for VPS35 and ISLET1 established in cultured iPSCs-derived MNs from an healthy volunteer (#8) and from an ALS patient (#27 SOD1Asp97Asn) with or without lead 2a (10 µM for 6 days). Panel H shows western blots for VPS35 and β-Actin in iPSCs-derived MNs treated with vehicle or with 2a (10 µM for 6 days). Each lane represents an independent well, quantification is plotted in histogram of the panel. Two-tailed Student's test was used to determine the statistical significance in panels L and N. ** p<0.01. Scale bars: 150 µm in H, 25 µm in H', 15 µm in J, 10 µm in K 20 µm in M.

### Description of the invention

According to one of its aspects, the invention relates to a novel compound of Formula (I) 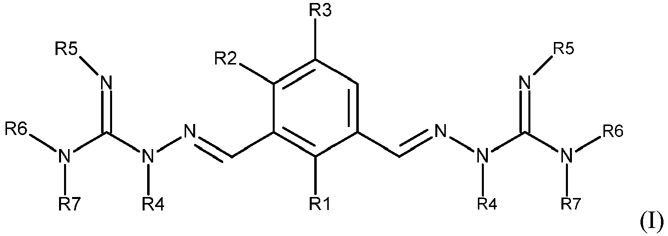 and salts thereof,
wherein:
R₁ is selected from hydrogen and halo;
R₂ is selected from hydrogen, halo, hydroxy, alkoxy and alkyl optionally substituted by a halogen atom, a hydroxy, a C₁-C₆ alkoxy, or an amino group;
R₃ is selected from hydrogen, halo, alkoxy and alkyl optionally substituted by a halogen atom, a hydroxy, a C₁-C₆ alkoxy, or an amino group, aralkyl, an optionally substituted aryl, which is optionally substituted by a halogen atom, a linear or branched C₁-C₆ alkyl, a hydroxy, a C₁-C₆ alkoxy, or an amino group, -NHCOR₈, -NHSO₂R₈,-COR₉, -NO₂, aminoguanidyl-hydrazone, -NHCOCH₃, and -NHSO₂CH₃;
R₄ is selected from hydrogen and alkyl optionally substituted by a halogen atom, a hydroxy, a C₁-C₆ alkoxy, or an amino group;
R₅ is selected from hydrogen and alkyl optionally substituted by a halogen atom, a hydroxy, a C₁-C₆ alkoxy, or an amino group;
R₆ is selected from hydrogen, alkyl optionally substituted by a halogen atom, a hydroxy, a C₁-C₆ alkoxy, or an amino group, an optionally substituted aryl, which is optionally substituted by a halogen atom, a linear or branched C₁-C₆ alkyl, a hydroxy, a C₁-C₆ alkoxy, or an amino group, and arylalkyl;
R₇ is selected from hydrogen and alkyl optionally substituted by a halogen atom, a hydroxy, a C₁-C₆ alkoxy, or an amino group;
   or
R₅ and R₆ together with the two nitrogen atoms to which they are bound, form a cycle selected from imidazolino, tetrahydropyrimidino and benzimidazolino;
   or
R₆ and R₇, each independently, together with the nitrogen atom to which they are bound, form a pyrrolidino or a piperidino group;
R₈ is selected from hydrogen, alkyl substituted by a halogen atom, a hydroxy, a C₁-C₆ alkoxy, or an amino group, an optionally substituted aryl, which is optionally substituted by a halogen atom, a linear or branched C₁-C₆ alkyl, a hydroxy, a C₁-C₆ alkoxy, or an amino group;
R₉ is selected from groups of Formula (IIIa), (IIIb), (IIIc), (IIId) and (IIIe) 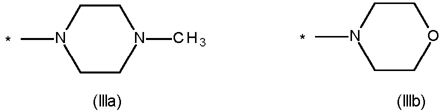 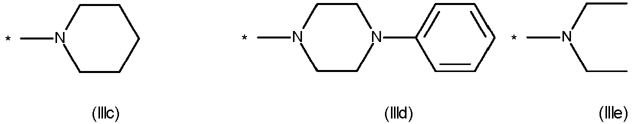
wherein the asterisk (*) indicates the linking bond,
as well as compounds of formula (I) wherein
   - R₃ is acetamido and R₁, R₂, R₄, R₅, R₆, R₇ are all hydrogen (Compound 2l)
   - R₃ is mesylamido and R₁, R₂, R₄, R₅, R₆, R₇ are all hydrogen (Compound 2n) and
   - R₃ is carbomethoxy and R₁, R₂, R₄, R₅, R₆, R₇ are all hydrogen (Compound 2p); provided that said compound of formula (I) is not one of the following:
   - R₁, R₂, R₃, R₄, R₅, R₆, R₇ are all hydrogen (Compound 2a)
   - R₂ is hydroxyl and R₁, R₃, R₄, R₅, R₆, R₇ are all hydrogen (Compound 2d)
   - R₃ is bromine and R₁, R₂, R₄, R₅, R₆, R₇ are all hydrogen (Compound 2h)
      - R3 is guanidinoiminomethyl and R1, R2, R4, R5, R6, R7 are all hydrogen (Compound 2r)
      - R5 and R6 together with the nitrogen atom to which they are bond form a imidazolidino group and R1, R2, R3, R4, R7 are all hydrogen (Compound 2x)
R5 and R6 together with the nitrogen atoms to which they are bond form a benzimidazolidino group and R1, R2, R3, R4, R7 are all hydrogen (Compound 2z) and its salts and solvates.

The term "halo" herein indicates any of any of the halogen atoms, preferably chlorine and bromine, advantageously bromine.

The term "alkyl" herein indicates a linear or branched, saturated C₁-C₆ alkyl, preferably C₁-C₄ alkyl, such as methyl, ethyl, n-propyl, i-propyl and n-butyl, i-butyl, sec-butyland tert-butyl. The alkyl group may be optionally substituted by a halogen atom, a hydroxy, a C₁-C₆ alkoxy, or an amino group.

The term "alcoxy" herein indicates a linear or branched, saturated C₁-C₆ alkoxy, preferably C₁-C₄ alkoxy, such as methoxy, ethoxy, propoxy and butoxy groups.

Said "optionally substituted aryl group" is preferably selected from phenyl and tolyl, such as p-tolyl.

The term "arylalkyl" herein indicates an aryl, preferably a monocyclic aryl, group which is bound to Formula (I) by an alkyl residue, preferably a methylene; a preferred arylalkyl group is benzyl.

According to a preferred embodiment, in the compound of Formula (I)
R₁ is selected from hydrogen and bromine;
R₂ is selected from hydrogen, bromine, hydroxy, methoxy, methyl, n-butyl and n-butyloxy;
R₃ is selected from hydrogen, bromine, methoxy, -NO₂, phenyl, -NHCOCH₃, -NHCO-p-tolyl, -NHSO₂CH₃, -NHSO₂-p-tolyl, -CO-R₉ and aminoguanidyl-hydrazone;
R₄ is selected from hydrogen and methyl;
R₅ is hydrogen;
R₆ is selected from hydrogen, methyl, n-butyl and benzyl;
R₇ is hydrogen,
   or
R₅ and R₆, together with the two nitrogen atoms to which they are bound, form a cycle selected from imidazolino, tetrahydropyrimidino and benzimidazolino;
   or
R₆ and R₇, together with the nitrogen atom to which they are bound, form a pyrrolidino or a piperidino group;
R₉ is selected from groups of Formula (IIIa), (IIIb), (IIIc), (IIId) and (IIIe) 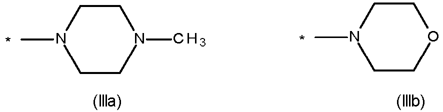 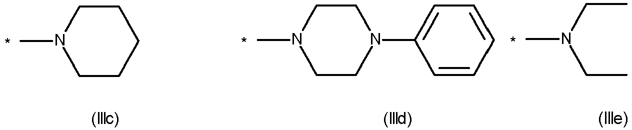
wherein the asterisk (*) indicates the linking bond.

Accoring to another of its aspects, the invention relates to a compound of Formula (I) 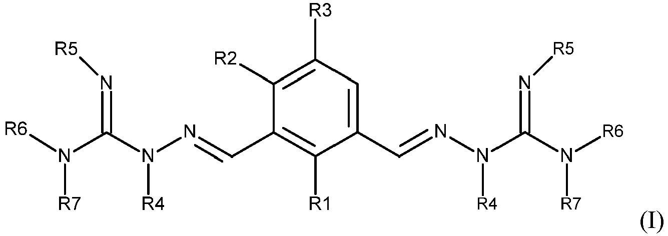 and its pharmaceutically acceptable salts and solvates,
wherein:
R₁ is selected from hydrogen and halo;
R₂ is selected from hydrogen, halo, hydroxy, alkoxy and alkyl;
R₃ is selected from hydrogen, halo, hydroxy, alkoxy and alkyl, aralkyl, aryl, -COOR₈, -NHCOR₈, -NHSO₂R₈, -COR₉, -NO₂, aminoguanidyl-hydrazone, -NHCOCH₃ and -NHSO₂CH₃;
R₄ is selected from hydrogen and alkyl;
R₅ is selected from hydrogen and alkyl;
R₆ is selected from hydrogen, alkyl, aryl and arylalkyl;
R₇ is selected from hydrogen and alkyl;
   or
R₅ and R₆ together with the two nitrogen atoms to which they are bound, form a cycle selected from imidazolino, tetrahydropyrimidino and benzimidazolino;
   or
R₆ and R₇, each independently, together with the nitrogen atom to which they are bound, form a pyrrolidino or a piperidino group;
R₈ is selected from hydrogen, alkyl optionally substituted by a halogen atom, a hydroxy, a C₁-C₆ alkoxy, or an amino group, aryl and p-tolyl;
R₉ is selected from groups of Formula (IIIa), (IIIb), (IIIc), (IIId) and (IIIe) 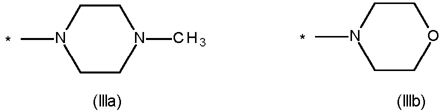 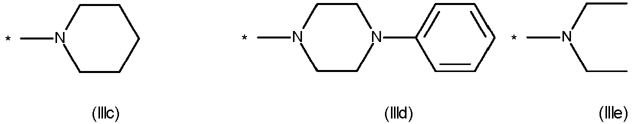
wherein the asterisk (*) indicates the linking bond;
as well as compounds of formula (I) wherein
   - R₃ is acetamido and R₁, R₂, R₄, R₅, R₆, R₇ are all hydrogen (Compound 2l)
   - R₃ is mesylamido and R₁, R₂, R₄, R₅, R₆, R₇ are all hydrogen (Compound 2n)
   and
R₃ is carbomethoxy and R₁, R₂, R₄, R₅, R₆, R₇ are all hydrogen (Compound 2p) for its use in the treatment and/or prevention of neurological diseases, including amyotrophic lateral sclerosis (ALS), Charcot-Marie Tooth disease, Azheimer's disease (AD), Parkinson's disease (PD).

Preferred compounds for use according to the invention are compounds of Formula (I) wherein:
- R₁, R₂, R₃, R₄, R₅, R₆, R₇ are all hydrogen (Compound 2a)
- R₁ is bromine and R₂, R₃, R₄, R₅, R₆, R₇ are all hydrogen (Compound 2b)
- R₂ is bromine and R₁, R₃, R₄, R₅, R₆, R₇ are all hydrogen (Compound 2c)
- R₂ is hydroxyl and R₁, R₃, R₄, R₅, R₆, R₇ are all hydrogen (Compound 2d)
- R₂ is methoxy and R₁, R₃, R₄, R₅, R₆, R₇ are all hydrogen (Compound 2e)
- R₂ is n-butyloxy and R₁, R₃, R₄, R₅, R₆, R₇ are all hydrogen (Compound 2f)
- R₃ is methyl and R₁, R₂, R₄, R₅, R₆, R₇ are all hydrogen (Compound 2g)
- R₃ is bromine and R₁, R₂, R₄, R₅, R₆, R₇ are all hydrogen (Compound 2h)
- R₃ is methoxy and R₁, R₂, R₄, R₅, R₆, R₇ are all hydrogen (Compound 2i)
- R₃ is nitro and R₁, R₂, R₄, R₅, R₆, R₇ are all hydrogen (Compound 2j)
- R₃ is phenyl and R₁, R₂, R₄, R₅, R₆, R₇ are all hydrogen (Compound 2k)
- R₃ is acetamido and R₁, R₂, R₄, R₅, R₆, R₇ are all hydrogen (Compound 2l)
- R₃ is p-tolylamido and R₁, R₂, R₄, R₅, R₆, R₇ are all hydrogen (Compound 2m)
- R₃ is mesylamido and R₁, R₂, R₄, R₅, R₆, R₇ are all hydrogen (Compound 2n)
- R₃ is p-tosylamido and R₁, R₂, R₄, R₅, R₆, R₇ are all hydrogen (Compound 2o)
- R₃ is carbomethoxy and R₁, R₂, R₄, R₅, R₆, R₇ are all hydrogen (Compound 2p)
- R₃ is 1-(4'-phenylpiperazinyl)carbonyl group and R₁, R₂, R₄, R₅, R₆, R₇ are all hydrogen (Compound 2q)
- R₃ is aminoguanidyl and R₁, R₂, R₄, R₅, R₆, R₇ are all hydrogen (Compound 2r)
- R₆ is n-butyl and R₁, R₂, R₃, R₄, R₅, R₇ are all hydrogen (Compound 2t)
- R₆ is benzyl and R₁, R₂, R₃, R₄, R₅, R₇ are all hydrogen (Compound 2u)
- R₆ and R₇ together with the nitrogen atom to which they are bond form a pyrrolidino group and R₁, R₂, R₃, R₄, R₅ are all hydrogen (Compound 2v)
- R₃ is p-tosylamido, R₆ is n-butyl and R₁, R₂, R₄, R₅, R₇ are all hydrogen (Compound 2w)
- R₅ and R₆ together with the nitrogen atom to which they are bond form a imidazolidino group and R₁, R₂, R₃, R₄, R₇ are all hydrogen (Compound 2x)
- R₄ is methyl, R₅ and R₆ together with the nitrogen atoms to which they are bond form a imidazolidino group and R₁, R₂, R₃, R₇ are all hydrogen (Compound 2y)
- R₅ and R₆ together with the nitrogen atoms to which they are bond form a benzimidazolidino group and R₁, R₂, R₃, R₄, R₇ are all hydrogen (Compound 2z).

Additional, preferred compounds for use according to the invention are compounds of formula (I) wherein:
- R₃ is benzyl and R₁, R₂, R₄, R₅, R₆, R₇ are all hydrogen (Compound 2aa)
- R₃ is 2'-aminoethylamido and R₁, R₂, R₄, R₅, R₆, R₇ are all hydrogen (Compound 2bb)
- R₃ is p-methoxybenzamido and R₁, R₂, R₄, R₅, R₆, R₇ are all hydrogen (Compound 2cc)
- R₃ is carbo(2-aminoethyloxy) and R₁, R₂, R₄, R₅, R₆, R₇ are all hydrogen (Compound 2dd)
- R₃ is carbo(2-(4'-morpholinyl)ethyloxy) and R₁, R₂, R₄, R₅, R₆, R₇ are all hydrogen (Compound 2ee)
- R₃ is a N,N-diethylaminocarbonyl group and R₁, R₂, R₄, R₅, R₆, R₇ are all hydrogen (Compound 2ff)
- R₃ is a N-morpholylcarbonyl group and R₁, R₂, R₄, R₅, R₆, R₇ are all hydrogen (Compound 2gg)
- R₃ is a 1-(4'-methylpiperazinyl)carbonyl group and R₁, R₂, R₄, R₅, R₆, R₇ are all hydrogen (Compound 2hh)
- R₃ is a 1-(4'-(3"-nitrophenyl)piperazinyl)carbonyl group and R₁, R₂, R₄, R₅, R₆, R₇ are all hydrogen (Compound 2ii)
- R₃ is a 1-(4'-(4"-methoxyphenyl)piperazinyl)carbonyl group and R₁, R₂, R₄, R₅, R₆, R₇ are all hydrogen (Compound 2jj)
- R₃ is a 1-(4'-(4"-chlorophenyl)piperazinyl)carbonyl group and R₁, R₂, R₄, R₅, R₆, R₇ are all hydrogen (Compound 2kk)
- R₅ and R₆ together with the nitrogen atoms to which they are bond form a tetrahydropyrimidino group and R₁, R₂, R₃, R₄, R₇ are all hydrogen (Compound 2ll)
- R₆ and R₇ together with the nitrogen atom to which they are bond form a piperidino group and R₁, R₂, R₃, R₄, R₅ are all hydrogen (Compound 2mm).

Most preferred compounds for use according to the invention are compounds 2a, 2b, 2c, 2d, 2e, 2f, 2g, 2i, 2l, 2v and 2z.

Any type of salt of the compounds of Formula (I) can be prepared, for instance in synthetic steps or for purification reasons and all salts are included in the scope of protection of the present invention.

According to a preferred embodiment, the salt of the compound of Formula (I) is preferably a pharmaceutically acceptable salt.

The compounds of Formula (I) can form salts with a variety of organic and inorganic acids. Such salts include those formed with hydrochloric acid, hydrobromic acid, hydroiodic acid, methanesulfonic acid, sulfuric acid, acetic acid, trifluoroacetic acid, oxalic acid, maleic acid, benzenesulfonic acid, toluenesulfonic acid and others (e.g., nitrates, phosphates, borates, tartrates, citrates, succinate, benzoates, ascorbates, salicylates and the like). Such salts may be formed as known to the skilled in the art. Preferred salts are hydrochloric acid, hydrobromic acid, hydroiodic acid and acetic acid salts.

The compound of Formula (I) may be prepared by any suitable synthetic method.

In the present disclosure, even if not expressly indicated, the compound Formula (I) may always be in the form of one of its salts and/or solvates.

Any possible stereoisomer as well as possible crystalline forms of the compound of Formula (I) and its salts or solvates are included in the scope of the invention.

According to one of its aspects, the present invention relates to a process for the preparation of a compound of Formula (I) or one of its salts or solvates, which comprises reacting a compound of Formula (IV) 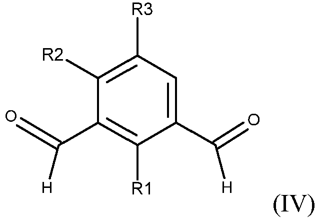 wherein R₁, R₂ and R₃ are as above defined, with a compound of Formula (V) 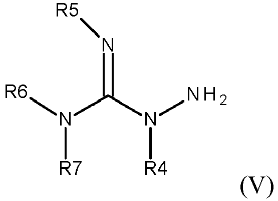 or a salt thereof, wherein R₄, R₅, R₆, and R₃ are as above defined, in a suitable solvent, and isolating the compound of Formula (I) thus obtained, optionally purifying and/o optionally converting it in a salt or solvate thereof.

The starting compounds of Formula (IV) and (V) are known compounds or can be prepared by tmethods known in the art.

Examples of their preparation is given in the following Schemes and Experimental Section.

The compound of Formula (V) is used in a molar amount which is at least double of the amount of the compound of Formula (IV).

According to a preferred embodiment, the compound of Formula (V) is used in a salified form, for instance in the form of hydrobromide, hydrochloride or hydroiodide salt.

The above reaction may be carried out at a temperature ranging from 40°C to the reflux temperature of the reaction mixture, preferably from 60°C to the reflux temperature of the reaction mixture, advantageously at about 80°C.

The solvent is preferably a lower alcohol, preferably ethanol. Other solvents may however be used in the process of the invention.

Preferably, a catalytic amount of an acid may be used in the above reaction, preferably of hydrochloric or acetic acid.

The reaction is completed in a few hours, for instance 1-12 hours. The skilled in the art is anyway perfectly able to check the development of the reaction with conventional methods, for instance chromatographic techniques, such as thin layer chromatography (TLC).

The compound of Formula (I) may be isolated with common techniques, for instance by filtration or extraction with appropriate solvents from the reaction mixture.

If necessary or desired, the compound of Formula (I) may be purified by conventional techniques, such as chromatography, crystallization etc.

Compound of Formula (I) wherein R₃ is an aminoguanidyl-hydrazone group, may be also prepared by reacting a compound of Formula (VI) 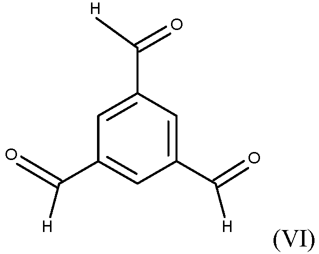 in lieu of the compound of Formula (IV), with the compound of Formula (V) as above defined, in the same reaction conditions above disclosed.

As a comparative example, the compound of Formula (VII) was prepared, 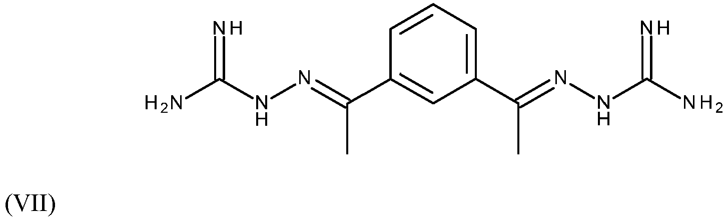 by reacting compound of Formula (VIII) 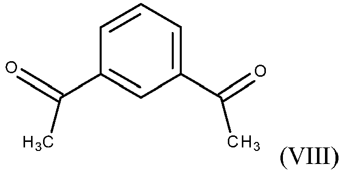 with the compound of Formula (V) as above defined, in the same reaction conditions above disclosed. Compound (VII) is disclosed in WO95/19767 as an inflammation modulator.

In the following synthetic schemes, the dashed lines indicate the delocalization of the charge between the two salified nitrogens.

Reagents and conditions of synthesis are given in the Experimental Section of the description.

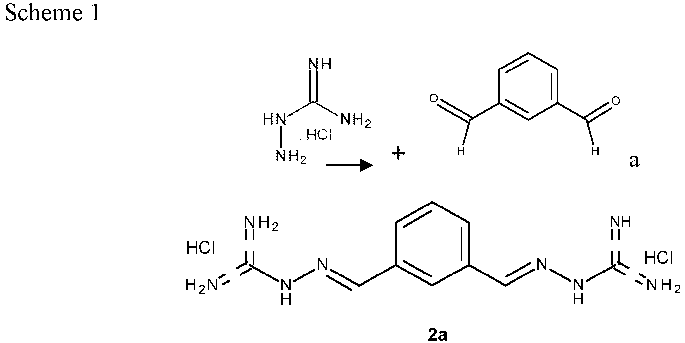

Reagents and conditions: cat. HCl, EtOH, 80°C, 2 hrs, 89%.

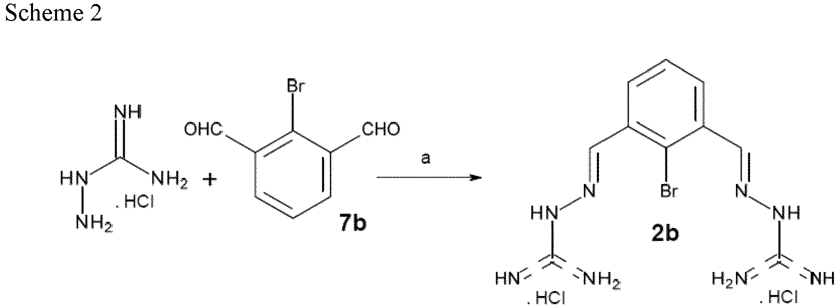

Reagents and conditions: (a) cat. HCl, EtOH, 80°C, 2 hrs, 89%.

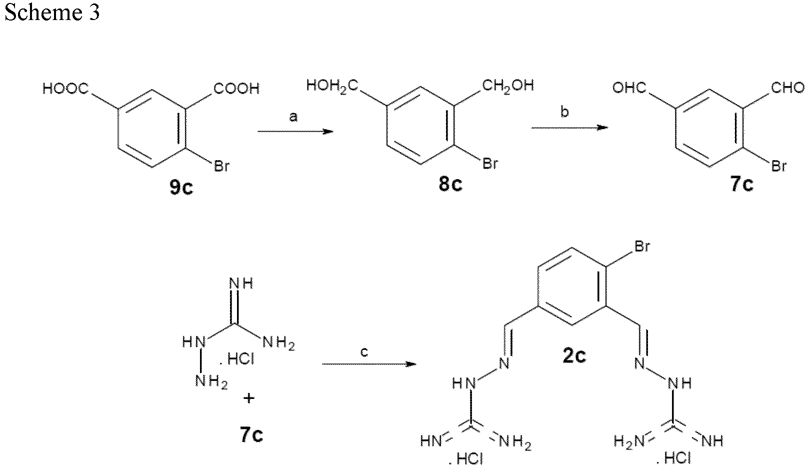

Reagents and conditions: (a) 1M BH₃ in THF, dry THF, N2, 0°C to rt, 48 hrs, 86%; (b) MnO₂, CHCl₃, 70°C, 16 hrs, 86%; (c) cat. HCl, EtOH, 80°C, 4 hrs, 88%.

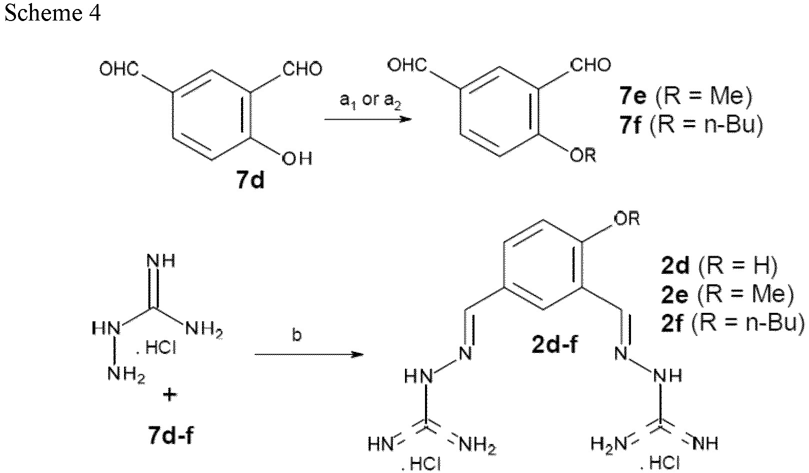

Reagents and conditions: (a1) MeI, K₂CO₃, dry DMF, N₂, rt, 24 hrs, 60%; (a2) n-BuBr, K₂CO₃, dry DMF, N2, 85°C, 24 hrs, 75%; (b) cat. HCl, EtOH, 80°C, 2 to hrs, 77% (2d), 79% (2e) or 80% (2f).

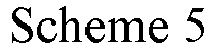

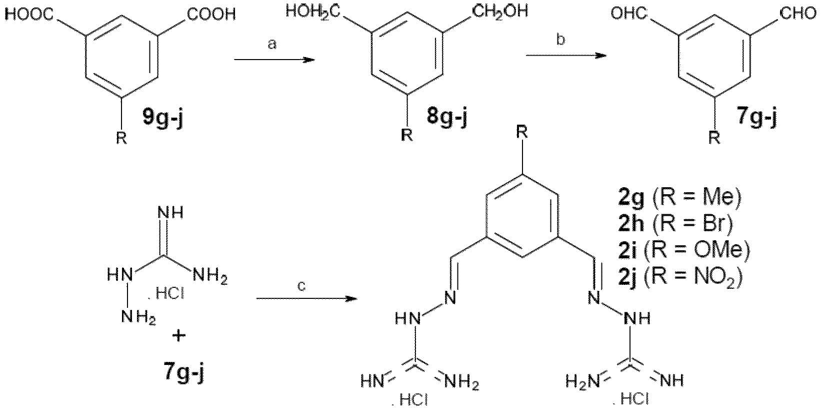

Reagents and conditions: (a) 1M BH₃ in THF, dry THF, N₂, 0°C to rt, 48 hrs, 80 to 88%; (b) MnO₂, CHCl₃, 70°C, 16 hrs, 77 to 92%; (c) cat. HCl, EtOH, 80°C, 2 to 8 hrs, 77 (2g), 88% (2h), 81% (2i) or 80% (2j).

Compound 2aa (R = CH₂Ph) can be prepared as shown in Scheme 3, starting from the corresponding 5-benzyl-phenyl 1,3-dicarboxylic acid 9aa.

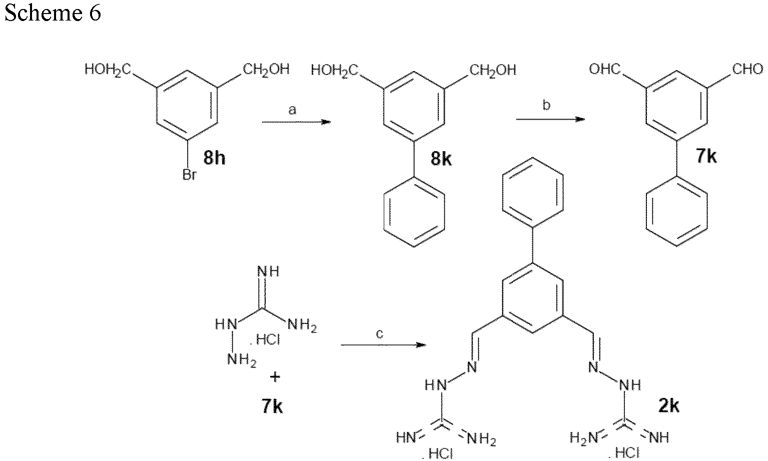

Reagents and conditions: (a) PhB(OH)2, Pd(PPh₃)₄, aq. 2N Na₂CO₃, dioxane, 105°C, 6 hrs, 82%; (b) MnO₂, CHCl₃, 70°C, 16 hrs, 81%; (c) cat. HCl, EtOH, 80°C, 8 hrs, 79.

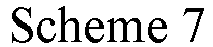

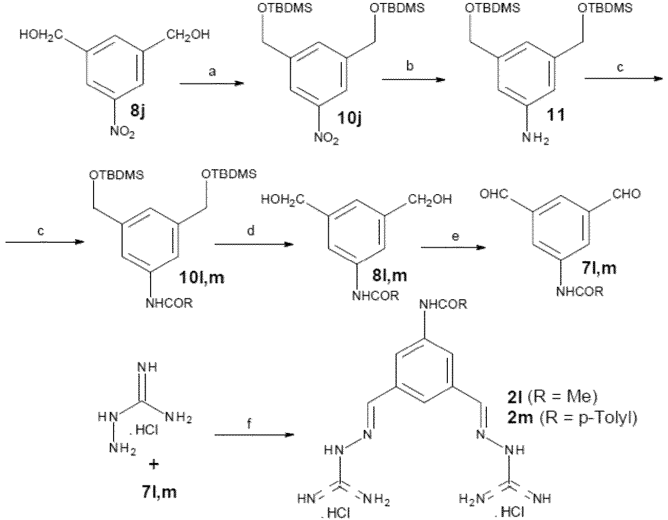

Reagents and conditions: (a) TBDMS-Cl, 1H-imidazole, CH₂Cl₂, 0°C to rt, 3 hrs; (b) cat. 10% Pd/C, H₂, EtOH, rt, 24 hrs, 78% (2 steps); (c) acylating agent, TEA, DMAP, CH₂Cl₂, 0°C to rt, 1 to 2 hrs, 89% (101) or 80% (10m); (d) AcCl, dry MeOH, N₂, 0°C, 30 min, 94% (8l) or 84% (8m); (e) MnO₂, CHCl₃, 70°C, 16 hrs, 88% (7l) or 83% (7m); (f) cat. HCl, EtOH, 80°C, 4 to 6 hrs, 85% (2l, 2m).

Compounds 2bb (R = CH₂CH₂NH₂) and 2cc (R = p-OMePh) can be prepared as shown in Scheme 7, using the corresponding acylating agents in step c, and obtaining respectively 10bb and 10cc.

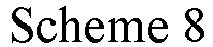

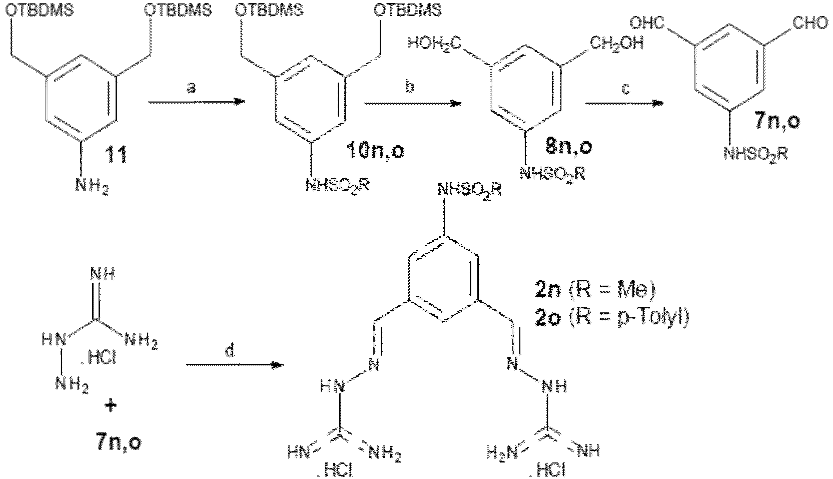

Reagents and conditions: (a) sulfonylating agent, pyridine, DMAP, CH₂Cl₂, 0°C to rt, 16 hrs, 85% (2o); (b) AcCl, dry MeOH, N₂, 0°C, 30 min, 81% (2o); (c) MnO₂, CHCl₃, 70°C, 16 hrs, 68% (2n, 3 steps) or 90% (2o); (d) cat. HCl, EtOH, 80°C, 4 hrs, 83% (2l) or 79% (2o).

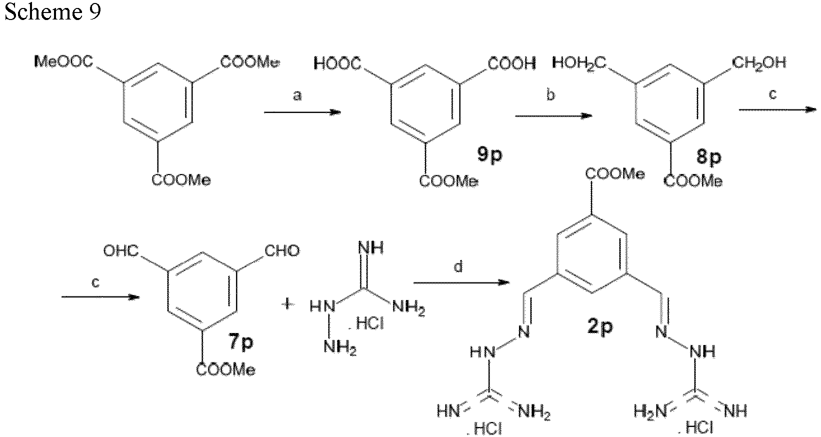

Reagents and conditions: (a) 1.8M aq. NaOH, MeOH, rt, 24 hrs; (b) 1M BH₃ in THF, dry THF, N₂, 0°C to rt, 24 hrs, 54% (2 steps); (c) MnO2, CHCl₃, 70°C, 16 hrs, 85%; (d) cat. HCl, EtOH, 80°C, 4 hrs, 71%.

Compounds 2dd (COOCH₂CH₂NH₂ ester) and 2ee (R= 2-morpholinylethyl ester) can be prepared as shown in Scheme 9, by hydrolysing compound 7p and esterifying it with N-protected ethanolamine or with N-hydroxyethyl morpholine.

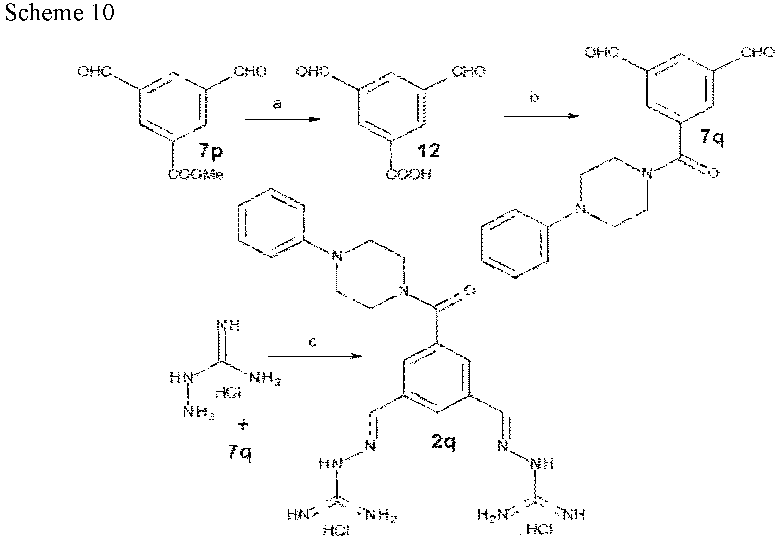

Reagents and conditions: (a) NaOH, water/THF, rt, 24 hrs; (b) 4-Ph-piperazine, EDC, HOBt, TEA, dry CH₂Cl₂, N2, rt, 24 hrs, 30% (2 steps); (c) cat. HCl, EtOH, 80°C, 4 hrs, 72%.

Compounds 2ff (diethylamide), 2gg (morpholinoamide), 2hh (4'-methylpiperazinylamide), 2ii (4'-(3"-nitrophenyl)piperazinylamide), 2jj (4'-(4"-methoxyphenyl)piperazinylamide) and 2kk (4'-(4"-chlorophenyl)piperazinylamide)) can be prepared as shown in Scheme 10, by substituting N-phenylpiperazine with the appropriate amidation reagent.

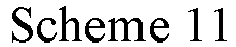

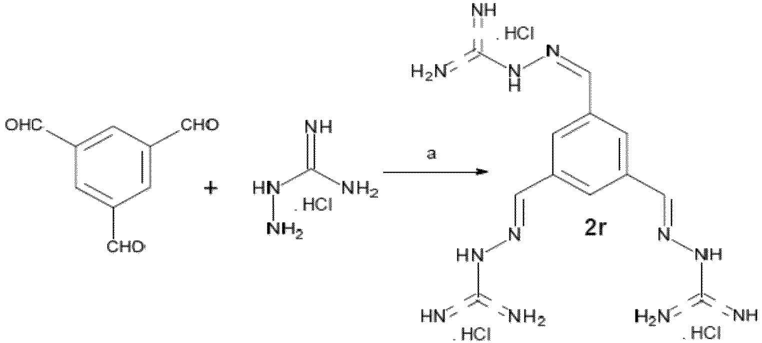

Reagents and conditions: (a) cat. HCl, EtOH, 80°C, 4 hrs, 78%

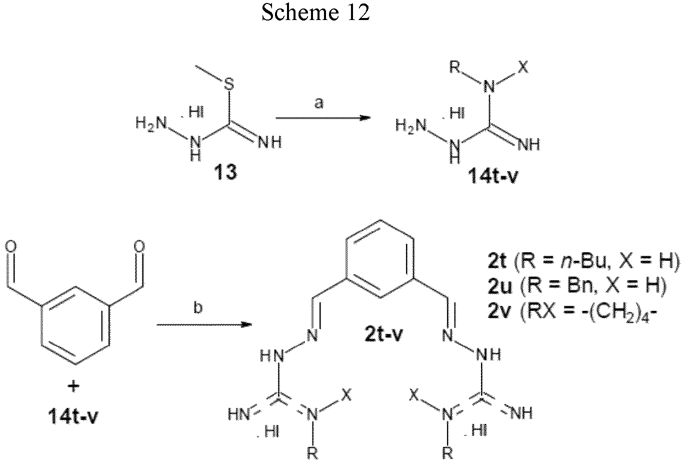

Reagents and conditions: (a) n-BuNH₂ (14t), BnNH2 (14u) or pyrrolidine (14v), MeOH, rt, 72 hrs, 80% (14t), 60% (14u) or 89% (14v); (b) EtOH, 80°C, 16 hrs, 53% (2s), 70% (2u) or 80% (2t).

Compound 2ll (RX = -(CH₂)₅-) can be prepared as shown in Scheme 12, by using piperidine as an amidating reagent in step a to yield 1411.

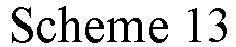

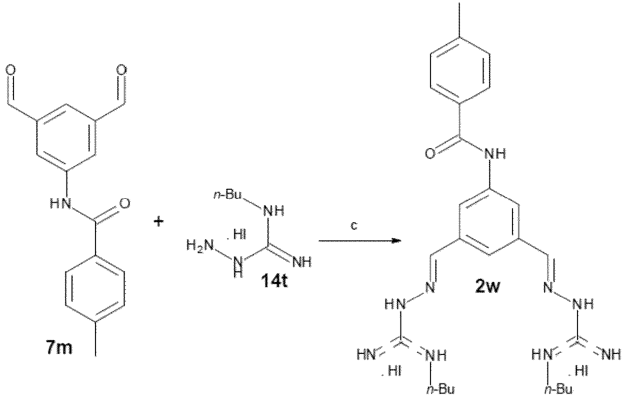

Reagents and conditions: (a) EtOH, 80°C, 16 hrs, 70%.

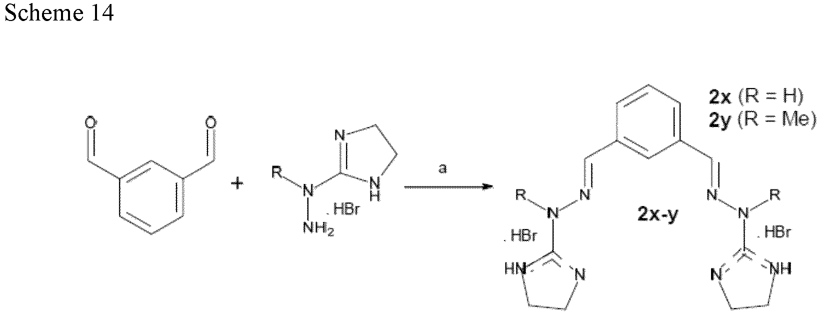

Reagents and conditions: (a) EtOH, 80°C, 16 hrs, 82% (2x, 2y).

Compound 2mm (RX = -(CH₂)₅-) can be prepared as shown in Scheme 14, by using a tetrahydropyrimidine-containing aminoguanidine as an aminoguanidylating reagent in step a to yield 2mm.

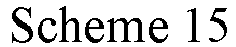

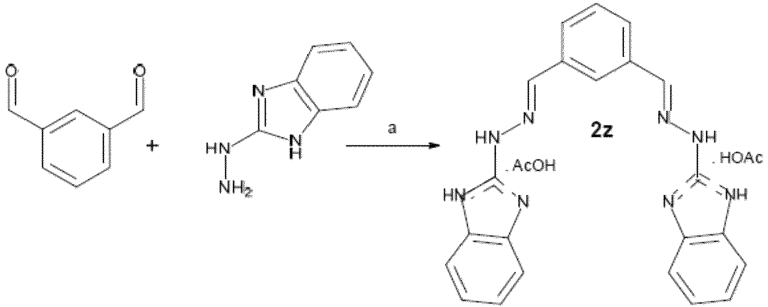

Reagents and conditions: (a) cat. AcOH, EtOH, 80°C, 2 hrs, 66%.

The compounds of Formula (I) or pharmaceutically acceptable salts thereof for use in therapy and diagnostics is a further subject-matter of the invention.

Compounds of Formula (I), especially the preferred compounds as above defined, showed an interesting retromer stabilizer activity and are useful in the treatment and/or prevention of several neurological diseases, such as for instance amyotrophic lateral sclerosis (ALS), Charcot-Marie Tooth disease, Azheimer's disease (AD), Parkinson's disease (PD) and other neurological diseases, wherein retromer stabilizing activity is beneficial.

Compounds of Formula (I) or one of their pharmaceutically acceptable salts for use in therapy are preferably administered in the form of pharmaceutical compositions.

According to one of its aspects, the invention relates to a pharmaceutical composition comprising at least one compound of Formula (I), or one of its pharmaceutically acceptable salts or solvates, preferably in combination with at least one pharmaceutically acceptable carrier and/or excipient.

The compositions of the invention include all the compositions where the compound of the present invention is comprised in an amount which is effective in achieving its intended aim. Although the individual needs vary, determining optimal ranges of effective amount per each component is known to persons skilled in the art. The administered dose will therefore depend on age, health and weight of the recipient, type of treatment, frequency of the treatment and nature of the desired effect.

Typically, the compound can be administered daily to mammals and in particular to humans, orally at a dose between 1 to 100 mg/kg, or an equivalent amount of one of its pharmaceutically acceptable salts, of the body weight of the treated mammal. Preferably, about 1 to 100 mg/kg are administered orally. For the intramuscular administration, the dose is generally half of the oral dose. For example, an acceptable intramuscular dose would be about 0.5 to 50 mg/kg, and more preferably, about 0.1 to 10 mg/kg.

Preferably the compositions, which can be administered by oral and parenteral route, are those known to the person skilled in the art, e.g., they will be in the form of: tablets, dragees, prolonged release pills and capsules, mouthwashes, gels, liquid suspension, hair dyes, hair gels, shampoos and other preparations which can be administered rectally, such as suppositories, as well as acceptable solutions for the parenteral, topical or oral administration, containing about 0.01 to 99%, preferably 0.25 to 75% of active ingredient(s).

The pharmaceutical compositions can be administered by any means to achieve the desired aim. For example, the administration can be parenteral, subcutaneous, intravenous, intramuscular, intraperitoneal, transdermal, buccal, intrathecal, intracranial, intranasal or oral.

The pharmaceutical preparations of the present invention are produced as is known to one skilled in the art, e.g., by conventional mixing, granulation, sugar-coating, dissolution, or freeze-drying processes. Therefore, the pharmaceutical preparations for oral use can be obtained by combining the active ingredient with solid excipients, optionally by grinding the resulting mixture and treating the granule mixture, after adding suitable excipients, if desired and necessary, in order to obtain tables or dragee cores.

Suitable excipients are, in particular, diluents and fillers such as sugars, celluloses and calcium phosphate, as well as binders such as, e.g., starch, gelatin, cellulose and polyvinyl pyrrolidone. If desired, disaggregating agents can be added as the above mentioned starches, cross-linked polyvinyl pyrrolidone, agar and alginic acids and the salts thereof. Other useful excipients are free-flowing agents and lubricants, e.g., silica, talc, stearic acid and salts thereof and polyethylene glycol. The cores of the pills and the tablets can have suitable coating that, if desired, is resistant to digestive juices. In order to produce the coatings resistant to digestive juices, it is possible to use suitable polymers as it is well known to the person skilled of the art.

Other pharmaceutical compositions that can be used orally include hard capsules made of gelatin, as well as sealed, soft capsules made of gelatin and a plasticizer such as, e.g., glycerol or sorbitol. Hard capsules can contain the active ingredient in the form of granules which can be mixed with diluents, binders and/or lubricants and, optionally, stabilizers. In the soft capsules, the active ingredient is preferably dissolved or suspended in a suitable liquid, such as fatty oils or liquid paraffin. Moreover, stabilizers can be added.

Possible pharmaceutical compositions that can be used rectally include, e.g., suppositories, consisting of a combination of one or more active ingredients together with suitable excipients. Such suitable excipients are, e.g., natural or synthetics triglycerides, or paraffin hydrocarbons.

Formulations suitable for the parenteral administration include aqueous solutions of the active ingredient in soluble form, preferably in water. Moreover, suspensions of the active ingredients can be administered, such as suspensions of suitable injectable oils. Suitable lipophilic solvents or carriers include fatty oils or esters of synthetic fatty acids. Aqueous injections can contain substances increasing the viscosity of the suspension, such as sorbitol and dextran. Optionally, the suspension can also contain stabilizers.

In addition to be administered in pharmaceutical compositions, the compounds of Formula (I) as described above may also be administered as pure compounds.

According to another of its aspects, the invention relates to a compound of Formula (I) or one of its pharmaceutically acceptable salts or solvates for use in the treatment and/or prevention of several neurological diseases, such as for instance amyotrophic lateral sclerosis (ALS), Charcot-Marie Tooth disease, Azheimer's disease (AD), Parkinson's disease (PD) and other neurological diseases, wherein retromer stabilizing activity is beneficial.

According to another of its aspects, the invention relates to a pharmaceutical composition of the invention for use in the treatment and/or prevention of several neurological diseases, such as for instance amyotrophic lateral sclerosis (ALS), Charcot-Marie Tooth disease, Azheimer's disease (AD), Parkinson's disease (PD) and other neurological diseases, wherein retromer stabilizing activity is beneficial.

According to another of its aspects, the invention relates to a method for the treatment and/or prevention of several neurological diseases, such as for instance amyotrophic lateral sclerosis (ALS), Charcot-Marie Tooth disease, Azheimer's disease (AD), Parkinson's disease (PD) and other neurological diseases, wherein retromer stabilizing activity is beneficial, said method comprising administering, to a subject in need thereof, an effective amount of a compound of Formula (I) or one of its pharmaceutically acceptable salts or solvates, or of a pharmaceutical composition according to the invention.

In therapy, especially in the treatment and/or prevention of neurological diseases such as those above disclosed, a compound of Formula (I) may be either administered alone, or in combination with another compound of Formula (I) and/or with another therapeutic agent useful in said treatment and/or prevention.

The compounds of the invention may also be used as a diagnostic tool comprising a compound of Formula (I) or one of its pharmaceutically acceptable salts.

The invention is further disclosed in the following Experimental Section for illustrative purposes.

### Experimental Section

### Biochemical characterization of amino guanidine hydrazones

Several experimental evidence shows that retromer has a neuroprotective role, especially in age-related neurodegenerative disorders. For example, the disruption of this ancient multimeric complex has been observed in a number of diseases including Parkinson's disease (PD) and Alzheimer's disease (AD) (Small and Petsko 2015). In PD a mutation of VPS35 (D620N) is dominant for a late onset form of the disease (Vilarino-Guell, Wider et al. 2011). Furthermore, VPS35 levels are significantly reduced in brain regions selectively vulnerable to AD (Small, Kent et al. 2005).

Thus, novel pharmacological chaperones were synthesized according to the invention, which are capable to significantly increase the levels of VPS35, and consequently of the retromer complex, would be promising compounds to be tested for their neuroprotective effects. To functionally screen the majority of synthetized amino guanidine hydrazones of the invention and determine their effects on the retromer complex, we incubated Neuro2a cells with each synthetized molecule at the concentration of 10 µM. We included controls in each experiment using the standard Compound R55 (Mecozzi, Berman et al. 2014) that efficiently and dose-dependently increased VPS35 levels after 48 hours of incubation (Figure 1A, B).

The test was executed in triplicate for each tested compound of the invention, cells lysates were established after 48 hours' incubation, and levels of VPS35 were normalized on the housekeeping gene β-Actin by Western blot. We observed a preliminary SAR among the compounds.

Most of the tested compound of the invention were able to increase the levels of VPS35 levels, although with a certain variability. In particular, Compound 2a was able to consistently increase VPS35 levels with a limited variability among replicates (Table 1).

Table 1 shows mean fold changes ± s.d. from independent cultures (n=3) receiving each compound at 10 µM for 48 hours. Fold changes are calculated over normalized VPS35 levels measured in vehicle-treated cells.

**Table 1**

| Compound | Mean fold changes | Standard deviation |
|---|---|---|
| Vehicle | 1 | x |
| 2a | 1.951 | 0.029 |
| 2b | 1.300 | 0.520 |
| 2c | 1.617 | 0.389 |
| 2d | 2.477 | 1.608 |
| 2e | 2.070 | x |
| 2f | 1.415 | 0.983 |
| 2g | 2.080 | 0.735 |
| 2i | 1.310 | 0.246 |
| 2l | 1.660 | 0.127 |
| 2m | 0.974 | 0.146 |
| 2n | 1.280 | 0.164 |
| 2o | 1.501 | 0.320 |
| 2p | 1.582 | 0.280 |
| 2q | 1.784 | 0.370 |
| 2r | 1.154 | 0.343 |
| 2v | 1.978 | 0.690 |
| 2z | 1.105 | x |
| 2w | 0.625 | 0.165 |

| | | |
|---|---|---|
| x: single experiment, no standard deviation | | |

As a comparison, compound (VII) was also tested in the same conditions above. The result of the test are reported in Table 2

| Compound | Mean fold changes | Standard deviation |
|---|---|---|
| Vehicle | 1 | x |
| ( VII) | 0.600 | x |

| | | |
|---|---|---|
| x: single experiment, no standard deviation | | |

### Retromer levels in Amyotrophic Lateral Sclerosis

While the role of retromer is assessed in several neurodegenerative diseases (Small, Kent et al. 2005; Vilarino-Guell, Wider et al. 2011) nothing is known about any putative role in ALS. Misfolded aggregated proteins are thought to participate to pathogenic mechanisms leading motor neurons (MNs) cell death (Cleveland 1999). For example, TAR DNA binding protein-43 (TDP-43) are identified as a core component of ubiquitinated inclusions in a large number sporadic ALS patients (Arai, Hasegawa et al. 2006). Retromer has been implicated in AD which is featured by amyloid beta plaques and aggregates of Aβ protein in the brain of patients, as well as mutations in VPS35 are identified in autosomal dominant PD patients (MacLeod, Rhinn et al. 2013).

We dertermined VPS35 levels in MNs of mice carrying the hSOD1G93A (mouse strain B6.Cg-Tg (SOD1G93A) 1Gur/J, here after G93A) and their wild type (WT) litters (Gurney, Pu et al. 1994). We scored VPS35 immunoreactivity in lumbar spinal cords (SCs) of preclinical G93A (day 30, 60 and 90) mice. Sections were double labelled with marker NeuN to identify MNs (Figure 2A). VPS35 is preferentially expressed in cortical excitatory neurons of layer IV-V, while inhibitory neurons express this protein at low level (Wen, Tang et al. 2011). Similarly, in WT spinal cords we observed VPS35 immunoreactivity in *bona fide* ventral MNs. Interestingly, VPS35 expression levels were substantially reduced in G93A MNs (Figure 2A). At days 30 and 60 G93A mice are asymptomatic (Gowing, Philips et al. 2008) and MNs are not yet degenerated as we shown using the neuronal marker NeuN. Thus, the loss of VPS35 was not attributable to a general loss of MNs. We next confirmed that CRC members of the retromer are reduced in G93A mice double labelling parallel sections for VPS26 and NeuN (Figure 2B). Interestingly the reduction of VPS35 and VPS26 protein levels did not derive from a reduction of their mRNA levels (Figure 2F). We next corroborated our observation by Western blotting total protein extracts from lumbar spinal cords of pre-clinical and clinical G93A mice. Also in thise experiment we noticed VPS35 levels that were significantly reduced in the G93A genetic background (Figure 2C). Similarly, levels of the VPS26b as well as VPS29 were significantly dampened in protein extracts from G93A lumbar SCs sampled at day 100 (Figure 2D and E).

### Retromer stabilization increases cell survival of G93A transfected cells

Pathological alteration of the Golgi apparatus, such as its fragmentation, has been described in many neurodegenerative disorders including ALS. Neuro2a cells transfected with plasmids encoding the G93A mutant form of the SOD1 show alteration of the Golgi transport (Soo, Halloran et al. 2015). Along the same lines, Golgi fragmentation precedes neuromuscular denervation and axon retraction in ALS mice (van Dis, Kuijpers et al. 2014). We transfected Neuro2a cells with plasmids encoding the G93A mutant form of the human SOD1 gene (hereafter G93A) in presence or in absence of compound 2a (10 µM). Cells were harvested after 48 hours, labelled for Golgin-97 and scored for the distribution of trans-Golgi. Untreated cells and cells receiving the control plasmid encoding the GFP showed a compact distribution of Golgin-97, while percentages of cells showing partial or complete fragmentation of the Golgi complex were greatly increased in cells transfected with the G93A plasmid. However, administration of the compound 2a significantly diminished these percentages, reconstituting the Golgin-97 distribution that we observed in control cells (Figure 3A). Of note, the fragmentation of Golgi in cells receiving G93A plasmids was not depending on Golgin97 expression levels (Figure 3A). We speculated that Golgi fragmentation may exacerbate cell death in this cell line. Therefore, we assayed cell survival of Neuro2a transfected with G93A plasmids. Accordingly, cells were transfected with G93A plasmids or with a control plasmid encoding the LacZ. We also assessed that 10 µM of compound 2a did not elicit toxic effects in cells. Rates of cell survival were similar among controls - i.e. cells receiving LacZ plasmids or compound 2a - when compared with untreated cells. On the other hand, the over-expression of G93A plasmids affected Neuro2a cell survival. The administration of compound 2a significantly reverted this phenotype (Figure 3B). We next asked whether the rescue of cell survival that we observed in cells receiving the compound 2a may depend on retromer functionality. We performed a further experiment in which we transfected cells with a plasmid encoding VPS35, instead of using the compound 2a to foster retromer stabilization. We transfected Neuro2a cells with G93A plasmids with or without plasmids encoding VPS35. As expected, cell viability was decreased in Neuro2a receiving G93A plasmids, while cells transfected with both G93A and VPS35 plasmids significantly increased the cell viability.

We next used further plasmids to perform a VPS35 short interference in Neuro2a cells, with the final goal of reducing VPS35 levels in cells that were also receiving G93A plasmids. We firstly established the efficacy of plasmids to inhibit VPS35 expression. We transfected 3 different commercial plasmids designed to interfere *Vps35* mRNA in Neuro2a cells. While all of them were able to reduce VPS35 levels, the Sh56 plasmid resulted in 60% reduction of VPS35 mRNA and protein levels (Figure 4A-C). Furthermore, immunofluorescence for VPS35 showed a substantial reduction of VPS35 immunoreactivity in Neuro2a cells transfected with Sh56-RNAi plasmids (Figure 4E)

We next transfected Neuro2a cells with G93A along with Sh56 or scramble plasmids. The simple reduction of VPS35 levels in Neuro2a cells did not affect their survival. As expected, cell survival was reduced in Neuro2a cells receiving G93A plasmids (Figure 3D). Interestingly, diminishing VPS35 levels by Sh56 in G93A transfected cells further diminished cell survival and above all, substantially affected the capability of compound 2a to revert this phenotype (Figure 3D).

We next assayed cytotoxicity by measuring lactate dehydrogenase (LDH) release with a LDH-Glo^{™} cytotoxicity assay. Cells were treated with plasmids encoding G93A and the Sh56-RNAi sequence with or without compound 2a, while supernatants were used to measure LDH contents. Overexpression of G93A increased the release of LDH by Neuro2a cells, when compared with cells transfected with LacZ-encoding plasmids or with LacZ+Sh56 plasmids (Figure 3E). Compound 2a significantly reduced LDH release in cells transfected with G93A plasmids. On the other hand, LDH release increased when cells received G93A plasmids along with Sh56 plasmids. Interestingly, high release of LDH was observed in Neuro2a cells transfected with G93A plasmids and Sh56 plasmids and treated with compound 2a, further confirming that 2a counteracts SOD1^{G93A}-mediated cytotoxicity by targeting the retromer complex (Figure 3E).

Compound 2a increases the stability of the retromer complex *in vitro.* We further validated this concept by measuring VPS35 degradation rates by a cycloheximide (CHX) chase assay. Neuro2a cells received vehicle or lead 2a (10 µM) for 24 hours, then CHX was added to the medium, and VPS35 levels were measured by western blot. We observed that vehicle-treated cells exhibited a drop of VPS35 levels (~40%) after 8 hours of CHX treatment. Conversely, pre-treating cells with lead 2a protected VPS35 from degradation (Figure 5A, B). As is the case with R55 (Mecozzi, Berman et al. 2014), compoud 2a should not influence the transcription levels of CRC genes in Neuro2a cells. Accordingly, mRNAs levels of *Vps35, Vps26* and *Vps29* did not change in Neuro2a cells treated with 2a (10 µM) for 48 hours (Figure 5C).

We next asked whether the compound 2a can cross the blood brain barrier (BBB) of the brain. We run a pharmacokinetic assay on C57BL6J mice that received daily intraperitoneal injections of compound 2a (10 mg/Kg/day). Mice were sacrificed after 1, 7, 15 and 30 days saline perfused to avoid the CNS contamination of compounds contained in blood circulation and brains used for the determination of compound 2a levels by mass spectrometry. We detected a significant amounts of compound 2a in the brain confirmed the ability of compound 2a to access the CNS (Figure 6A). We next investigated *in vivo* if the retromer stabilization occurs in response to Compound 2a treatement. We intraperitoneally injected R55 and Compound 2a in adult C57BL6J mice as above described. After one week, mice were sacrificed, proteins extracts were assayed by western blot. While treatments with R55 did not increase VPS35 levels in SC extracts, compound 2a significantly increased such levels (≈1.7, Figure 6B), further confirming the ability of compound 2a to stabilize the retromer complex.

In all these experiments we did not observe cell toxicity in Neuro2a as well as any adverse events in mice injected with the compound 2a. Nonetheless, we assessed the cytotoxicity of compound 2a, measuring cell survival of Neuro2a cells exposed to increasing concentrations of 2a. The calculated LD₅₀ was ~260 µM (Figure 7A), a much higher value than the concentration needed to stabilize the retromer in our *in vitro* experiments. The final objective of our approach is to stabilize the retromer in ALS MNs or, in general, in neurons in which the retromer expression is substantially dampened. Therefore, we checked compound 2a toxicity in neurons by using primary neuronal cultures coupled with Micro Electrode Array (MEA) devices. Neurons from mouse E16.5 cerebral cortices were plated on MEAs and kept in culture for 14 days to allow the development of spontaneous firing activity. We recorded spikes in neurons receiving increasing concentrations of 2a (from 1 to 100 µM, Figure 7B, C). Low concentrations of compound 2a did not alter neuronal firing, while we observed a slight, but not significant, reduction of spikes when neurons were incubated with the highest concentration of 2a (Figure 7D). Altogether these results show a wide therapeutic window for lead 2a, considering the low µM retromer-impacting concentration needed in vitro to increase VPS35 levels.

We tested the specificity of compound 2a in an assay based on H₂O₂-induced chromosomal breaks that lead to DNA fragmentation and cell death. In principle such DNA lesions are the cause of cell death according to a process that is not influenced by the retromer. Thus, our leads should not be able to modulate rates cell survival in Neuro2a cells receiving H₂O₂. We incubated Neuro2a cells with 200µM of H₂O₂ along with compounds 2a, 2c, 2d and 2g (10µM). Comparing cell survival between cells receiving H₂O₂ with or without our compounds did not result in any significant difference (Figure 7E), further corroborating the idea that our leads operate on the retromer and of course they do not prevent cell death caused by DNA fragmentation. Altogether these results indicate that G93A-mediated alteration of the Golgi as well as the increased cell mortality observed in Neuro2a cells are counteracted by the pharmacological chaperone 2a, acting specifically on the retromer complex. Finally, we also demonstrated that compound 2a can cross the BBB in mice.

### Compound 2a attenuates clinical impairment and MN cells death in G93A mice

Having established that compond 2a crosses the BBB we started to treat mice carrying the G93A mutant form of the SOD1. Starting from day 30, G93A mice and their WT litters were daily injected with compound 2a (10 mg/kg) or vehicle. Mice receiving 2a displayed a small drop of the body weight when compared with mice receiving the vehicle treatment, although such reduction did not reach statistical significance (Figure 8A). However, G93A receiving 2a displayed a substantial amelioration of the locomotion performances that was mirrored by increased latency to fall in the accelerating rotarod test. Indeed, starting from day 80 the latency to fall progressively diminished in vehicle-treated G93A. Indeed we recorded a 30% reduction of this parameter at day 100 when compared to values recorded at day 50 (Figure 8C). G93A mice receiving 2a displayed motor performances that were pretty constant along the time and were overlapping to values measured in WT mice, suggesting that a stabilization of the retromer complex can foster protective effects in vivo (Figure 8B, C).

Mice were sacrificed at day 100, and lumbar SCs assayed for studying MNs cell morphology and numbers. We initially scored NeuN⁺ neurons in the ventral horn of the SC to monitor *bona fide* motor neurons. Mice receiving compound 2a displayed significantly higher numbers of motor neurons than vehicle-treated G93A mice (Figure 8D). Accordingly, we observed similar results scoring the number of Choline Acetyltransferase⁺ (ChAT) motor neurons on parallel sections (Figure 8E).

We next performed histopathological analysis of sciatic nerves from WT control and G93A mice receiving compound 2a or vehicle. Consistent with the advanced stage of the disease, G93A mice at day 100 displayed axonal degeneration in sciatic nerves when compared with their WT litters (Figure 9A, B). On the other hand, G93A mice treated with 2a showed a significant protection of peripheral nerve fibers that was mirrored by a reduction of fiber undergoing degeneration (Figure 9C,D). Using Luxol Fast Blue (LFB) staining, which binds lipoproteins of the myelin sheath, we analyzed myelination in sagittal sections of these nerves. We observed that G93A mice receiving compound 2a displayed a substantial attenuation of the demyelination process, when compared with vehicle-treated G93A mice (Figure 10A-D). Parallel sections, labelled for myelin basic protein (MBP) and medium neurofilaments (NF-M), showed deranged myelin in vehicle-treated G93A mice while the administration of compound 2a preserved the integrity of myelin in G93A mice (Figure 10E). Therefore, we observed a reduction of fibers degeneration and demyelination in sciatic nerves of G93A mice trated with compound 2a.

We next assayed retromer proteins labelling sections of mice sacrificed at day 100 for VPS35 and NeuN. The analysis of confocal stacks of lumbar ventral horns revealed that the fluorescent intensity levels of VPS35 (measured as MFI) were significantly increased in G93A mice receiving compound 2a, if compared with the intensity measured in vehicle-treted G93A mice (Figure 11A). Similarly, we observed that VPS26 levels were incresed in G93A mice receiving compound 2a (Figure 11B). We further corroborated our experimental finding observing that compound 2a increased retromer functionality. The cation-independent mannose 6-phosphate receptor (CI-MPR) is a cargo (Arighi et al., 2004) of the retromer complex that is unstable in VPS26 knockout cells (Seaman 2004). We observed a substantial reduction of CI-MPR MFI levels in MNs of vehicle-treated G93A mice when compared with WT litters (Figure 11C). However, MFI levels significantly increased in G93A MNs of mice treated with lead 2a (Figure 11C). Sortilin encodes for a receptor belonging to the Vps10 family, and it has been involved in Frontotemporal Dementia (FTD), (Lane 2012). Sortilin shares functional homologies with MPRs and is another key retromer cargo (Nielsen et al., 2001) that co-immunoprecipitates with VPS35 in brain extracts (Muhammad 2008) and is downregulated in VPS35 knockout cells (Pan 2017). Sortilin MFI levels were significantly reduced in MNs of vehicle-treated G93A mice, thus mirroring the reduction of CI-MPR that we previously observed. However, treatment with lead 2a significantly increased Sortilin levels in G93A MNs (Figure 11D).

We next investigated Cathepsin D (CTSD) expression and distribution in G93A mice, since it is known that the trafficking of this lysosomal enzyme involves the retromer complex (Seaman 2004). In humans, maturation of CTSD starts with a pre-pro-CTSD that is converted to pro-CTSD (52 kDa) in the endoplasmic reticulum. Recycling of pro-CTSD, and its downstream migration to acidic compartments involves CI-MPR and the retromer pathway. In late endosomes, pro-CTSD is further processed to an intermediate form (48 kDa), and finally converted to mature CTSD (34 kDa) in lysosomes (Laurent-Matha 2006). The inactivation of VPS35 or the knockdown of VPS26 affect the maturation of CTSD. We scored CTSD levels in equal amounts of lumbar SC extracts obtained from vehicle-treated WT, G93A mice receiving the vehicle, and G93A treated with lead 2a. We observed that the mature CTSD/pro-CTSD ratio was slightly, but not significantly, reduced in SC extracts of vehicle-treated G93A mice when compared with WT litters. Possibly, we did not see a reduction of mature CTSD in total protein lysates because at day 100 glial cells of G93A mice express high amounts of mature CTSD (Figure 11E). However, levels of mature CTSD significantly increased in lead 2a-treated G93A mice (Figure 11E). We next analyzed CTSD levels in MNs by confocal microscopy. We observed a significant reduction of this protease in MNs of vehicle-treated G93A, while such levels increased in G93A mice treated with compound 2a (Figure 11F). Similarly, numbers of CTSD⁺ puncta in lumbar NeuN⁺ MNs were reduced in vehicle-treated G93A MNs, while they increased in compound 2a-treated mice (Figure 11F). These data suggest that the stabilization of the retromer complex mediated by lead 2a restores Sortilin and CI-MPR expression levels and improves the maturation of CTSD, thus restoring lysosome health in ALS mice.

To finally prove that compound 2a can increase the stabilization of the retromer complex we probed SCs extracts for VPS35 and VPS26. These experiments further confirmed the ability of 2a to increase VPS35 and VPS26b levels in G93A mice (Figure 12A-D).

Ubiquitinated inclusions accumulate in MNs of sALS and fALS patients and in lumbar SCs of ALS mice (Kabashi 2001). High levels of ubiquitinated proteins may represent an overwhelmed response of MNs to protein misfolding and aggregation, as well as a reduction of lysosome functionality. We characterized poly-ubiquitinated proteins in lumbar SCs protein extracts by 10% denaturating PAGE. We included in our experiments protein extracts from Neuro2a cells treated with the proteasome inhibitor Bortezomib (BTZ), able to increase poly-ubiquitination, as an experimental control (Figure 13A). Densitometry analysis of these blots showed that vehicle-treated G93A mice displayed abundant poly-ubiquitinated proteins. However, poly-ubiquitination was substantially reduced in mice treated with compound 2a (Figure 13A, B). We next scored ubiquitin MFI levels in ventral horn MNs of G93A mice. According to data obtained with thewestern blot, MFI levels of ubiquitin fluorescence in vehicle-treated G93A MNs were higher than levels scored in compound 2a-treated G93A mice, further confirming ours', and others' results (Saxesa 2009), (Figure 13C and D). As previously observed in Neuro2a cells, the overexpression of G93A plasmids affects the Golgi apparatus, therefore we asked whether similar alterations can occur in G93A mice by labeling lumbar sections for the cis-Golgi matrix protein GM130. We observed that a great proportion of MNs from vehicle-treated G93A mice displayed a reduction of GM130-covered areas, possibly indicating the fragmentation of this organelle (Figure 13E). However, this phenomenon was substantially reverted in MNs of compound 2a-treated G93A mice (Figure 13E).

SOD1 mutations increase the propensity of this protein to aggregate in cells (Johnston 2000). We checked whether compound 2a could reduce the accumulation of G93A aggregates in vivo. Starting at post-natal day 83, we injected G93A mice and WT littermates daily with compound 2a (10 mg/kg). We scored motor performances in both vehicle- and compound 2a-treated G93A mice until day 103. Mice receiving compound 2a displayed a general attenuation of motor deficits that confirmed our previous observations (Figure 14A). At day 103, we resolved SC protein extracts by blue-native polyacrylamide gel electrophoresis, and probing these gels with anti-SOD1 antibodies we observed high molecular weight smears in G93A mice that were completely absent in WT litters. The intensity of these high molecular weight entities was lowered in mice treated with lead 2a (Figure 14B, C). Altogether, retromer stabilization by compound 2a increases lysosomal homeostasis in vitro and in vivo, decreases poly ubiquitination of proteins, and therefore diminishes levels of G93A aggregation.

These results indicate that ubiquitinated proteins (Figure13A, B) and high molecular weight SOD1 aggregates (Figure 14B, C) are both reduced in compound 2a-treated mice. Besides increasing the functionality of lysosomes we hypothesised that compound 2a could also enhance the autophagy system. Therefore we used a pHfluorin-LC3-based assay to monitor the autophagy process in living Neuro2a cells. We have established in our laboratory a Neuro2a stable cell clone (7B11) that expresses the Microtubule-associated protein 1A/1B-light chain 3 (LC3) coupled with the ph-sensitive GFP/phlourin. This assay is based on flow cytometry and has been validated using a well-known autophagy inhibitor (Bafilomycin A1) and an activator (Torin-1) that allowed the establishment of the upper and the lower bounds of the assay activity (not shown). Furthermore, we validated the quality of our assay calculating the Z-factor and the Strictly standardized mean difference (SSMD) parameters using Bafilomycin A1 and Torin-1 (not shown).

Neuro2a-7B11 cells were incubated with increasing concentrations of compound 2a (0.1-100 µM) as well as with the control molecules Bafilomycin A1 and Torin-1. After 48 hours the GFP/phluorin signal, which is ph-sensitive, was measured by flow cytometry and fold changes calculated over untreated cells. As expected, we observed augmented fluorescence blocking the fusion of autophagosomes with lysosomes by Bafilomycin A1 treatment. On the other hand, fluorescence signals were significantly dampened by the mTORC1/2 inhibitor Torin 1 (Figure 14D). GFP/phluorin signals did not change in cells receiving 0.1 and 1 µM of compound 2a, while we observed a significant reduction of fluorescence levels in cells treated with 10 and 100µM (Figure 14D). Levels of fluorescence that we recorded in cells that received 100µM of compound 2a did not differ from levels measured in cells treated with 0.25µM of Torin 1. Therefore, we can envisage that part of the degradation of the ubiquitinated proteins that we observed in compound 2a-treated mice might involve a direct/indirect enhancement of the autophagy system.

We next determined whether a reduction of VPS35 levels in Neuro2a cells could affect lysosomal homeostasis, mimicking what we and others observed in the ALS mouse model (Cheng 2018). We transfected Neuro2a cells with plasmids encoding the Sh56 RNAi to downregulate VPS35. After 24 hours of starvation, we analyzed the lysosomes using the Lyso-tracker. We observed an enlarged and abnormal distribution of lysosomes in cells with VPS35 interference by Sh56 (Figure 15A-C). We next assessed that poly-ubiquitinated protein levels were doubled in Neuro2a cells receiving a VPS35-targeted RNAi, further suggesting that loss of retromer functionality alters lysosomal homeostasis, and therefore poly-ubiquitinated proteins' accumulation (Figure 15D, E). We asked whether G93A plasmids would also result in similar lysosomal alterations. We transfected Neuro2a cells with plasmids encoding G93A, with or without compound 2a (10 µM). After 24 hours of starvation, we monitored the lysosomes in such cells with the Lyso-tracker. Cells receiving only G93A plasmids showed enlarged lysosomes, although treatment with compound 2a significantly reverted this phenotype (Figure 15F, G).

We observed that the molecular structure featuring compound 2a is reminiscent of the patented compound CNI-1493 (N,N'-bis [3,5-bis [1(aminoiminomethyl) hydrazono]ethyl]phenyl]decanediamide tetrahydrochloride (CAS Reg. No. 164301-51-3)), (Figure 16A). CNI-1493 inhibits macrophage activation in the animal model of Multiple Sclerosis producing a general amelioration of clinical and pathological outcomes of these mice (Martiney, Rajan et al. 1998). Furthermore, the administration of CNI-1493 to G93A mice delayed the onset of the disease and increased the lifespan of mice (Dewil, dela Cruz et al. 2007). CNI-1493 suppresses the production of pro-inflammatory cytokines, belonging to the plethora of detrimental signals that accompany neurodegenerative processes in several disorders. In particular, CNI-1493 inhibits the expression of TNFα, IL-1β, IL-6 and macrophage inflammatory proteins 1α and 1β (Bianchi, Bloom et al. 1996). Obviously, the ability of this compound to modulate the retromer has never been assayed. We firstly tested whether CNI-1493 can increase VPS35 levels in Neuro2a treating cells with this compound for 48 hours at the concentration of 10µM. However, VPS35 levels did not increased in CNI-1493-treated cells, while such levels were increased by compound 2a (Figure 16B). We next assayed the ability of compound 2a to inhibit LPS-mediated TNFα expression in the murine macrophage-like cell line Raw 264.7. We followed experimental procedures published by Bianchi and colleagues that describe the ability of CNI-1493 to blunt the production of this cytokine (Bianchi, Bloom et al. 1996). Cells were incubated with CNI-1493 (10µM) or with compound 2a (10µM) for 1 hour and then we added the LPS (100 ng/ml) to media. We next collected supernatants (4 hours after the LPS stimulation) form cells for the evaluation of TNFα levels. Raw cells receiving LPS efficiently induced TNFα, while CNI-1493 significantly inhibited the expression of this cytokine (Figure 16C). On the other hand, raw cells receiving compound 2a did not show any reduction of TNFα, suggesting that 2a does not operate of the same molecular pathway of CNI-1493.

### Loss of VPS35 expression in post mortem ASL samples and in iPSCs-derived MNs from familiar ALS fibroblasts

We next investigated VPS35 in post mortem cerebral alpha-MNs located in the ventral horn of the human SC (Figure 17A). Immunoreactivity for VPS35 was substantially reduced in MNs derived from ALS specimens, confirming our earlier observations made in G93A mice (Figure 17B). Parallel sections probed for VPS26 revealed a substantial reduction of this protein in ALS samples (Figure17C, D).

We next generated iPSC lines from fALS patients carrying three SOD1 gene variants (ALS 8: p.Asn65Ser; ALS 13: p.Leu144Phe; ALS 27: p.Asp97Asn), and from three age and sex-matched healthy volunteers. Efficient reprogramming was assessed labelling cultures for OCT3/4, SOX2, NANOG, TRA1-60 and SSEA4 (not shown). According to a published protocol we induced iPSCs lines to acquire the phenotype of caudal neuroepithelial progenitors expressing OLIG2⁺PAX6⁻ (Du 2015). We differentiated these progenitors to acquire the OLIG2⁻ISLET1⁺ cell phenotype. We scored the percentages of ISLET1⁺ out the total number of nuclei, and we observed similar rates of cell differentiation among controls and ALS samples (controls: 81.6%±9; ALS: 81.8%±12). Using immunofluorescence and WBs, we observed at day 33 a substantial reduction of VPS35 levels in ISLET1⁺ MNs deriving from ALS patients (Figure 17E, F). We next selected one control cell line (#8) and one ALS cell line (ALS 27) to apply our compound 2a. Upon we have established OLIG2⁺PAX6⁻progenitors we started to differentiate these cells including lead 2a (10 µM, 6 days) in the culture medium. Cells were labelled for ISLET1 and VPS35 and by confocal microscopy, and we scored VPS35⁺ puncta with or compound 2a. ALS 27 cells displayed less VPS35⁺ puncta than controls, albeit both controls and ALS significantly increased this number if treated with lead 2a (Figure 17G). We next confirmed the ability of compound 2a to increase VPS35 protein levels by WB (Figure 17H). These results suggest that 2a increases VPS35 levels and therefore can be used to stabilize the human retromer complex

### Conclusion

The retromer complex is highly conserved in eukaryotes and it has been associated to several neurodegenerative disorders, including PD and AD, although, never investigated in ALS. Using G93A transgenic mice we observed that CRC protein levels are substantially reduced in ALS motor neurons. Interestingly, such reduction occurs before the appearance of any signs of the disease and, above all before motor neurons degeneration. Parallel experiments performed on post mortem SCs from ALS patients further indicate the reduction of VPS35 and VPS26 levels in ventral horn bona fide motor neurons. These results suggest that retromer failure can anticipate the degeneration of these cells and therefore strategies fostering retromer stabilization can be neuroprotective in ALS. Compound 2a is able to significantly increase VPS35 levels and, as consequence, the retromer functionality. Such compound rescues Golgi fragmentation *in vitro* and *in vivo* and, above all, reduced rates of cell death in Neuro2a cell line receiving G93A plasmids. In a preclinical experiment in which we injected G93A mice with compound 2a, we observed that by fostering retromer functionality *in vivo* we were able to reduce MNs degeneration, demyelination and peripheral fibers loss. Consequently, G93A mice receiving compound 2a display increased locomotion performances. Furthermore, the increased locomotion performacies were mirrored by increased levels of VPS35 and VPS26 that we scored in MNs as well as by increased levels of two retromer cargos, namely CI-MPR and Sortilin. We also observed a substantial increase of mature CTSD in spinal cords of G93A treated with compound 2a. The genral increase of CTSD and possibly of other lysosomial enzymes decreased the accumulation of polyubiquitianted proteins, a pathological hallmark of ALS. Interestingly, we also observed a reduction of aggregated SOD1 in these mice. These findings open a new avenue for the treatment of ALS.

### Synthetic methods

**General Procedures.** ¹H NMR spectra were recorded on a Bruker Avance 400MHz instrument in CDCl₃, CD₃OD or D₂O as solvent at 400 MHz. ¹³C NMR spectra were recorded in CDCl₃, CD₃OD or D₂O as solvent at 101 MHz. Coupling constants are given in Hertz and are rounded to the nearest 0.1 Hz. LC-MS data were collected with a Waters Acquity^{™} Ultra performance LC equipped with an Acquity UPLC^{™} HSS T3 column (2.1 mm x 50 mm, 1.8 µm) and a SQD detector. Purifications were carried out either by flash chromatography on silica gel (particle size 60 µm, 230-400 mesh), on Kieselgel, or by Biotage^{™} flash chromatography [Biotage columns Si-25-M (150 x 25 mm; silica gel (40-63 µm), flow rate 25 mL/min)], or by Biotage^{™} C₁₈ reverse phase chromatography [Biotage column C₁₈HS (150 x 25 mm; KP-C₁₈-HS (35-70 µm), flow rate 25mL/min)]. Some final compounds were purified by C₁₈ reverse phase semi-preparative HPLC using a Waters X-Bridge column (19 mm x 15.0 cm, 5 µm). Melting points were determined with a Stuart Scientific SMP3 melting point apparatus. Solvents were distilled and dried according to standard procedures, and reactions requiring anhydrous conditions were performed under nitrogen or argon atmosphere.

**Substituted isophthalic acids, reduction to alcohols, general procedure A (8c, 8g-j, 8p).** A suspension of substituted isophthalic acid (2.4 - 3.3 mmoles, 1 eq.) in anhydrous THF (≈1 mL / mmol) was vigorously stirred and cooled to 0°C under nitrogen atmosphere. A solution of 1M BH₃ in THF (9.6-13.2 mL, 4 eqs.) was added dropwise in 1 hr. The reaction mixture was allowed to warm slowly to rt, and was stirred for additional 48 hrs. MeOH (7-10 mL) was added dropwise to the reaction mixture and the solvent was evaporated under reduced pressure. Addition of MeOH and evaporation was repeated (7-10 mL x 3 times). Then, the crude was dissolved in EtOAc (40 mL), washed with saturated NaHCO₃ (2 x 20 mL) and brine (10 mL). The organic layer was dried with anhydrous Na₂SO₄, filtered and evaporated at reduced pressure. The resulting solid was purified by flash chromatography on silicagel (eluant mixture: n-hexane/EtOAc) to afford the corresponding pure substituted **1,3-bis-benzylic alcohol.**

**Substituted 1,3-bis-benzylic alcohols, oxidation to aldehydes, general procedure B (7c, 7g-j, 7k-p).** Manganese dioxide (2.5-10 mmoles, 5 eqs.) was added under vigorous stirring to a solution of a substituted 1,3-bis-benzylic alcohol (0.5-2 mmoles, 1 eq.) in chloroform (≈5 mL/mmol), and the reaction mixture was heated at reflux. After overnight reflux/stirring, the reaction mixture was cooled to rt, and filtered through a Celite pad. The pad was thoroughly washed with CH₂Cl₂ (40 mL), the combined organic phase was concentrated *in vacuo,* and the residue was purified by flash chromatography on silicagel (eluant mixture: n-hexane/EtOAc) to afford the corresponding pure substituted isophthalaldehyde.

**Substituted isophthalaldehydes, condensation with aminoguanidine, general procedure C (2a-z,).** Aminoguanidine hydrochloride (1-2 mmoles, 1 eq.) and aqueous 1N HCl (3-5 drops, catalytic) were sequentially added to a warm, vigorously stirred solution of a substituted isophthalaldehyde (0.5-1 mmoles, 1 eq.) in absolute EtOH (≈10 mL/mmol). The reaction mixture was refluxed for 2-8 hours, with TLC monitoring (8:2 CH₂Cl₂:MeOH with a few AcOH drops). The reaction mixture was then cooled to rt, the solid product was filtered, washed with cold EtOH (10 mL), with Et₂O (10 mL), and dried in vacuum to yield the corresponding pure N-unsubstituted 1,3-bis-amino guanidine phenyl hydrazine as a dihydrochloride salt.

**Substituted isophthalaldehydes, condensation with N-substituted aminoguanidines, general procedure D (2a-z).** A N-substituted aminoguanidine salt (0.6-1.8 mmoles, 1 eq.) was added to a warm, vigorously stirred solution of substituted isophthalaldehyde (0.3-0.9 mmoles, 1 eq.) in absolute EtOH (≈7.5 mL/mmol). The reaction mixture was refluxed for 8 hours, with TLC monitoring (8:2 CH₂Cl₂:MeOH with a few AcOH drops). The reaction mixture was then cooled to rt, the solid product was filtered, washed with cold EtOH (10 mL), with Et₂O (10 mL), and dried in vacuum to yield the corresponding pure N-substituted 1,3-bis-amino guanidine phenyl hydrazine as a dihydrohalide salt (n some cases, a preparative HPLC separation was needed).

**1,3-Bis-amino guanidine phenyl hydrazone, dihydrochloride salt (2a).** The title compound (280 mg, 0.88 mmoles, **88%** yield, ≥95% purity, white solid) was prepared as a dihydrochloride salt from isophthalaldehyde (134 mg, 1 mmoles, 1.0 eq.) and aminoguanidine hydrochloride (221 mg, 2 mmoles, 1 eq.) in absolute ethanol (10 mL), following the experimental protocol of **general procedure C. ¹H_NMR (400 MHz, DMSO-d6):** δ(ppm) 12.26 (bs, 2H, NH), 8.32 (s, 1H, H2), 8.22 (s, 2H, H1', H3'), 8.15-7.58 (bs, 6H, NH), 7.96 (d, J = 7.6 Hz, 2H, H4), 7.53 (t, J = 7.6 Hz, 1H, H5). **¹³C_NMR (100 MHz, D₂O):** δ(ppm) 154.7, 147.3, 133.2, 129.2, 126.6. **MS (ESI⁺):** *m*/*z* 247.30 [M+H⁺]. Calculated MS, C₁₀H₁₄N₈: 246.28.

**2-Bromo-1,3-bis-aminoguanidine phenyl hydrazone, dihydrochloride salt (2b).** The title compound (280 mg, 0.88 mmoles, **88%** yield, ≥95% purity, white solid) was prepared as a dihydrochloride salt from 2-bromo isophthalaldehyde (197 mg, 0.93 mmoles, 1.0 eq.) and aminoguanidine hydrochloride (205 mg, 1.86 mmoles, 1 eq.) in absolute ethanol (9 mL), following the experimental protocol of **general procedure C. ¹H_NMR (400 MHz, DMSO-d6):** δ(ppm) 12.22 (bs, 2H, NH), 8.65 (s, 2H, H1', H3'), 8.38 (d, J = 7.7 Hz, 2H, H4), 8.30-7.60 (bs, 6H, NH), 7.51 (t, J = 7.7 Hz, 1H, H5). **¹³C_NMR (100 MHz, D₂O):** δ(ppm) 155.6, 146.7, 132.9, 130.0, 126.9. **MS (ESI⁺):** *m*/*z* 325.29 and 327.29 [M+H⁺]. Calculated MS, C₁₀H₁₃BrN₈: 324.18 and 326.18.

**1,3-Bis-(hydroxymethyl)-4-bromobenzene (8c).** The title compound (455 mg, 2.10 mmol, **86%** yield) was prepared from 4-bromoisophthalic acid (590 mg, 2.41 mmoles, 1.0 eq.) and 1M BH₃ in THF (10 mL, 10.00 mmoles, ≈4 eqs.) in THF (2.5 mL), following the experimental protocol of **general procedure A. ¹H_NMR (400 MHz, DMSO-d6):** δ(ppm) 7.39 (m, 3H, H2, H5, H6), 5.39 (t, J = 5.6 Hz, 2H, OH), 4.52 (d, J 5.6 Hz, 4H, H1', H3'). **MS (ESI⁺):** *m*/*z* 218.12 [M+H⁺]. Calculated MS, C₈H₉BrO₂: 217.04.

**4-Bromo isophthalaldehyde (7c).** The title compound (108 mg, 0.51 mmol, **87%** yield) was prepared from **8c** (128 mg, 0.59 mmoles, 1.0 eq.) and manganese dioxide (257 mg, 2.95 mmoles, 5 eqs.) in chloroform (3 mL), following the experimental protocol of **general procedure B. ¹H_NMR (400 MHz, DMSO-d6):** δ(ppm) 10.40 (s, 1H, H1'), 10.06 (s, 1H, H3'), 8.42 (d, J = 2.09 Hz, 1H, H2), 7.98 (dd, J = 2.09 Hz, J = 7.88 Hz, 1H, H6), 7.72 (d, J = 7.88 Hz, 1H, H5). MS (ESI⁺): *m*/*z* 214.16 [M+H⁺]. Calculated MS, C₈H₅BrO₂: 213.01.

**4-Bromo-1,3-bis-amino guanidine phenyl hydrazone, dihydrochloride salt (2c).** The title compound (178 mg, 0.44 mmoles, **88%** yield, ≥95% purity, white solid) was prepared as a dihydrochloride salt from 4-bromo isophthalaldehyde (108 mg, 0.51 mmoles, 1.0 eq.) and aminoguanidine hydrochloride (112 mg, 1.02 mmoles, 1 eq.) in absolute ethanol (5 mL), following the experimental protocol of **general procedure C. ¹H_NMR (400 MHz, DMSO-d6):** δ(ppm) 12.40 (bs, 2H, NH), 8.58 (d, J = 2.0 Hz, 1H, H2), 8.54 (s, 1H, H1'), 8.20 (s, 1H, H3'), 8.20-7.60 (bs, 6H, NH), 7.98 (dd, J = 2.0 Hz, J = 8.4 Hz, H6), 7.78 (d, J = 8.4 Hz, 1H, H5). **¹³C_NMR (100 MHz, DMSO-d6):** δ(ppm) 157.0, 156.8, 146.3, 145.8, 134.8, 134.7, 133.8, 131.1, 128.5, 126.5. **MS (ESI⁺):** *m*/*z* 315.33 and 317.33 [M+H⁺]. Calculated MS, C₁₀H₁₃BrN₈: 324.18 and 326.18.

**1-Hydroxy-2,4-bis-aminoguanidine phenyl hydrazone, dihydrochloride salt (2d).** The title compound (224 mg, 0.67 mmoles, 77% yield, ≥95% purity, white solid) was prepared as a dihydrochloride salt from **7d** (132 mg, 0.87 mmoles, 1.0 eq.) and aminoguanidine hydrochloride (193 mg, 1.75 mmoles, 1 eq.) in absolute ethanol (8 mL), following the experimental protocol of **general procedure C. ¹H_NMR (400 MHz, DMSO-d6):** δ(ppm) 12.05 (bs, 2H, NH), 10.95 (bs, 1H, OH), 8.52 (s, 1H, H, H1'), 8.48 (d, J = 1.8 Hz, 1H, H2), 8.12 (s, 1H, H3'), 8.20-7.40 (bs, 6H, NH), 7.95 (dd, J = 8.6 Hz, J = 1.8 Hz, 1H, H6), 7.05 (d, J = 8.6 Hz, 1H, H5). **¹³C_NMR (100 MHz, DMSO-d6):** δ(ppm) 158.8, 155.7, 155.4, 148.4, 147.4, 131.6, 129.4, 126.1, 119.3, 117.7. **MS (ESI⁺):** *m*/*z* 263.37 [M+H⁺]. Calculated MS, C₁₀H₁₄N₈O: 262.28.

**4-Methoxy isophthalaldehyde (7e).** MeI (0.13 mL, 2 mmoles, 1 eq.) was added to a stirred mixture of 4-hydroxy isophthalaldehyde **7d** (150 mg, 1 mmol, 1 eq.) and K₂CO₃ (420 mg, 3 mmoles, 1.5 eqs.) in dry DMF (5 mL) under a nitrogen atmosphere. The reaction mixture was stirred at r.t. for 24 hrs, then the solvent was removed at reduced pressure. The crude was dissolved in EtOAc (30 mL), washed with 5% aqueous NaOH (3 x 20 mL) and with brine (10 mL). The combined organic layer was dried on anhydrous Na₂SO₄, filtered and concentrated at reduced pressure. 4-Methoxy isophthalaldehyde **7e** (105 mg, 0.60 mmoles, **60%** yield) was obtained as a white solid pure enough to be used without further purification. **¹H_NMR (400 MHz, CDCl₃):** δ(ppm) 10.50 (s, 1H, H1'), 10.01 (s, 1H, H3'), 8.35 (d, J = 2.0 Hz, 1H, H2), 8.12 (dd, J = 2.0 Hz, J = 9.1 Hz, 1H, H6), 7.15 (d, J = 9.1 Hz, 1H, H5), 4.00 (s, 3H, OMe). **MS (ESI⁺):** *m*/*z* 179.21 [M+H⁺]. Calculated MS, C₉H₈NO₃: 178.17.

**1-Methoxy-2,4-bis-amino guanidine phenyl hydrazone, dihydrochloride salt (2e).** The title compound (126 mg, 0.36 mmoles, **79%** yield, ≥95% purity, white solid) was prepared as a dihydrochloride salt from 4-methoxy isophthalaldehyde (75 mg, 0.46 mmoles, 1.0 eq.) and aminoguanidine hydrochloride (101 mg, 0.92 mmoles, 1 eq.) in absolute ethanol (4.5 mL), following the experimental protocol of **general procedure C. ¹H_NMR (400 MHz, DMSO-d6):** δ(ppm) 12.12 (bs, 2H, NH), 8.51 (s, 1H, H1'), 8.49 (s, 1H, H2), 8.19 (s, 1H, H3'), 8.20-7.40 (m, 6H, NH), 8.00 (d, J = 6.6 Hz, 1H, H6), 7.12 (d, J = 6.6 Hz, 1H, H5), 3.92 (s, 3H, OMe). **¹³C_NMR (100 MHz, DMSO-d6):** δ(ppm) 160.1, 156.9, 147.2, 142.7, 132.2, 127.4, 127.0, 123.0, 114.2, 56.2. **MS (ESI⁺):** *m*/*z* 277.44 [M+H⁺]. Calculated MS, C₁₁H₁₆N₈O: 276.31.

**4-(n-Butoxy) isophthalaldehyde (7f).** n-BuBr (0.22 mL, 2 mmoles, 1 eq.) was added to a stirred mixture **of 7d** (150 mg, 1 mmol, 1 eq.) and K₂CO₃ (420 mg, 3 mmoles, 1.5 eqs.) in dry DMF (5 mL) under a nitrogen atmosphere. The reaction mixture was heated to 85°C and stirred for 24 hrs, then cooled at r.t. After solvent removal at reduced pressure, the crude was dissolved in EtOAc (30 mL), washed with 5% aqueous NaOH (3 x 20 mL) and with brine (10 mL). The combined organic layer was dried on anhydrous Na₂SO₄, filtered and concentrated at reduced pressure. 4-(n-Butoxy) isophthalaldehyde **7f** (155 mg, 0.75 mmoles, **75%** yield) was obtained as a white solid pure enough to be used without further purification. **¹H_NMR (400 MHz, CDCl₃):** δ(ppm) 10.50 (s, 1H, H'), 10.00 (s, 1H, H3'), 8.35 (d, J = 2.0Hz, 1H, H2), 8.12 (dd, J = 2.0 Hz, J = 9.1 Hz, 1H, H6), 7.15 (d, J = 9.1 Hz, 1H, H5), 4.23 (t, J = 2.4 Hz, 2H, H4'), 1.91 (m, 2H, H4''), 1.56 (m, 2H, H4‴), 1.04 (t, J = 7.8 Hz, 3H, H4ʺʺ). **MS (ESI⁺):** *m*/*z* 221.35 [M+H⁺]. Calculated MS, C₁₂H₁₄NO₃: 220.23.

**1-(n-Butoxy)-2,4-bis-aminoguanidine phenyl hydrazone, dihydrochloride salt (2f).** The title compound (166 mg, 0.42 mmoles, **80%** yield, ≥95% purity, white solid) was prepared as a dihydrochloride salt from **7f** (110 mg, 0.53 mmoles, 1.0 eq.) and aminoguanidine hydrochloride (118 mg, 1.06 mmoles, 1 eq.) in absolute ethanol (5 mL), following the experimental protocol of **general procedure C. ¹H_NMR (400 MHz, DMSO-d6):** δ(ppm) 12.12 (bs, 2H, NH), 8.51 (s, 2H, H1', H1), 8.16 (s, 1H, H3'), 8.20-7.40 (bs, 6H, NH), 8.00 (d, J = 8.7 Hz, 1H, H6), 7.12 (d, J = 8.7 Hz, 1H, H5), 4.15 (t, J = 2.4 Hz, 2H, H4'), 1.80 (m, 2H, H4''), 1.52 (m, 2H, H4‴), 0.99 (t, J = 7.8 Hz, 3H, H4ʺʺ). **¹³C_NMR (100 MHz, DMSO-d6):** δ(ppm) 160.1, 156.9, 147.2, 142.7, 132.2, 127.4, 127.0, 123.0, 114.2, 69.1, 31.3, 19.3, 14.3. **MS (ESI⁺):** *m*/*z* 319.66 [M+H⁺]. Calculated MS, C₁₄H₂₂N₈O: 318.38.

**1,3-bis-(hydroxymethyl)-5-methyl benzene (8g).** The title compound (410 mg, 2.70 mmol, **81%** yield) was prepared from 5-methyl isophthalic acid (600 mg, 3.33 mmoles, 1.0 eq.) and 1M BH₃ in THF (13.3 mL, 13.3 mmoles, 4 eqs.) in THF (3 mL), following the experimental protocol of **general procedure A. ¹H_NMR (400 MHz, CDCl₃):** 6(ppm) 6.96 (s, 1H, H2), 6.80 (s, 2H, H4), 4.62 (s, 4H, H1', H3'), 3.81 (s, 3H, Me), 2.11 (s, 2H, OH). **MS (ESI⁺):** *m*/*z* 153.21 [M+H⁺]. Calculated MS, C₉H₁₂O₂: 152.19.

**5-Methyl isophthalaldehyde (7g).** The title compound (151 mg, 1.02 mmol, 77% yield) was prepared from **8g** (200 mg, 1.32 mmoles, 1.0 eq.) and manganese dioxide (575 mg, 6.60 mmoles, 5 eqs.) in chloroform (6.5 mL), following the experimental protocol of **general procedure B. ¹H_NMR (400 MHz, CDCl₃):** δ(ppm) 10.15 (s, 2H, CHO), 8.25 (s, 1H, H2), 8.00 (s, 2H, H4), 2.53 (s, 3H, Me). **MS (ESI⁺):** *m*/*z* 149.29 [M+H⁺]. Calculated MS, C₉H₈O₂: 148.16.

**5-Methyl-1,3-bis-aminoguanidine phenyl hydrazone, dihydrochloride salt (2g).** The title compound (179 mg, 0.54 mmoles, 77% yield, ≥95% purity, white solid) was prepared as a dihydrochloride salt from 7g (104 mg, 0.70 mmoles, 1.0 eq.) and aminoguanidine hydrochloride (155 mg, 1.40 mmoles, 1 eq.) in absolute ethanol (7 mL), following the experimental protocol of **general procedure C. ¹H_NMR (400 MHz, DMSO-d6):** δ(ppm) 12.25 (bs, 2H, NH), 8.21 (s, 2H, H1', H3'), 8.10 (s, 1H, H2), 8.00-7.50 (bs, 6H, NH), 7.82 (s, 2H, H4), 2.41 (s, 3H, Me). **¹³C_NMR(100 MHz, DMSO-d6):** δ(ppm) 156.9, 147.4, 139.9, 135.2, 130.9, 125.5, 21.4. **MS (ESI⁺):** *m*/*z* 261.45 [M+H⁺]. Calculated MS, C₁₁H₁₆N₈: 260.28.

**1,3-bis-(hydroxymethyl)-5-bromo benzene (8h).** The title compound (475 mg, 2.19 mmol, **88%** yield) was prepared from 5-bromo isophthalic acid (610 mg, 2.49 mmoles, 1.0 eq.) and 1M BH₃ in THF (10 mL, 10 mmoles, ≈4 eqs.) in THF (2.5 mL), following the experimental protocol of **general procedure A. ¹H_NMR (400 MHz, acetone-d6):** δ(ppm) 7.43 (s, 2H, H4), 7.28 (s,1H, H2), 4.67 (s, 4H, H1', H3'), 1.79 (s, 2H, OH). **MS (ESI⁺):** *m*/*z* 217.20 and 219.20 [M+H⁺]. Calculated MS, C₈H₉BrO₂: 216.15 and 218.15.

**5-Bromo isophthalaldehyde (7h).** The title compound (97 mg, 0.45 mmol, **76%** yield) was prepared from **8h** (128 mg, 0.59 mmoles, 1.0 eq.) and manganese dioxide (257 mg, 2.95 mmoles, 5 eqs.) in chloroform (3 mL), following the experimental protocol of **general procedure B. ¹H_NMR (400 MHz, CDCl₃):** δ(ppm) 10.06 (s, 2H, CHO), 8.30 (t, J = 1.4 Hz, 1H, H2), 8.26 (d, J = 1.4 Hz, 2H, H4). **MS (ESI⁺):** *m*/*z* 213.24 and 215.24 [M+H⁺]. Calculated MS, C₈H₅BrO₂: 212.12 and 214.12.

**5-Bromo-1,3-bis-aminoguanidine phenyl hydrazone, dihydrochloride salt (2h).** The title compound (133 mg, 0.33 mmoles, **91%** yield, ≥95% purity, white solid) was prepared as a dihydrochloride salt from **7h** (75 mg, 0.35 mmoles, 1.0 eq.) and aminoguanidine hydrochloride (78 mg, 0.70 mmoles, 1 eq.) in absolute ethanol (3.5 mL), following the experimental protocol of **general procedure C. ¹H_NMR (400 MHz, DMSO-d6):** δ(ppm) 12.31 (bs, 2H, NH), 8.27 (d, J = 1.2 Hz, 2H, H4), 8.22 (t, J = 1.2 Hz, 1H, H2), 8.17 (s, 2H, H1', H3'), 8.20-7.60 (bs, 6H, NH). **¹³C_NMR (100 MHz, DMSO-d6):** δ(ppm) 156.9, 145.8, 137.5, 132.0, 127.8, 124.1. **MS (ESI⁺):** *m*/*z* 327.45 and 329.45 [M+H⁺]. Calculated MS, C₁₀H₁₃BrN₈: 324.25 and 326.25.

**3,5-bis-(hydroxymethyl)-1-methoxybenzene (8i).** The title compound (377 mg, 2.24 mmol, **80%** yield) was prepared from 5-methoxy isophthalic acid (550 mg, 2.80 mmoles, 1.0 eq.) and 1M BH₃ in THF (11.2 mL, 11.2 mmoles, 4 eqs.) in THF (2.8 mL), following the experimental protocol of **general procedure A. ¹H_NMR (400 MHz, CDCl₃):** δ(ppm) 6.96 (s, 1H, H2), 6.80 (s, 2H, H4), 4.62 (s, 4H, H1', H3'), 3.81 (s, 3H, Me), 2.11 (s, 2H, OH). **MS (ESI⁺):** *m*/*z* 169.21 [M+H⁺]. Calculated MS, C₉H₁₂O₃: 168.19.

**5-methoxy isophthalaldehyde (7i).** The title compound (297 mg, 1.81 mmol, **92%** yield) was prepared from **8i** (330 mg, 1.97 mmoles, 1.0 eq.) and manganese dioxide (825 mg, 9.95 mmoles, 5 eqs.) in chloroform (10 mL), following the experimental protocol of **general procedure B. ¹H_NMR (400 MHz, CDCl₃):** δ(ppm) 10.30 (s, 2H, H1', H3'), 7.96 (s, 1H, H2), 7.61 (s, 2H, H4), 3.99 (s, 3H, Me). **MS (ESI⁺):** *m*/*z* 165.29 [M+H⁺]. Calculated MS, C₉H₈O₃: 164.16.

**1-Methoxy-3,5-bis-aminoguanidine phenyl hydrazone, dihydrochloride salt (2i).** The title compound (161 mg, 0.46 mmoles, **81%** yield, ≥95% purity, white solid) was prepared as a dihydrochloride salt from **7i** (93 mg, 0.57 mmoles, 1.0 eq.) and aminoguanidine hydrochloride (125 mg, 1.14 mmoles, 1 eq.) in absolute ethanol (6 mL), following the experimental protocol of **general procedure C. ¹H_NMR (400 MHz, DMSO-d6):** δ(ppm) 12.25 (bs, 2H, NH), 8.22-7.61 (bs, 6H, NH), 8.17 (s, 2H, H1', H3'), 7.83 (t, J = 1.08 Hz, 1H, H2), 7.59 (d, J = 1.08 Hz, 2H, H4), 3.82 (s, 3H, OMe). **¹³C_NMR (100 MHz, DMSO-d6):** δ(ppm) 160.3, 155.9, 146.3, 135.7, 120.6, 114.7, 56.2. **MS (ESI⁺):** *m*/*z* 277.47 [M+H⁺]. Calculated MS, C₁₁H₁₆N₈O: 276.30.

**3,5-bis-(hydroxymethyl)-1-nitro benzene (8j).** The title compound (504 mg, 2.75 mmol, **83%** yield) was prepared from 5-nitro isophthalic acid (700 mg, 3.32 mmoles, 1.0 eq.) and 1M BH₃ in THF (13.2 mL, 13.2 mmoles, 4 eqs.) in THF (3 mL), following the experimental protocol of **general procedure A. ¹H_NMR (400 MHz, DMSO-d6):** δ(ppm) 8.16 (s, 2H, H4), 7.72 (s, 1H, H2), 4.83 (s, 4H, H1', H3'). **MS (ESI⁺):** *m*/*z* 184.24 [M+H⁺]. Calculated MS, C₈H₉NO₄: 183.17.

**5-nitro isophthalaldehyde (7j).** The title compound (94 mg, 0.53 mmol, **78%** yield) was prepared from **8j** (123 mg, 0.67 mmoles, 1.0 eq.) and manganese dioxide (291 mg, 3.395 mmoles, 5 eqs.) in chloroform (4 mL), following the experimental protocol of **general procedure B. ¹H_NMR (400 MHz, CDCl₃):** δ(ppm) 10.21 (s, 2H, H1', H3'), 8.96 (s, 2H, H4), 8.72 (s, 1H, H2). **MS (ESI⁺):** *m*/*z* 180.29 [M+H⁺]. Calculated MS, C₈H₅NO₄: 179.14.

**1-Nitro-3,5-bis-aminoguanidine phenyl hydrazone, dihydrochloride salt (2j).** The title compound (154 mg, 0.42 mmoles, **80%** yield, ≥95% purity, white solid) was prepared as a dihydrochloride salt from **7j** (94 mg, 0.53 mmoles, 1.0 eq.) and aminoguanidine hydrochloride (117 mg, 1.06 mmoles, 1 eq.) in absolute ethanol (5 mL), following the experimental protocol of **general procedure C. ¹H_NMR (400 MHz, DMSO-d6):** δ(ppm) 12.44 (bs, 2H, NH), 8.80 (d, J = 1.6 Hz, 2H, H4), 8.75 (t, J = 1.6 Hz, 1H, H2), 8.34 (s, 2H, H1', H3'), 8.25-7.75 (bs, 6H, NH). **¹³C_NMR (100 MHz, DMSO-d6):** δ(ppm) 156.0, 149.4, 144.5, 136.3, 132.7, 123.2. **MS (ESI⁺):** *m*/*z* 292.47 [M+H⁺]. Calculated MS, C₁₀H₁₃N₉O₂: 291.29.

**1,3-Bis-(hydroxymethyl)-5-phenylbenzene (8k).** A 2M aqueous Na₂CO₃ solution (1.86 mL, 3.72 mmoles, 3 eqs.) was added under vigorous stirring at r.t. under nitrogen atmosphere to a mixture of **8h** (270 mg, 1.24 mmoles, 1 eq.), phenylboronic acid (177 mg, 1.45 mmoles, 1.2 eqs.) and Pd(PPh₃)₄ (143 mg, 0.124 mmoles, 0.1 eqs.) in 1,4-dioxane (8 mL). The mixture was heated at reflux and stirred for 6 hrs. After cooling to r.t., the reaction mixture was then diluted with EtOAc (40 mL), washed with sat. aq. NaHCO₃ (20 mL) and brine (15 mL). The organic layer was concentrated at reduced pressure, and the crude product was purified by flash chromatography (eluant: 75:25 n-hexane/EtOAc) to yield pure 1,3-bis-(hydroxymethyl)-5-phenylbenzene **8k** (220 mg, 1.02 mmoles, **86%** yield) as a white solid. **¹H_NMR (400 MHz, CDCl₃):** δ(ppm) 7.62 (d, J = 7.2 Hz, 2H, H2"), 7.54 (s, 2H, H4), 7.46 (t, J = 7.2 Hz, 2H, H3''), 7.38 (m, 2H, H2, H4''), 4.79 (s, 4H, H1', H3'), 1.86 (bs, 2H, OH). **MS (ESI⁺):** *m*/*z* 215.43 [M+H⁺]. Calculated MS, C₁₄H₁₄O₂: 214.26.

**5-Phenyl isophthalaldehyde (7k).** The title compound (135 mg, 0.64 mmol, **81%** yield) was prepared from **8k** (170 mg, 0.79 mmoles, 1.0 eq.) and manganese dioxide (343 mg, 3.95 mmoles, 5 eqs.) in chloroform (4 mL), following the experimental protocol of **general procedure B. ¹H_NMR (400 MHz, CDCl₃):** δ(ppm) 10.21 (s, 2H, H1', H3'), 8.39 (dd, J = 1.5 Hz, 2H, H4), 8.37 (t, J = 1.5 Hz, 1H, H2), 7.71 (dd, J = 8.6 Hz, J = 1.5 Hz, 2H, H2"), 7.54 (dt, J = 8.6 Hz, J = 7.3 Hz, 2H, H3"), 7.48 (dt, J = 7.3 Hz, J = 1.5 Hz, 1H, H4"). **MS (ESI⁺):** *m*/*z* 211.31 [M+H⁺]. Calculated MS, C₁₄H₁₀O₂: 210.23.

**5-Phenyl-1,3-bis-amino guanidine phenyl hydrazone, dihydrochloride salt (2k).** The title compound (190 mg, 0.48 mmoles, **79%** yield, ≥95% purity, white solid) was prepared as a dihydrochloride salt from **7k** (127 mg, 0.61 mmoles, 1.0 eq.) and aminoguanidine hydrochloride (134 mg, 1.22 mmoles, 1 eq.) in absolute ethanol (6 mL), following the experimental protocol of **general procedure C. ¹H_NMR (400 MHz, DMSO-d6):** δ(ppm) 12.35 (bs, 2H, NH), 8.30-8.25 (m, 5H, H2, H4, H1', H3'), 8.20-7.60 (bs, 6H, NH), 7.83 (d, J = 8.4 Hz, 2H, H2"), 7.53 (t, J = 8.4 Hz, 2H, H3''), 7.44 (t, J = 8.4 Hz, 1H, H4"). **¹³C_NMR (100 MHz, DMSO-d6):** δ(ppm) 156.0, 146.4, 1411.7, 139.3, 135.1, 129.4, 128.5, 127.5, 126.4. **MS (ESI⁺):** *m*/*z* 323.53 [M+H⁺]. Calculated MS, C₁₆H₁₈N₈: 322.38.

**3,5-Bis-(t-butyldimethylsilyloxymethyl)-1-nitro benzene (10j).** 1H-imidazole (500 mg, 7.35 mmoles, 1.5 eqs.) and **8j** (450 mg, 2.45 mmoles, 1 eq.) were dissolved in CH₂Cl₂ (12 mL) at r.t under stirring. Then the solution was cooled at 0°C, and t-butyldimethylsilyl chloride (930 mg, 6.12 mmoles, 1.25 eq.) was added in a single portion. The reaction mixture was slowly warmed to rt and stirred for additional 3 hrs. The reaction mixture was then diluted with CH₂Cl₂ (15 mL), washed with 5% aqueous citric acid (15 mL), 5% aqueous NaOH (15 mL) and brine (15 mL). The combined organic layer was dried on anhydrous Na₂SO₄, filtered and evaporated at reduced pressure. Crude 3,5-bis-(t-butyldimethylsilyloxymethyl)-1-nitro benzene **10j** (900 mg, 2.19 mmoles) was obtained as a yellow oil, used without further purification in the next reaction step. **¹H_NMR (400 MHz, CDCl₃):** δ(ppm) 8.00 (s, 2H, H4), 7.62 (s, 1H, H2), 4.81 (s, 4H, H1', H3'), 0.98 (s, 18H, t-Bu), 0.1 (s, 12H, Me). **MS (ESI⁺):** *m*/*z* 412.84 [M+H⁺]. Calculated MS, C₂₀H₃₇Si₂NO₄: 411.69.

**3,5-bis-(t-butyldimethylsilyloxymethyl)-1-amino benzene (11).** 10% Pd/C (80 mg, catalytic) was added to a solution of **10j** (797 mg, 1.93 mmol, 1 eq.) in absolute EtOH (10 mL), and the reaction mixture was vigorously stirred at r.t. under hydrogen atmosphere for 24 hrs. The reaction mixture was then filtered through a path of celite using MeOH (50 mL) for repeated washings. After concentration at reduced pressure, the crude was purified by flash chromatography on silica gel (eluant mixture: neat CH₂Cl₂ to 95:5 CH₂Cl₂/EtOAc), to afford pure 3,5-bis-(t-butyldimethylsilyloxymethyl)-1-amino benzene **11** (591 mg, 1.559 mmoles, **78%** yield for two steps) as a light yellow oil. **¹H_NMR (400 MHz, CDCl₃):** δ(ppm) 6.68 (s, 1H, H2), 6.59 (s, 2H, H4), 4.65 (s, 4H, H1', H3'), 4.00 (bs, 2H, NH), 0.98 (s, 18H, t-Bu), 0.1 (s, 12H, Me). **MS (ESI⁺):** *m*/*z* 382.82 [M+H⁺]. Calculated MS, C₂₀H₃₉Si₂NO₂: 381.70.

**3,5-bis-(t-butyldimethylsilyloxymethyl)-1-acetamido benzene (101).** TEA (0.55 mL, 4 mmoles, ≈5 eqs.) and **11** (300 mg, 0.79 mmoles, 1 eq.) were dissolved under stirring in CH₂Cl₂ (8 mL). Then, acetic anhydride (0.22 mL, 2.36 mmoles, 3 eqs.) and dimethylaminopyridine (spatula tip, catalytic) were added, and the solution was stirred at r.t. for 2 additional hrs. After dilution with CH₂Cl₂ (15 mL), the solution was washed with 5% aqueous citric acid (10 mL), saturated aqueous NaHCO₃ (10 mL) and brine (10 mL). The combined organic layer was dried with anhydrous Na₂SO₄, filtered and evaporated at reduced pressure. 3,5-Bis-(t-butyldimethylsilyloxymethyl)-1-acetamido benzene **101** was obtained as a crude yellow oil (350 mg), and was used as such in the next reaction step. **¹H_NMR (400 MHz, CDCl₃):** δ(ppm) 7.33 (s, 2H, H4), 7.28 (bs, 1H, NH), 7.08 (s, 1H, H2), 4.74 (s, 4H, H1', H3'), 2.22 (s, 3H, CO-Me), 0.98 (s, 18H, t-Bu), 0.1 (s, 12H, Si-Me). **MS (ESI⁺):** *m*/*z* 446.88 [M+Na⁺]. Calculated MS, C₂₂H₄₁Si₂NO₃: 423.74.

**3,5-bis-(hydroxymethyl)-1-acetamido benzene (81).** Compound **101** (350 mg, theor. 0.79 mmoles, 1eq.) was dissolved in dry MeOH (8 mL) under nitrogen atmosphere. The solution was cooled at 0°C, and acetyl chloride (30 µL, 0.39 mmoles, ≈0.5 eqs.) was added under stirring. The reaction mixture was stirred at 0° for additional 30 min. The solvent was then removed under reduced pressure, and the residue was triturated in acetone (10 mL), and filtered. The solid was washed with cold acetone (3 x 8 mL) and dried, to yield pure 3,5-bis-(hydroxymethyl)-1-acetamido benzene **81** (143 mg, 0.67 mmol, **84%** yield over two steps) as a white solid. **¹H_NMR (400 MHz, DMSO-d6):** δ(ppm) 9.92 (s, 1H, NH), 7.45 (s, 2H, H4), 6.90 (s, 1H, H2), 5.18 (bs, 2H, OH), 4.41 (s, 4H, H1', H3'), 2.00 (s, 3H, CO-Me). **MS (ESI⁺):** *m*/*z* 196.31 [M+H⁺]. Calculated MS, C₁₀H₁₃NO₃: 195.22.

**5-Acetamido isophthalaldehyde (71).** The title compound (104 mg, 0.54 mmoles, **88%** yield) was prepared from **81** (120 mg, 0.62 mmoles, 1.0 eq.) and manganese dioxide (268 mg, 3.10 mmoles, 5 eqs.) in chloroform (10 mL), following the experimental protocol of **general procedure B. ¹H_NMR (400 MHz, CD₃CN):** δ(ppm) 10.01 (s, 2H, H1', H3'), 8.65 (bs, 1H, NH), 8.72 (s, 2H, H4), 8.02 (s, 1H, H2), 2.09 (s, 3H, CO-Me). **MS (ESI⁺):** *m*/*z* 192.23 [M+H⁺]. Calculated MS, C₁₀H₉NO₃: 191.19.

**1-Acetamido-3,5-bis-aminoguanidine phenyl hydrazone, dihydrochloride salt (21).** The title compound (163 mg, 0.43 mmoles, **85%** yield, ≥95% purity, white solid) was prepared as a dihydrochloride salt from **71** (97 mg, 0.51 mmoles, 1.0 eq.) and aminoguanidine hydrochloride (113 mg, 1.02 mmoles, 1 eq.) in absolute ethanol (5 mL), following the experimental protocol of **general procedure C. ¹H_NMR (400 MHz, DMSO-d6):** δ(ppm) 12.18 (bs, 2H, NH), 10.22 (s, 1H, NH), 8.19 (s, 2H, H1', H3'), 8.17 (s, 1H, H2), 8.00-7.75 (m, 6H, NH), 7.94 (s, 2H, H4), 2.08 (s, 3H, CO-Me). **¹³C_NMR (100 MHz, DMSO-d6):** δ(ppm) 170.2, 156.8, 147.6, 141.2, 135.6, 122.1, 121.4, 24.4. **MS (ESI⁺):** *m*/*z* 326.40 [M+Na⁺]. Calculated MS, C₁₂H₁₇N₉O: 303.33.

**3,5-bis-(t-butyldimethylsilyloxymethyl)-1-p-tolylamido benzene (10m).** TEA (0.23 mL, 1.63 mmoles, 2 eq.) and **11** (310 mg, 0.82 mmoles, 1 eq.) were dissolved under stirring at 0°C in CH₂Cl₂ (8 mL). Then, 4-methylbenzoyl chloride (0.14 mL, 1.06 mmoles, 1.3 eqs.) was added, and the solution was stirred at 0°C for 1 additional hr. After quenching with MeOH (1 mL) and dilution with CH₂Cl₂ (15 mL), the solution was washed with 5% aqueous citric acid (10 mL), saturated aqueous NaHCO₃ (10 mL) and brine (10 mL). The combined organic layer was dried with anhydrous Na₂SO₄, filtered and evaporated at reduced pressure. The crude was purified by flash chromatography on silica gel (eluant mixture: neat CH₂Cl₂ to 95:5 CH₂Cl₂ /EtOAc), to afford pure 3,5-bis-(t-butyldimethylsilyloxymethyl)-1-amino(p-methylbenzoyl) benzene **10m** (265 mg, 0.53 mmol, **67%** yield) as a pale yellow oil. **¹H_NMR (400 MHz, acetone-d6):** δ(ppm) 9.29 (bs, 1H, NH), 7.75 (d, *J =* 8.2Hz, 2H, H7) 7.59 (s, 2H, H2), 7.18 (d, *J* = 8.2 Hz, 2H, H8), 7.01 (s, 1H, H1), 4.64 (s, 4H, H3), 2.27 (s, 3H, H6), 0.82 (s, 18H, H5), 0.00 (s, 12H, H4). **MS (ESI⁺):** *m*/*z* 523.06 [M+Na⁺]. Calculated MS, C₂₈H₄₅Si₂NO₃: 499.84.

**3,5-bis-(hydroxymethyl)-1-p-tolylamido benzene (8m).** Compound **10m** (260 mg, 0.52 mmol, 1eq) was dissolved in dry MeOH (10 mL) under nitrogen atmosphere. The solution was cooled at 0°C, and acetyl chloride (20 µL, 0.26 mmoles, ≈0.5 eqs.) was added under stirring. The reaction mixture was stirred at 0° for additional 30 min. The solvent was then removed under reduced pressure to yield the crude 3,5-bis-(hydroxymethyl)-1-p-tolylamido benzene **8m** (140 mg), that was used in the next step without further purification. **¹H_NMR (400 MHz, acetone-d6):** δ(ppm) 9.40 (bs, 1H, NH), 7.95 (d, *J* = 8.2 Hz, 2H, H2'') 7.75 (s, 2H, H4), 7.33 (d, *J* = 8.2 Hz, 2H, H3"), 7.12 (s, 1H, H2), 4.64 (s, 4H, H1', H3'), 2.42 (s, 3H, H4''). **MS (ESI⁺):** *m*/*z* 272.48 [M+H⁺]. Calculated MS, C₁₆H₁₇NO₃: 271.32.

**5-p-tolylamido isophthalaldehyde (7m).** The title compound (113 mg, 0.42 mmoles, **83%** yield) was prepared from **8m** (140 mg, 0.52 mmoles, 1.0 eq.) and manganese dioxide (340 mg, 5.20 mmoles, 10 eqs.) in chloroform (10 mL), following the experimental protocol of **general procedure B. ¹H_NMR (400 MHz, CDCl₃):** δ(ppm) 10.18 (s, 2H, H1', H3'), 9.96 (bs, 1H, NH), 8.73 (d, *J* = 4.1 Hz, 2H, H4), 8.22 (t, J = 4.1 Hz, 1H, H2), 8.00 (d, *J* = 8.2 Hz, 2H, H2''), 7.38 (d, *J* = 8.2 Hz, 2H, H3"), 2.44 (s, 3H, H4''). **MS (ESI⁺):** *m*/*z* 268.38 [M+H⁺]. Calculated MS, C₁₆H₁₃NO₃: 267.29.

**1-p-Tolylamido-3,5-bis-aminoguanidine phenyl hydrazone, dihydrochloride salt (2m).** The title compound (69 mg, 0.15 mmoles, **83%** yield, ≥95% purity, white solid) was prepared as a dihydrochloride salt from **7m** (47 mg, 0.18 mmoles, 1.0 eq.) and aminoguanidine hydrochloride (41 mg, 0.37 mmoles, 1 eq.) in absolute ethanol (4 mL), following the experimental protocol of **general procedure C. ¹H_NMR (400 MHz, DMSO-d6):** δ(ppm) 12.15 (bs, 2H, NH), 10.43 (bs, 1H, NH), 8.23 (s, 2H, H1', H3'), 8.20 (s, 1H, H2), 8.17 (s, 2H, H4), 7.95 (d, *J* = 8.2 Hz, 2H, H2"), 7.83 (bs, 6H, NH), 7.37 (d, *J =* 8.2 Hz, 2H, H3''), 2.41 (s, 3H, H4''). **¹³C_NMR (100 MHz, DMSO-d6):** δ(ppm) 165.9, 155.9, 146.6, 142.4, 140.3, 134.8, 131.9, 129.5, 128.2, 122.9, 121.6, 21.5. **MS (ESI⁺):** *m*/*z* 380.50 [M+H⁺]. Calculated MS, C₁₈H₂₁N₉O: 379.43.

**3,5-bis-(t-butyldimethylsilyloxymethyl)-1-methylsulfonamido benzene (10n).** Pyridine (0.72 mL, 0.87 mmoles, 2 eqs.) and **11** (166 mg, 0.43 mmoles, 1 eq.) were dissolved under stirring at 0°C in CH₂Cl₂ (4 mL). Then, 4-methylsulfonyl chloride (50 µL, 0.65 mmoles, 1.5 eqs.) was added, the solution was stirred at 0°C for 1 hr, and at r.t. for 15 hrs. After dilution with CH₂Cl₂ (15 mL), the solution was washed with water (10 mL) and brine (10 mL). The combined organic layer was dried with anhydrous Na₂SO₄, filtered and evaporated at reduced pressure. The crude was quickly eluted on silica gel (eluant mixture: 98:2 CH₂Cl₂/EtOAc), to afford crude 3,5-bis-(t-butyldimethylsilyloxymethyl)-1-methylsulfonamido benzene **10n** (200 mg) as a pale yellow oil, that was used in the next step without further purification. **¹H_NMR (400 MHz, acetone-d6):** δ(ppm) 8.44 (bs, 1H, NH), 7.12 (s, 2H, H4), 7.01 (s, 1H, H2), 4.64 (s, 4H, H1', H3'), 2.84 (s, 3H, SO₂-Me), 0.83 (s, 18H, t-Bu), 0.00 (s, 12H, Si-Me). **MS (ESI⁺):** *m*/*z* 460.94 [M+H⁺]. Calculated MS, C₂₁H₄₁Si₂NO₄S: 459.80.

**3,5-bis-(hydroxymethyl)-1-methylsulfonamido benzene (8n).** Compound **10n** (200 mg, theoretical 0.43 mmol, 1 eq.) was dissolved in dry MeOH (8 mL) under nitrogen atmosphere. The solution was cooled at 0°C, and acetyl chloride (20 µL, 0.26 mmoles, ≈0.7 eqs.) was added under stirring. The reaction mixture was stirred at 0° for additional 30 min. The solvent was then removed under reduced pressure to yield the crude 3,5-bis-(hydroxymethyl)-1-methylsulfonamido benzene **8n** (100 mg) as a pale brown solid, that was used in the next step without further purification. **MS (ESI⁺):** *m*/*z* 232.41 [M+H⁺]. Calculated MS, C₉H₁₃NO₄S: 231.27.

**5-methylsulfonamido isophthalaldehyde (7n).** The title compound (65 mg, 0.29 mmoles, **68%** yield over three steps) was prepared from **8n** (100 mg, theoretical 0.43 mmoles, 1.0 eq.) and manganese dioxide (300 mg, 4.33 mmoles, ≈10 eqs.) in chloroform (6 mL), following the experimental protocol of **general procedure B. ¹H_NMR (400 MHz, acetone-d6):** δ(ppm) 10.02 (s, 2H, H1', H3'), 9.02 (bs, 1H, NH), 8.09 (s, 1H, H2), 8.01 (s, 2H, H4), 3.01 (s, 3H, SO₂-Me). **MS (ESI⁺):** *m*/*z* 228.30 [M+H⁺]. Calculated MS, C₉H₉NO₄S: 227.24.

**1-Methylsulfonamido-3,5-bis-aminoguanidine phenyl hydrazone, dihydrochloride salt (2n).** The title compound (100 mg, 0.24 mmoles, **83%** yield, ≥95% purity, white solid) was prepared as a dihydrochloride salt from **7n** (65 mg, 0.29 mmoles, 1.0 eq.) and aminoguanidine hydrochloride (63 mg, 0.58 mmoles, 1 eq.) in absolute ethanol (3.5 mL), following the experimental protocol of **general procedure C. ¹H_NMR (400 MHz, DMSO-d6):** δ(ppm) 12.25 (bs, 2H, NH), 10.01 (s, 1H, NH), 8.24 (s, 1H, H2), 8.21 (s, 2H, H1', H3'), 7.86 (bs, 6H, NH), 7.60 (s, 2H, H4), 3.10 (s, 3H, SO₂-Me). **¹³C_NMR (100 MHz, DMSO-d6):** δ(ppm) 155.9, 146.3, 149.6, 135.4, 122.1, 121.4, 39.98. **MS (ESI⁺):** *m*/*z* 340.50 [M+H⁺]. Calculated MS, C₁₁H₁₇N₉O₂S: 339.37.

**3,5-bis-(t-butyldimethylsilyloxymethyl)-1-p-tolylsulfonamido benzene (10o).** Pyridine (0.72 mL, 0.87 mmoles, 2 eqs.) and **11** (166 mg, 0.43 mmoles, 1 eq.) were dissolved under stirring at 0°C in CH₂Cl₂ (5 mL). Then, p-tosylsulfonyl chloride (125 mg, 0.65 mmoles, 1.5 eqs.) was added, the solution was stirred at 0°C for 1 hr, and at r.t. for 6 hrs. After dilution with CH₂Cl₂ (15 mL), the solution was washed with saturated aqueous NaHCO₃ (10 mL) and brine (10 mL). The combined organic layer was dried with anhydrous Na₂SO₄, filtered and evaporated at reduced pressure. The crude was purified by flash chromatography on silica gel (eluant mixture: neat CH₂Cl₂ to 95:5 CH₂Cl₂/EtOAc), to afford pure 3,5-bis-(t-butyldimethylsilyloxymethyl)-1-p-tolylsulfonamido benzene **10o** (195 mg, 0.37 mmoles, **86%** yield) as a white solid. **¹H_NMR (400 MHz, acetone-d6):** δ(ppm) 8.87 (bs, 1H, NH), 7.61 (d, *J =* 8.2 Hz, 2H, H2''), 7.22 (d, J = 8.2Hz, 2H, H3''), 7.06 (s, 2H, H4), 6.93 (s, 1H, H2), 4.60 (s, 4H, H1', H3'), 2.28 (s, 3H, Ar-Me), 0.85 (s, 18H, t-Bu), 0.00 (s, 12H, Si-Me). **MS (ESI⁺):** *m*/*z* 536.94 [M+H⁺]. Calculated MS, C₂₇H₄₅Si₂NO₄S: 535.89.

**3,5-bis-(hydroxymethyl)-1-p-tolylsulfonamido benzene (8o).** Compound **10o** (195 mg, 0.36 mmoles, 1 eq.) was dissolved in dry MeOH (8 mL) under nitrogen atmosphere. The solution was cooled at 0°C, and acetyl chloride (10 µL, 0.13 mmoles, ≈0.4 eqs.) was added under stirring. The reaction mixture was stirred at 0° for additional 2 hrs. The solvent was then removed under reduced pressure, and the residue was chromatographed on silica gel (eluant mixture: 7:3 *n*-hexane/EtOAc) to yield pure 3,5-bis-(hydroxymethyl)-1-p-tolylsulfonamido benzene **8o** (90 mg, 0.29 mmoles, **81**% yield) as a white solid. **¹H_NMR (400 MHz, acetone-d6):** δ(ppm) 8.72 (bs, 1H, NH), 7.57 (d, *J = 8.2* Hz, 2H, H2''), 7.18 (d, J = 8.2Hz, 2H, H3"), 7.00 (s, 2H, H4), 6.89 (s, 1H, H2), 4.39 (s, 4H, H1', H3'), 4.03 (s, 2H, OH), 2.22 (s, 3H, Me). **MS (ESI⁺):** *m*/*z* 330.41 [M+Na⁺]. Calculated MS, C₁₅H₁₇NO₄S: 307.36.

**5-p-tolylsulfonamido isophthalaldehyde (7o).** The title compound (79 mg, 0.26 mmoles, **90%** yield over three steps) was prepared from **8o** (90 mg, 0.29 mmoles, 1.0 eq.) and manganese dioxide (200 mg, 2.9 mmoles, 10 eqs.) in chloroform (6 mL), following the experimental protocol of **general procedure B. ¹H_NMR (400 MHz, acetone-d6):** δ(ppm) 9.95 (s, 2H, H1', H3'), 8.02 (s, 1H, H2), 7.90 (s, 2H, H4), 7.63 (d, *J =* 8.2 Hz, 2H, H2"), 7.22 (d, J = 8.2Hz, 2H, H3"), 2.22 (s, 3H, Me). **MS (ESI⁺):** *m*/*z* 304.39 [M+H⁺]. Calculated MS, C₁₅H₁₃NO₄S: 303.33.

**1-p-Tolylsulfonamido-3,5-bis-aminoguanidine phenyl hydrazone, dihydrochloride salt (2o).** The title compound (80 mg, 0.16 mmoles, **76%** yield, ≥95% purity, white solid) was prepared as a dihydrochloride salt from **7o** (64 mg, 0.21 mmoles, 1.0 eq.) and aminoguanidine hydrochloride (46 mg, 0.42 mmoles, 1 eq.) in absolute ethanol (3 mL), following the experimental protocol of **general procedure** C. **¹H_NMR (400 MHz, DMSO-d6):** δ(ppm) 12.15 (bs, 2H, NH), 10.56 (s, 1H, NH), 8.17 (s, 1H, H2), 8.12 (s, 2H, H1', H3'), 7.82 (bs, 6H, NH), 7.71 (d, J = 8.2 Hz, 2H, H2"), 7.46 (s, 2H, H4), 7.36 (d, J = 8.2 Hz, 2H, H3"), 2.34 (s, 3H, Me). **¹³C_NMR (100 MHz, DMSO-d6):** δ(ppm) 155.4, 145.8, 143.5, 138.8, 136.6, 134.9, 129.8, 126.8, 121.2, 120.7, 21.0. **MS (ESI⁺):** *m*/*z* 416.56 [M+H⁺]. Calculated MS, C₁₇H₂₁N₉O₂S: 415.47.

**1,3,5-Benzene tricarboxylate monomethyl ester (9p).** 1.8M Aqueous NaOH (9.1 mL, 16.4 mmoles, ≈2 eqs.) was added dropwise to a vigorously stirred suspension of 1,3,5-benzene tricarboxylate trimethyl ester (2.0 g, 8.0 mmoles, 1 eq.) in MeOH (64 mL) at r.t.. Stirring at r.t. was continued for 5 hrs, obtaining a clear solution. After solvent evaporation at reduced pressure, water (25 mL) was added and the pH was brought to2.0 by dropwise addition of aqueous 6N HCl. The resulting suspension was extracted with EtOAc (2 x 25 mL). After washing with water (2 x 10 mL) and brine (2 x 10 mL), the combined organic phase was concentrated at reduced pressure. The crude 1,3,5-benzene tricarboxylate monomethyl ester **9p** (1.5 g) was used in the next reaction step without further purification. **MS (ESI⁺):** *m*/*z* 225.22 [M+H⁺]. Calculated MS, C₁₀H₈O₆: 224.17.

**Methyl 3,5-bis-(hydroxymethyl)-benzene carboxylate (8p).** The title compound (775 mg, 3.94 mmol, **54%** yield over two steps) was prepared from **9p** (1.5 g, theoretical 6.7 mmoles, 1.0 eq.) and 1M BH₃ in THF (33 mL, 33 mmoles, ≈5 eqs.) in THF (3 mL), following the experimental protocol of **general procedure A. ¹H_NMR (400 MHz, DMSO-d6):** δ(ppm) 7.80 (s, 2H, H4), 7.49 (s, 1H, H2), 4.58 (s, 4H, H1', H3'), 3.45 (s, 2H, OH). **MS (ESI⁺):** *m*/*z* 197.27 [M+H⁺]. Calculated MS, C₁₀H₁₂O₄: 196.20.

**5-Carbomethoxy isophthalaldehyde (7p).** The title compound (645 mg, 3.35 mmoles, **85%** yield) was prepared from **8p** (775 mg, 3.94 mmoles, 1.0 eq.) and manganese dioxide (1.9 g, 19.5 mmoles, ≈5 eqs.) in chloroform (20 mL), following the experimental protocol of **general procedure B. MS (ESI⁺):** *m*/*z* 193.26 [M+H⁺]. Calculated MS, C₁₀H₈O₄: 192.17.

**1-Carbomethoxy-3,5-bis-amino guanidine phenyl hydrazone, dihydrochloride salt (2p).** The title compound (109 mg, 0.29 mmoles, **71%** yield, ≥95% purity, white solid) was prepared as a dihydrochloride salt from **7p** (78 mg, 0.41 mmoles, 1.0 eq.) and aminoguanidine hydrochloride (90 mg, 0.81 mmoles, 1 eq.) in absolute ethanol (4 mL), following the experimental protocol of **general procedure C. ¹H_NMR (400 MHz, DMSO-d6):** δ(ppm) 12.30 (bs, 2H, NH), 8.64 (s, 1H, H2), 8.44 (s, 2H, H1', H3'), 8.30 (s, 2H, H4), 7.91 (bs, 6H, NH), 3.92 (s, 3H, OMe). **¹³C_NMR (100 MHz, DMSO-d6):** δ(ppm) 166.1, 156.0, 145.7, 135.1, 131.5, 130.7, 130.0, 53.0. **MS (ESI⁺):** *m*/*z* 305.33 [M+H⁺]. Calculated MS, C₁₂H₁₆N₈O₂: 304.26.

**5-Carboxy isophthalaldehyde (12).** 2M aqueous NaOH (0.47 mL, 0.94 mmoles, 1.05 eqs) was addet to a suspension **of 7p** (174 mg, 0.90 mmoles, 1 eq.) in MeOH (10 mL) under vigorous stirring at r.t.. The resulting suspension was stirred at r.t. for 24 After 24 h, the solvent was removed at reduced pressure, and crude 5-carboxy isophthaldehyde (160 mg) was used in the next step without further purification. **MS (ESI⁺):** *m*/*z* 169.21 [M+H⁺]. Calculated MS, C₉H₆O₄: 168.14.

**5-(N₄-phenylpiperazinyl)carboxamido isophthalaldehyde (7q).** HOBt (162 mg, 1.2 mmoles, 1.25 eqs.) and EDC (230 mg, 1.2 mmoles, 1.25 eqs.) were sequentially added to a suspension of crude **12** (160 mg, theoretical 0.90 mmoles) in dry CH₂Cl₂ (10 mL) under vigorous stirring at r.t. under nitrogen atmosphere. The resulting mixture was stirred at r.t. for 15 minutes, then 4-phenyl-1-piperazine (195 mg, 1.2 mmoles, 1.25 equivand TEA (0.28 mL, 1.8 mmoles, 2 eqs.) were sequentially added. The resulting solution was stirred at r.t. for 24 hrs, and was then concentrated under reduced pressure. The residue was taken up in EtOAc (20 mL), washed with 5% citric acid solution (5 mL), saturated aqueous NaHCO₃ (5 mL) and brine (5 mL). After drying over Na₂SO₄, the combined organic phase was filtered and concentrated under reduce pressure. Pure 5-(N₄-phenylpiperazinyl)carboxamido isophthalaldehyde **7q** (90 mg, 0.28 mmoles, **30%** yield over 2 steps) was obtained after chromatographic purification (eluant mixture: 4:6 n-hexane/AcOEt + 1% TEA). **¹H NMR (400 MHz, acetone-d₆):** δ: 10.15 (s, 2H, H1', H3'), 8.46 (s, 1H, H2), 8.23 (s, 2H, H4), 7.32 (m, 2H, H3‴), 6.94 (m, 3H, H2‴, H4‴), 3.99 (bs, 3H, H2"), 3.61 (bs, 1H, H2''), 3.30 (bs, 3H, H3"), 3.18 (bs, 1H, H3"). **MS (ESI⁺):** *m*/*z* 323.46 [M+H⁺]. Calculated MS, C₁₉H₁₈N₂O₃: 322.36.

**5-(N₄-phenylpiperazinyl)carboxamido-3,5-bis-amino guanidine phenyl hydrazone, dihydrochloride salt (2q).** The title compound (110 mg, 0.20 mmoles, **72%** yield, ≥95% purity, white solid) was prepared as a dihydrochloride salt from **7q** (90 mg, 0.28 mmoles, 1.0 eq.) and aminoguanidine hydrochloride (62 mg, 0.56 mmoles, 1 eq.) in absolute ethanol (5 mL), following the experimental protocol of **general procedure C.¹H_NMR (400 MHz, DMSO-d6):** δ(ppm) 12.32 (bs, 2H, NH), 8.35 (s, 1H, H2), 8.25 (s, 2H, H1', H3'), 8.09 (s, 2H, H4), 7.85 (bs, 6H, NH), 7.32 (m, 2H, H3‴), 7.21 (m, 2H, H2‴), 6.98 (m, 1H, H4‴), 3.92 (bs, 1H, H2"), 3.57 (bs, 1H, H2''), 3.28 (bs, 1H, H3"), 3.17 (bs, 1H, H3''). **¹³C_NMR (100 MHz, DMSO-d6):** δ(ppm) 168.5, 156.0, 145.7, 137.3, 134.9, 129.7, 128.2, 127.4, 121.2. **MS (ESI⁺):** *m*/*z* 435.63 [M+H⁺]. Calculated MS, C₂₁H₂₆N₁₀O: 434.58.

**1-3,5-tris-amino guanidine phenyl hydrazone, trihydrochloride salt (2r).** The title compound (105 mg, 0.24 mmoles, **78%** yield, ≥95% purity, white solid) was prepared as a trihydrochloride salt from 1,3,5-benzene tricarboxaldehyde (50 mg, 0.31 mmoles, 1.0 eq.) and aminoguanidine hydrochloride (105 mg, 0.94 mmoles, 1 eq.) in absolute ethanol (5 mL), following the experimental protocol of **general procedure C. ¹H_NMR (400 MHz, D₂O):** δ(ppm) 7.57 (s, 3H, H1'), 7.30 (s, 3H, H2). **¹³C_NMR (100 MHz, D₂O):** δ(ppm) 154.5, 146.3, 133.4, 127.6. **MS (ESI⁺):** *m*/*z* 331.53 [M+H⁺]. Calculated MS, C₁₂H₁₈N₁₂: 330.36.

**N₁-*n*-Butylamino guanidine hydroiodide (14t).** S-methyl isothiosemicarbazide dihydroiodide **13** (510 mg, 2.19 mmoles, 1 eq.) was dissolved in MeOH (6 mL), and *n*-butylamine (325 µL, 3.28 mmoles, 1.5 eqs.) were added under stirring at r.t.. The reaction mixture was concentrated at reduced pressure after 72 hours. Crude N₁-*n-*butylamino guanidine hydroiodide **14t** was obtained as a red oil (565 mg) that was used in the next step without purification. **¹H_NMR (400 MHz, DMSO-d6):** δ(ppm) 8.46 (bs, 1H, NH), 7.25 (bs, 3H, NH), 4.69 (bs, 2H, NH), 3.13 (s, 2H, H1), 1.48 (m, 2H, J = 7 Hz, H2) 1.28 (m, 4H, J = 7 Hz, H3), 0.92 (m, 3H, J = 7 Hz, H4).**MS (ESI⁺):** *m*/*z* 131.26 [M+H⁺]. Calculated MS, C₅H₁₄N₄: 130.19.

**1,3-bis-(N₃-*n*-butylamino) guanidine phenyl hydrazone, dihydroiodide salt (2t).** The title compound (265 mg, 0.44 mmoles, **53%** yield, ≥95% purity, pale yellow solid) was prepared as a dihydroiodide salt from isophthalaldehyde (110 mg, 0.82 mmoles, 1.0 eq.) and **14t** (490 mg, theoretical 1.90 mmoles, 1.15 eqs.) in absolute ethanol (5 mL), following the experimental protocol of **general procedure D,** and a preparative HPLC separation (eluant mixture: neat water to neat CH₃CN). **¹H_NMR (400 MHz, DMSO-d6):** δ(ppm) 11.44 (bs, 2H, NH), 8.25 (m, 3H, H1', H3', H2), 8.21-7.90 (bs, 4H, NH), 8.03 (d, J = 8.1 Hz, 2H, H4), 7.57 (t, J=8 Hz, 1H, H5), 3.37 (m, 4H, H6), 1.54 (m, 4H, H7), 1.35 (m, 4H, H8), 0.94 (t, J = 7.12 Hz, 6H, H9). **¹³C_NMR (100 MHz, DMSO-d6):** δ(ppm) 154.4, 146.5, 134.5, 129.6, 127.5, 41.3, 31.1, 19.7, 14.1.**MS (ESI⁺):** *m*/*z* 359.69 [M+H⁺]. Calculated MS, C₁₈H₃₀N₈: 358.51.

**N₁-Benzylamino guanidine hydroiodide (14u).** S-methyl isothiosemicarbazide hydroiodide **13** (995 mg, 4.28 mmoles, 1 eq.) was dissolved in MeOH (8 mL), and benzylamine (470 µL, 4.28 mmoles, 1 eq.) were added under stirring at r.t.. The reaction mixture was concentrated at reduced pressure after 72 hours. N₁-Benzylamino guanidine hydroiodide **14u** was obtained after recrystallization from EtOH/diethyl ether as an orange solid (425 mg, 1.46 mmoles, **34%** yield). **¹H_NMR (400 MHz, DMSO-d6):** δ(ppm) 8.69 (bs, 1H, NH), 7.46-7.20 (m, 8H, NH, H2, H3, H4), 4.76 (s, 2H, NH₂), 4.42 (s, 2H, H1'). **MS (ESI⁺):** *m*/*z* 165.27 [M+H⁺]. Calculated MS, C₈H₁₂N₄: 164.21.

**1,3-bis-(N₃-benzylamino) guanidine phenyl hydrazone, dihydroiodide salt (2u).** The title compound (177 mg, 0.26 mmoles, **70%** yield, ≥95% purity, pale yellow solid) was prepared as a dihydroiodide salt from isophthalaldehyde (50 mg, 0.37 mmoles, 1.0 eq.) and **14u** (250 mg, 0.86 mmoles, 1.3 eqs.) in absolute ethanol (5 mL), following the experimental protocol of **general procedure D,** and a preparative HPLC separation (eluant mixture: neat water to neat CH₃CN). **¹H_NMR (400 MHz, DMSO-d6):** δ(ppm) 11.60 (bs, 2H, NH), 8.57 (m, 2H, H4), 8.27 (m, 3H, H2, H1', H3'), 8.05 (bs, 4H, NH), 7.55 (t, J = 7.8 Hz, 1H, H5), 7.35 (m, 10H, H2'', H3'', H4"), 4.60 (d, J = 5.4 Hz, 4H, H1''). **¹³C_NMR (100 MHz, DMSO-d6):** δ(ppm) 154.7, 146.9, 137.5, 134.5, 129.6, 129.0,1128.0, 127.7, 127, 5, 44.5.**MS (ESI⁺):** *m*/*z* 449.69 [M+Na⁺]. Calculated MS, C₂₄H₂₆N₈: 426.53.

**N₁-Pyrrolidinyl guanidine hydroiodide (14v).** S-methyl isothiosemicarbazide hydroiodide **13** (1.21 g, 5.21 mmoles, 1 eq.) was dissolved in MeOH (10 mL), and pyrrolidine (650 µL, 7.81 mmoles, 1.5 eqs.) were added under stirring at r.t.. The reaction mixture was concentrated at reduced pressure after 72 hours. Crude N₁-pyrrolidinyl guanidine hydroiodide **14v** was obtained from trituration with EtOAc (20 mL), solvent concentration under reduced pressure, trituration with diethyl ether (20 mL), filtration and washing with diethyl ether (3 x 5 mL). Crude **14v** (1.22 g, pink solid) was used as such in the next reaction step. **¹H_NMR (400 MHz, DMSO-d6):** δ(ppm) 8.74 (bs, 1H, NH), 7.22 (bs, 3H, NH), 4.78 (bs, 2H, NH₂), 3.30 (t, 4H, J = 6.4, H1), 1.90 (m, 4H, J = 6.4, H2). **MS (ESI⁺):** *m*/*z* 129.22 [M+H⁺]. Calculated MS, C₅H₁₂N₄: 128.18.

**1,3-bis-(N₃-pyrrolidinyl) guanidine phenyl hydrazone, dihydroiodide salt (2v).** The title compound (128 mg, 0.21 mmoles, **70%** yield, ≥95% purity, pale yellow solid) was prepared as a dihydroiodide salt from isophthalaldehyde (40 mg, 0.30 mmoles, 1.0 eq.) and **14v** (168 mg, 0.66 mmoles, 1.1 eqs.) in absolute ethanol (3 mL), following the experimental protocol of **general procedure D. ¹H_NMR (400 MHz, DMSO-d6):** δ(ppm) 11.31 (bs, 2H, NH), 8.43 (s, 2H, H1', H3'), 8.28 (s, 1H, H2), 8.06 (d, J = 7.84 Hz, 2H, H4), 7.97 (bs, 4H, NH), 7.57 (t, J = 7.84 Hz, 1H, H5), 3.50 (t, J = 6.4 Hz, 8H, H1''), 2.00 (t, J = 6.4 Hz, 8H, H2"). **¹³C_NMR (100 MHz, DMSO-d6):** δ(ppm) 152.3, 147.1, 134.5, 129.8, 129.7, 127.4, 48.1, 25.1.**MS (ESI⁺):** *m*/*z* 355.58 [M+H⁺]. Calculated MS, C₁₈H₂₆N₈: 354.46.

**1-p-Tolylamido-3,5-bis-(N₃-*n*-butylamino) guanidine phenyl hydrazone, diformate salt (2w).** The title compound (170 mg, 0.29 mmoles, **69%** yield, ≥95% purity, pale pink solid) was prepared as a diformate salt from **7m** (110 mg, 0.42 mmoles, 1.0 eq.) and **14t** (254 mg, 0.98 mmoles, 1.2 eqs.) in absolute ethanol (7 mL), following the experimental protocol of **general procedure D,** and a preparative HPLC separation (eluant mixture: neat water to neat MeOH + 0.3% formic acid). **¹H_NMR (400 MHz, DMSO-d6):** δ(ppm) 10.32 (bs, 1H, NH), 8.64 (bs, 2H, NH), 8.31 (s, 2H, NH), 8.13 (s, 2H, H1', H3'), 8.08 (s, 1H, H2), 8.05 (s, 2H, H4), 7.93 (d, *J* = 8.2 Hz, 2H, H2"), 7.83 (bs, 4H, NH), 7.36 (d, *J* = 8.2 Hz, 2H, H3''), 3.24 (bs, 4H, H1‴), 2.40 (s, 3H, H4"), 1.53 (m, 4H, H2‴), 1.36 (m, 4H, H3‴), 0.92 (t, *J* = 8.3 Hz, 6H, H4‴). **¹³C_NMR (100 MHz, DMSO-d6):** δ(ppm) 166.2, 165.9, 144.9, 142.3, 140.1, 132.1, 129.4, 128.2, 122.1, 121.8, 40.4, 31.3, 21.5, 19.9, 14.1. **MS (ESI⁺):** *m*/*z* 492.70 [M+H⁺]. Calculated MS, C₂₆H₃₇N₉O: 491.64.

**1,3-Bis-(N₂N₃-imidazolidinyl) guanidine phenyl hydrazone, dihydrobromide salt (2x).** The title compound (196 mg, 0.43 mmoles, **82%** yield, ≥95% purity, white solid) was prepared as a dihydrobromide salt from isophthalaldehyde (70 mg, 0.52 mmoles, 1.0 eq.) and imidazolidinamino guanidine hydrobromide (198 mg, 1.10 mmoles, 1.05 eqs.) in absolute ethanol (3.5 mL), following the experimental protocol of **general procedure D. ¹H_NMR (400 MHz, DMSO-d6):** δ(ppm) 12.42 (bs, 2H, NH), 8.75 (bs, 4H, NH), 8.27 (s, 2H, H1', H3'), 8.22 (s, 1H, H2), 7.99 (d, J = 7.79 Hz, 2H, H4), 7.59 (t, J = 7.79 Hz, 1H, H5), 3.77 (s, 8H, H1"). **¹³C_NMR (100 MHz, DMSO-d6):** δ(ppm) δ 157.9, 148.1, 132.9, 130.0, 129.6, 128.0, 43.0. **MS (ESI⁺):** *m*/*z* 295.47 [M+H⁺]. Calculated MS, C₁₄H₁₈N₈: 294.35.

**1,3-Bis-(N₁-methyl-N₂N₃-imidazolidinyl) guanidine phenyl hydrazone, dihydrobromide salt (2y).** The title compound (205 mg, 0.42 mmoles, **80%** yield, ≥95% purity, white solid) was prepared as a dihydrobromide salt from isophthalaldehyde (70 mg, 0.52 mmoles, 1.0 eq.) and N₃-methyl imidazolidinamino guanidine hydrobromide (213 mg, 1.10 mmoles, 1.05 eqs.) in absolute ethanol (3.5 mL), following the experimental protocol of **general procedure D. ¹H_NMR (400 MHz, DMSO-d6/D₂O mixture):** δ(ppm) 8.38 (s, 1H, H2), 8.19 (s, 2H, H1', H3'), 8.05 (d, J = 7.75 Hz, 2H, H4), 7.60 (t, J = 7.75 Hz, 1H, H5), 3.81 (s, 6H, N-Me), 3.75 (s, 8H, H1''). **¹³C_NMR (100 MHz, DMSO-d6):** δ(ppm) 159.5, 144.4, 134.5, 130.0, 129.7, 128.3, 43.6, 22.2. **MS (ESI⁺):** *m*/*z* 327.49 [M+H⁺]. Calculated MS, C₁₆H₂₂N₈: 326.41.

**1,3-Bis-(N₂N₃-benzimidazolidinyl) guanidine phenyl hydrazone, diacetate salt (2z).** N,N'-Benzimidazolyl amidinoguanidine (110 mg, 0.74 mmoles, 1.05 eqs.) and AcOH (50 µL) were added under stirring at r.t. to a solution of isophthalaldehyde (46 mg, 0.34 mmoles, 1 eq.) in absolute EtOH (5 mL). The reaction mixture was heated and refluxed for 2 hrs. After cooling to r.t and filtration, the solid precipitate was collected, washed with water (2 x 5 mL), and dried to afford pure 1,3-bis-N₂N₃-benzimidazolyl guanidine phenyl hydrazone, diacetate salt **2z** (87 mg, 0.22 mmoles, **66%** yield) as a yellow solid. **¹H_NMR (400 MHz, DMSO-d6):** δ(ppm) 11.68 (bs, 4H, NH), 8.36 (s, 1H, H2), 8.15 (s, 2H, H1', H3'), 7.75 (d, J = 7.7 Hz, 2H, H4), 7.59 (t, 1H, J = 7.7 Hz, H5), 7.33 (m, 4H, H1''), 7.05 (m, 4H, H2"). **¹³C_NMR (100 MHz, DMSO-d6):** δ(ppm) 149.8, 146.4, 134.8, 131.6, 129.7, 128.9, 127.4, 123.6, 112.6. **MS (ESI⁺):** *m*/*z* 395.55 [M+H⁺]. Calculated MS, C₂₂H₁₈N₈: 394.44.

### Reference Example

**1',3'-Dimethyl-1,3-bis-aminoguanidine phenyl hydrazone, dihydrochloride salt (Compound (VII)).** The title compound (274 mg, 0.73 mmoles, **70%** yield, ≥95% purity, white solid) was prepared as a dihydrochloride salt from 1,3-diacetylbenzene (170 mg, 1.05 mmoles, 1.0 eq.) and aminoguanidine hydrochloride (232 mg, 2.10 mmoles, 1 eq.) in absolute ethanol (10 mL), following the experimental protocol of **general procedure C.¹H_NMR (400 MHz, DMSO-d6):** δ(ppm) 11.40 (bs, 2H, NH), 8.35 (s, 1H, H2), 8.06 (d, J = 9.2 Hz, 2H, H4), 8.15-7.60 (bs, 6H, NH), 7.50 (t, J = 9.2 Hz, 1H, H5), 2.41 (s, 6H, Me). **¹³C_NMR (100 MHz, D₂O):** δ(ppm) 155.3, 152.7, 136.0, 128.6, 127.6, 124.0, 13.3. **MS (ESI⁺):** *m*/*z* 275.45 [M+H⁺]. Calculated MS, C₁₂H₁₈N₈: 274.33.

### Biology

### Cell cultures

Mouse albino neuroblastoma Neuro2a cells were maintained in standard medium (DMEM + 2mM Glutamine + 10% Fetal Bovine Serum (FBS)). Split sub-confluent cultures (70-80%) 1:3 to 1:8 i.e. seeding at 4x10 000 cells cm² and kept in 5% CO₂; 37°C. Transfections were performed using Lipofectamine^{®} LTX Transfection Reagent (Thermofisher) according manufacturer' instruction. The following plasmids were used: pcDNA3.1(+)SOD1G93A, pCAGG GFP, p CMV-LacZ, CMV-VPS35, Sigma Mission shRNA: TRCN000011556 (Sh56), TRCN000011559 (Sh59), TRCN000011558 (Sh58), scramble.

Cell survival experiments were run on 96-well plates using Cell Counting Kit-8 (CCK8) survival kit (96992-Sigma) according the manufacturer's recommendations. Compound 2a cytotoxicity was tested on Neuro2a cells receiving increasing amount of compound 2a (1, 10, 50, 100, 200, 500 and 1000 µM) for 48 hours.

LDH cytotoxicity was run on 96 well plates using LDH-Glo^{™} Cytotoxicity Assay (Promega). Briefly, Neuro2a cells were transfected with plasmids encoding pCMV-LacZ; pcDNA3.1(+)SOD1G93A with or without VPS35 short interfering plasmids (Sh56) or compound 2a (10 µM). After 48 hours, 5µl of supernatants were 100 fold diluted in LDH storage buffer (Tris-HCl 200mM, 10% Glycerol, 1% BSA, Sigma-Aldrich) and used to determine LDH, according to manufacturer's recommendations. Maximum LDH Release Control was obtained lysing untreated Neuro2a cells with 2 µL of 10% Triton X-100 (Sigma-Aldrich). In each experiment we calculated % Cytotoxicity=(Exp.LDH Release-Medium Background)/(Max LDH Release Control-Medium Background), and we run parallel positive controls treating cells with 200 µM H₂O₂.

Cycloheximide (CHX) assay was done on Neuro2a cells seeded in 6-well plates and incubated with compound 2a (10 µM) or vehicle for 24 hours. We next added CHX (Sigma) at 10 µg/mL and cells were collected at 0, 2, 4 and 8 hours for the time-course assay of VPS35 levels.

Autophagy assay were done using a stable Neuro2a cell clone expressing the pHluorin-mKate2-tagged human LC3 plasmids, Cell clones were generated according our published methods (Mazzocchi 2016). Upon transfection, cells were selected by 400 µg/mL G418 Disulfate Salt Solution (Sigma-Aldrich, St. Louis, MO) and further cloned by limiting dilution. Candidate cell clones were evaluated by flow cytometry (Cytoflex-S) and the clone 7B11 was selected. Quality controls of the assay were done calculating Z-factor (>0.5) and SSMD (> 4.7 for Bafilomycin A1 and <4.7 for Torin 1) values in Neuro2a 7B11 cells treated BafilomycinA1 (200nM) and Torin-1 (250nM), (7 independent experiments). Neuro2a 7B11 cells were treated with Compond 2a (0.1-100µM) for 48 hours then MFI levels of the pHfluorin were measured in living cells by flow cytometry.

### Screening the library of amino guanidine hydrazones 2a-2mm in Neuro2a cells

Neuro2a cells were seeded on 12 wells multiwell at the concentration of 100 000 cells/well. Cells were incubated with each compound, including the standard R55, at 10µM for 48 hours. Cells were then washed in PBS 1x and lysed in a buffer containing Tris-HCl 10 mM pH8, EDTA 1mM pH8, NaCl 100 mM, NP40 1%, with protease inhibitor cocktail (Sigma) 1X. Using a cell scraper total lysates were collected from each well, quantified with BCA (BCA protein assay, Thermofisher). Five µg were resolved on precast 10% acrylamide gel (Biorad). Proteins were transferred to a PVDF membrane (Millipore) and western blot were performed using the following antibodies: goat αVPS35 (1:1000), mouse α-βActin (1:10000). Detection of proteins was obtained by a chemiluminescence detection kit (PerkinElmer).

### Immunoblotting

Lumbar spinal cords were dissected from saline perfused mice and rapidly homogenized in the following lysis buffer: Tris-HCl 10 mM pH 8, EDTA pH 8 1 mM, NaCl 100mM, NP40 1% and protease inhibitor cocktail (Sigma). Protein extracts were obtained using a tight-fitting glass Potter tissue grinder (1 ml; Wheaton) and then sonicated at a frequency of 20 kHz (10 times-1s). Protein lysates from cells cultures were obtained from plates receiving PBS1x washing and subsequently the lysis buffer followed by sonication. Protein concentrations were measured by BCA protein assay kit (Thermofisher) according to the manufacturer's recommendations. Ten µg of protein extract was loaded on 10% Mini-PROTEAN TGX Stain-Free precast gels for PAGE (Bio-Rad). Gel electrophoresis was performed at 100 V for 2 hours. Gels were transferred on Immobilon^{®}-P PVDF membrane according to the manufacturer's instructions (Millipore). Blots were blocked in 5% BSA in Tris buffered saline plus 0.1% Tween-20 (TBS-T) for 1 hour before receiving primary antibodies: goat α-VPS35 1:800 (ab10099, Abcam); rabbit α-VPS26b 1:1000 (15915-1-AP, ProteinTech); rabbit α-VPS29 1:1000 (ab236796 Abcam); mouse α-βActin 1:25000 (Sigma); mouse α-Golgin97 1:1000 (Invitrogen); mouse α-Ubiquitin 1:500 (Merk) overnight at 4°C on shaker. Following several washes with TBS-T 1X, blots were incubated in appropriate HRP-labeled secondary antibodies (Bio-Rad) for 1 hour at room temperature. Visualization of protein bands was obtained using ClarityMax-ECL (Bio-Rad) according to the manufacturer's instructions and images were acquired on ChemiDoc (Bio-Rad). Quantifications were done using the Image Lab 6.0.1 software (Bio-Rad).

### Blue-Native gel electrophoresis

G93A mice and their WT litters were injected daily with vehicle or compound 2a (10 mg/kg, from day 83 to day 103). Lumbar spinal cords dissected from saline perfused mice were lysed in Tris-HCl 10 mM pH 8, EDTA 1 mM pH 8, NaCl 100mM, NP40 1% and protease inhibitor cocktail (Sigma). Extracts were obtained using a tight-fitting glass Potter tissue grinder (1 ml; Wheaton). We incubated 250µl of each extract with 100mM Iodoacetamide (Sigma). Samples were mixed with 4x Sample NativePAGE (Thermofisher) sample buffer and the G-250 additive (Thermofisher) and proteins were resolved on native 4-15% Bis-Tris gel (Bio-Rad) with Dark Blue Cathode buffer (Thermofisher) at 150 V for 1/3 of the gel length. We replaced the running buffer with Light Blue Cathode Buffer to complete the run. At the end of the electrophoresis, gels were washed with 5 mM tris(2-carboxyethyl)phosphine (Sigma) for 15 min and then proteins were transferred to nitrocellulose and fixed on the membrane by 8% acetic acid for 15 min. Rabbit α-SOD1 (1:2000, Genetex) was incubated overnight as above described. Visualization of proteins was performed using ClarityMax-ECL (Bio-Rad) according to the manufacturer's instructions and images were acquired on ChemiDoc (Bio-Rad).

### Immunohistochemistry, immunofluorescence and morphometric analyses

Paraffin embedded SCs were obtained from ALS patients and non-neurological controls from the Target ALS Human Postmortem Tissue Core. Sections were stored at the INSPE tissue Bank. Sections were incubated with a polyclonal VPS35 antibody (1:400 Abcam ab97545) and with the polyclonal VPS26 antibody (ab23892 Abcam) after rehydration and antigen retrieval (slides were warmed-up at 97°C in TRIS EDTA pH=9 by water bath). Peroxidase-diaminobenzidine (DAB) reaction was used for detecting primary antibody, biotinylated anti-rabbit IgG (H+L) (1:500 Vector laboratories) and avidin-biotin complex (ABC 1:100 Vector laboratories). An operator that was blind to the disease status and demographic data subsequently evaluated these sections.

Sections from mice (frozen cryo sections) or glass covers containing cells were washed three times in PBS 1X (Lonza, 5min each), and incubated in the following blocking mix: PBS 1X/FBS 10% (Invitrogen), BSA 1 mg/mL (Sigma), TritonX100 0.1% (Sigma), for 1h at room temperature. Antibodies were diluted in blocking mix and incubated at +4°C overnight according manufacturer's instructions. The following day, sections were rinsed in PBS and fluorescent secondary antibodies -i.e. never deriving from the species from which the primary antibodies are derived- (Alexafluor conjugated) diluted in blocking mix, were used according to the manufacturer's instructions. Slides were incubated in Hoechst 33342 (Sigma) for nuclei counterstaining. When necessary, we performed antigens retrieval by boiling samples in 10 mM sodium citrate (pH 6) for 5 min. The following antibodies and working concentrations were used: rabbit α-VPS35 1:1000 (ab97545 Abcam); rabbit α-VPS26 1:1000 (ab23892 Abcam); rabbit α-Sortilin 1:1000 (ab16640 Abcam); mouse α-CI-MPR 1:100 (Novus bio. NB-300-514), mouse α-NeuN 1:800 (Millipore MAB377); mouse α-Ubiquitin 1:500 (Millipore MAB1510); mouse α-Golgin97 1:700 (Thermofisher A21270); rabbit α-ISLET 1:1000 (ab20670 Abcam); goat α-ChAT 1:200 (Millipore ab144p); chicken α-GFP 1:1000 (Invitrogen); chicken α-Neurofilament-medium 1:500 (Biolegend PCK-593P); rat α-MBP 1:100 (kindly provided by Dr. A. Bolino); mouse α-GM130 1:100 (BD biosciences, 610823); rat α-CTSD 1:100 (R&D system MAB1029). Tyramide Signal Amplification (TSA, PerkinElmer) was used, when appropriate, to improve fluorescent intensity in single and double immunofluorescence

### Imaging

We used Olympus, BX51 with the following objectives: X20, X40 and X63 equipped with the following cameras: Leica CCD Microscope DFC3000 G and DMC2900 to obtain 16-bit light and fluorescence images (1296x966 pixels). Fluorescence images were merged using Photoshop (Adobe) CS4 or NHI-Image J software (US National Institutes of Health). We obtained confocal images using Leica SP8 with X40 and X64 objectives and equipped with super-sensitive HyD detectors. We recorded each fluorescence signal as square 8-bit images (1024x1024 pixels). Images were post processed to generate maximal projections of Z-stacks (acquired with a 0.4-0.8 µm step) and cross-sectional profiles of the Z-stack (acquired with a 0.3 µm step) and pseudo-colored using Las-X software.

### ELISA assay

Raw 264.7 cells (10⁵/well) were treated with compound 2a, CNI-1493 or vehicle for 1 hour then we applied LPS (100 ng/ml) and supernatants were collected after 4 hours. We used the Enzyme-linked immunosorbent assays (ELISA) procedure with OptEIA^{™} set for the detection of mouse TNFa (BD-Biosciences) according to manufacturer's instructions. Concentrations of TNFα were calculated according to a standard curve and expressed as nanograms per microliter. When concentrations of the cytokine were below the detection threshold, they were assumed to be 0 ng/µl.

### Quantification of MFI levels in tissue MNs

Fluorescence intensity levels of VPS35, VPS26, CI-MPR, Sortilin, CTSD and Ubiquitin were calculated using NHI-Image J software (US National Institutes of Health) according to the following protocol: stacks of confocal images were used to generate the maximal projections (Las-X) and then post-processed by NHI-Image J to crop single NeuN⁺ MNs from the ventral horn of the spinal cord. We next performed background (calculated on adjacent slices only receiving secondary antibodies) subtraction for each channel, before applying the NHI-Image J mean filter (with radius 2.0 pixels) to calculate mean NeuN fluorescence levels in individual MN. Using this approach, we established a region of interest (ROI) that gated single MN. On this selected ROI, we calculated mean fluoresce levels (MFI) of each protein.

### Morphological assessment of Golgi apparatus in Neuro2a

Golgi morphology was assessed in control or in Neuro2a cells receiving G93A plasmids with or without compound 2a (10 µM) according to published methods (Stafa 2012). In each experiment, we included GFP-transfected cells as additional control. We used Golgin97 immunofluorescence to label trans-Golgi network membranes. We classified Golgi morphology as follow: normal (polarized network), intermediate (partially fragmented) or fragmented (severely fragmented with Golgi stacks dispersed in the cytoplasm throughout), (Stafa 2012). In each experiment, cells were randomly sampled across three coverslips. Golgi subclasses were expressed as a percent of the total number of Golgi scored for each experimental condition.

### Micro Electrode Array (MEA) recordings

Primary neuronal cultures were established from the cerebral cortex of E16.5 C57BL6J embryos. Upon dissection, cells were plated on MEA biochips (60 electrode MEA biochips with 200 µm electrode spacing and 30 µm electrodes diameter with an integrated reference electrode, Multichannel Systems, GmbH) at the density of 3x10⁵ cells/MEA and kept in Neurobasal medium supplemented with B27 for 14 days. At day 14 neuronal cultures were exposed to increasing concentration of lead 2a (1, 3, 10, 30 and 100µM) in Krebs-Ringer-Hepes buffer (KRH, 130 mM NaCl, 5 mM KCl, 1.2 mM KH2PO4, 1.2 mM MgSO4, 2 mM CaCl2, 25 mM Hepes, 0.6% glucose, 1% glutamine, Sigma). Firing activity was recorded using a pre-amplifier stage (MEA-1060-Inv-BC-Standard, gain: 55, bandwidth: 300 Hz-8 kHz, MCS GmbH), an amplification and filtering stage (FA64S, gain 20, bandwidth: 10 Hz-6 kHz, MCS GmbH) and a data acquisition device (sampling frequency: 25 kHz). Then, an off-line signal processing was performed and raw data were analyzed by MC-Rack Software (MCS GmbH).

### Real time PCR

Lumbar spinal tracts dissected from saline perfused mice or Neuro2a cells were lysed to extract total RNAs using the RNeasy Mini Kit (Qiagen) according to the manufacturer's recommendations, including DNase (Promega) digestion. We synthetized the cDNA using ThermoScript RT-PCR System (Invitrogen) and Random Hexamer (Invitrogen), according to the manufacturer's instructions. The LightCycler 480 System (Roche) and LightCycler 480 SYBR Green I Master Mix (Roche) were used for real-time PCR experiments. Normalization was obtained using the housekeeping gene Histone H3
(H3 F: 5'-GGTGAAGAAACCTCATCGTTACAGGCCTGGTAC-3' H3 R: 5'-CTGCAAAGCACCAATAGCTGCACTCTGGAAGC-3'). The following specific primers were used for gene expression analysis:
VPS35F: 5'-GTGCCGTGGTGTGCAGCATCCG-3'
VPS35R: 5'-ATGCTGCATCCGCACCCAGAGC-3'
VPS26AF: 5'-GCTAAGTATGAAATAATGGATGGGGC-3'
VPS26AR: 5'-CTTGTTCACATCTCTCATCGTGGGG-3'
VPS29F: 5'-CCAGCACATCCTCTGCACCGGC-3'
VPS29R: 5'-CCACGGAATAACTTGGTGTCCGTG-3'

### Pharmacology - in vivo

### Detection of Compound 2a in brain and blood samples isolated from mice

Healthy mice were daily injected with vehicle or compound 2a (10 mg/kg) and sacrificed afer 1, 7, 15 and 30 days. Upon saline perfusion, brains were dissociated from skulls, carefully weighted and rapidly stored in dry ice. Then, brains (~400 mg) were checked for the presence of Compound 2a. The extraction will be performed with MeOH according to the protocol reported in Yuan et al., 2012. After the extraction, each biological sample was re-suspended in 60 µl of LC/MS grade water and 5 µl analyzed by LC-MS/MS using the UPLC 1290 (Agilent Technologies) coupled to the TripleTOF 5600+ mass spectrometer (SCIEX) in positive mode. A Waters ACQUITY UPLC BEH HILIC column (2.1 x 10mm, 1.7 µm) was used for the chromatographic separation through a gradient of solvent A (ACN + 0.1% FA) and solvent B (H₂O + 0.1% FA). Calibration curves were constructed by plotting the peak area versus the corresponding concentrations using MultiQuant 2.1 software (SCIEX).

### Generation of MNs from human inducible Pluripotent Stem Cells(hiPSCs)

Skin biopsies from ALS patients and healthy volunteers were performed under local anesthesia, after informed consent at San Raffaele Hospital. The protocol was approved by the Local Etic Committee. Primary fibroblasts were grown in Dulbecco's modified Eagle's medium with Glutamax I (GIBCO). Human iPS cell lines were generated using non integrating Sendai virus, maintained in feeder-free conditions in mTeSR1 (Stem Cell Technologies) on embryonic stem cell (HESC) Matrigel (Corning). MNs were generated following the protocol published by Du and colleagues with minimal modification (Du et al., 2015). Briefly, when hiPSCs reached confluence, they have been detached using EDTA and plated 1:4 on Matrigel ES coated dishes in mTeSR-1 supplemented with ROCK inhibitor (StemMACS, Miltenyi Biotec). On the following day the medium was replaced with a chemically defined neural medium: DMEM/F12, Neurobasal medium at 1:1 ratio, 1% B27, 0.5% N2 (all from Gibco, ThermoFisher Scientific), 1% P/S (Gibco), 1% Glutamax (Gibco) and 0.1 mM Ascorbic Acid (Sigma). CHIR99021 (3 µM, Tocris), DMH1 (2 µM, Tocris) and SB431542 (2 µM, Miltenyi Biotec) were added to the medium. The culture medium was changed every other day, until day 6. On day 7, cells have been dissociated with Dispase (1U/mL), split 1:4 and kept on Matrigel growth factor reduced (MaGR) for 6 days in the basal neural medium described above, supplemented with 1 µM CHIR99021, 2 µM DMH1, 2 µM SB431542, RA (Retinoic Acid, 0.1 µM, Sigma) and SAG (Smoothened Agonist, 0.5 µM, Calbiochem). On day 13, cells have been dissociated with Dispase (1U/mL), split 1:4, and kept for 6 days in neural medium supplemented with 3 µM CHIR99021, 2 µM DMH1, 2 µM SB431542, 0.1 µM RA, 0.5 µM SAG and 0.5 mM Valproic Acid (VPA, Tocris). To induce MN differentiation, cells were dissociated with Dispase (1U/mL) and cultured in suspension for 6 days in neural medium with 0.5 µM RA and 0.1 µM SAG. On day 25, cells were dissociated into single-cells with Accumax (Sigma) and plated on MaGR coated plates, in neural medium with 0.5 µM RA, 0.1 µM SAG, 0.1 µM Compound E (Calbiochem) until day 35. Half of the medium has usually been changed every other day.

### Animals

Mice were maintained under pathogen-free conditions at San Raffaele Hospital mouse facility (Milan, Italy). All efforts were made to minimize animal suffering and to reduce the number of mice used in accordance with the European Communities Council Directive of 24 November 1986 (86/609/EEC). All animal experimental protocols were approved by the Ethics Review Committee for Animal Experimentation of the Italian Ministry of Health. Procedures were performed according to the guidelines of the Institutional Animal Care and Use Committee of the San Raffaele Scientific Institute (protocol number 693/2015PR). Transgenic mutant SOD1 mice carrying the SOD1^{G91A} allele (strain B6.Cg-Tg (SOD1^{G93A}) 1Gur/J) were purchased from Jackson laboratories, while C57BL6J used for breeding and experimental purposes were purchased from Charles River (Italy). Compound 2a (10 mg/kg) was daily intraperitoneally injected by an operator blind to the treatment and to the genotype. At the sacrifice, mice were given an overdose of anesthetic drugs and transcardially perfused with saline followed by 80/100 ml 4% paraformaldehyde in PBS, pH 7.2 (Sigma). Spinal cords were coronally sliced at ~6-mm thickness and post fixed in 4% paraformaldehyde (Sigma) in PBS, pH 7.2 for 12 h at + 4°C. Tissues were cryoprotected in PBS/30% Sucrose (Sigma), embedded in OCT inclusion media and stored at -80°C before processing. Lumbar spinal cords were 12 µM sectioned, labeled and digital images were acquired every 350 µm in a region encompassing 2.5 mm of the spinal cord. Animal cohort numbers were determined by power analysis based on preliminary results.

Comparative studies on sciatic nerves were done on tissues removed upon saline perfusion and rapidly fixed with 2% glutaraldehyde in 0.12 mol/L phosphate buffer, post fixed with 1% osmium tetroxide, and embedded in Epon (Fluka). Semi-thin sections (1 µm thick) were stained with toluidine blue and examined by light microscopy (Olympus BX51). Digitized not overlapping semi-thin section images from corresponding levels of the sciatic nerve were obtained with a digital camera (Leica DFC300F) using a 40X objective. Quantifications were done on at least 5 acquired images from each mouse. The numbers of degenerating nerve fibers were quantified using ImageJ software (National Institutes of Health, Bethesda, MD).

### Motor function

Motor activity of G93A mice was assessed by rotarod. Mice were habituated for 1 min on a static rotor and 1 min at constant speed (4 rpm) for two times and then tested for motor function over three trials performed over three consecutive days (one per day). Each trial consisted of three test sessions with 15 min interval between sessions. For each session five mice were placed on an accelerating rotor (4-40 rpm) and the latency to fall was recorded, with a maximum limit for individual animal set at 900 s.

### Statistics

We express data as the mean value (± standard error of the mean (s.e.m.) or ± standard deviation (s.d.)) of independent experiments. We assessed normality of data applying either Kolmogorov-Smirnov test (with Dallas-Wilkinson-Lille for P value) or D'Agostino & Pearson omnibus normality test. Comparisons were made using the following tests: unpaired t-test, one-way or two-way analysis of variance (ANOVA) tests, followed by Tukey's multiple Comparison test or by Bonferroni multiple comparison test. Statistical analyses were performed using PRISM5.01 (GraphPad Software, La Jolla, CA, USA). Values lower than 0.05 were considered statistically significant.

### Bibliography

Arai, T., M. Hasegawa, et al. (2006). "TDP-43 is a component of ubiquitin-positive tau-negative inclusions in frontotemporal lobar degeneration and amyotrophic lateral sclerosis." Biochem Biophys Res Commun 351(3): 602-611.
Arighi, C. N., Hartnell, L. M., Aguilar, R. C., Haft, C. R. & Bonifacino, J. S. Role of the mammalian retromer in sorting of the cation-independent mannose 6-phosphate receptor. J Cell Biol 165, 123-133, doi:10.1083/jcb.200312055 (2004).
Bhalla, A., C. P. Vetanovetz, et al. (2012). "The location and trafficking routes of the neuronal retromer and its role in amyloid precursor protein transport." Neurobiology of disease 47(1): 126-134.
Bianchi, M., O. Bloom, et al. (1996). "Suppression of proinflammatory cytokines in monocytes by a tetravalent guanylhydrazone." The Journal of experimental medicine 183(3): 927-936.
Cleveland, D. W. (1999). "From Charcot to SOD1: mechanisms of selective motor neuron death in ALS." Neuron 24(3): 515-520.
Cheng, X. T. et al. Characterization of LAMP 1-labeled nondegradative lysosomal and endocytic compartments in neurons. J Cell Biol 217, 3127-3139, doi:10.1083/jcb.201711083 (2018).
Convertino, M., J. Das, et al. (2016). "Pharmacological Chaperones: Design and Development of New Therapeutic Strategies for the Treatment of Conformational Diseases." ACS chemical biology 11(6): 1471-1489.
Dewil, M., V. F. dela Cruz, et al. (2007). "Inhibition of p38 mitogen activated protein kinase activation and mutant SOD1(G93A)-induced motor neuron death." Neurobiology of disease 26(2): 332-341.
Du, Z. W. et al. Generation and expansion of highly pure motor neuron progenitors from human pluripotent stem cells. Nat Commun 6, 6626, doi:10.1038/ncomms7626 ncomms7626 [pii] (2015).
Futerman, A. H. and G. van Meer (2004). "The cell biology of lysosomal storage disorders." Nature reviews. Molecular cell biology 5(7): 554-565.
Gowing, G., T. Philips, et al. (2008). "Ablation of proliferating microglia does not affect motor neuron degeneration in amyotrophic lateral sclerosis caused by mutant superoxide dismutase." J Neurosci 28(41): 10234-10244.
Gurney, M. E., H. Pu, et al. (1994). "Motor neuron degeneration in mice that express a human Cu,Zn superoxide dismutase mutation." Science 264(5166): 1772-1775.
Hierro, A., A. L. Rojas, et al. (2007). "Functional architecture of the retromer cargo-recognition complex." Nature 449(7165): 1063-1067.
Huotari, J. and A. Helenius (2011). "Endosome maturation." The EMBO journal 30(17): 3481-3500.
Johnston, J. A., Dalton, M. J., Gurney, M. E. & Kopito, R. R. Formation of high molecular weight complexes of mutant Cu, Zn-superoxide dismutase in a mouse model for familial amyotrophic lateral sclerosis. Proc Natl Acad Sci U S A 97, 12571-12576, doi:10.1073/pnas.220417997, 220417997 [pii] (2000).
Kabashi, E. et al. Proteasomes remain intact, but show early focal alteration in their composition in a mouse model of amyotrophic lateral sclerosis. J Neurochem 105, 2353-2366, doi:10.1111/j.1471-4159.2008.05317.x (2008).
Lane, R. F. et al. Vps10 family proteins and the retromer complex in aging-related neurodegeneration and diabetes. J Neurosci 32, 14080-14086, doi:10.1523/JNEUROSCI.3359-12.2012 (2012).
Laurent-Matha, V., Derocq, D., Prebois, C., Katunuma, N. & Liaudet-Coopman, E. Processing of human cathepsin D is independent of its catalytic function and auto-activation: involvement of cathepsins L and B. J Biochem 139, 363-371, doi:10.1093/jb/mvj037 (2006).
Lucin, K. M., C. E. O'Brien, et al. (2013). "Microglial beclin 1 regulates retromer trafficking and phagocytosis and is impaired in Alzheimer's disease." Neuron 79(5): 873-886.
MacLeod, D. A., H. Rhinn, et al. (2013). "RAB7L1 interacts with LRRK2 to modify intraneuronal protein sorting and Parkinson's disease risk." Neuron 77(3): 425-439.
Martiney, J. A., A. J. Rajan, et al. (1998). "Prevention and treatment of experimental autoimmune encephalomyelitis by CNI-1493, a macrophage-deactivating agent." Journal of immunology 160(11): 5588-5595.
Mazzocchi N, De Ceglia R, Mazza D, Forti L, Muzio L, Menegon A. Fluorescence-Based Automated Screening Assay for the Study of the pH-Sensitive Channel ASIC1a. J Biomol Screen. 2016 Apr;21(4):372-80. doi: 10.1177/1087057115617455. Epub 2015 Nov 23. PMID: 26597957
Mecozzi, V. J., D. E. Berman, et al. (2014). "Pharmacological chaperones stabilize retromer to limit APP processing." Nat Chem Biol 10(6): 443-449.
Muhammad, A. et al. Retromer deficiency observed in Alzheimer's disease causes hippocampal dysfunction, neurodegeneration, and Abeta accumulation. Proc Natl Acad Sci U S A 105, 7327-7332, doi:10.1073/pnas.0802545105 (2008).
Nielsen, M. S. et al. The sortilin cytoplasmic tail conveys Golgi-endosome transport and binds the VHS domain of the GGA2 sorting protein. Embo J 20, 2180-2190, doi:10.1093/emboj/20.9.2180 (2001)
Norwood, S. J., D. J. Shaw, et al. (2011). "Assembly and solution structure of the core retromer protein complex." Traffic 12(1): 56-71.
Pan, X., Zaarur, N., Singh, M., Morin, P. & Kandror, K. V. Sortilin and retromer mediate retrograde transport of Glut4 in 3T3-L1 adipocytes. Mol Biol Cell 28, 1667-1675, doi:10.1091/mbc.E16-11-0777 (2017).
Riva, N., L. Chaabane, et al. (2014). "Defining peripheral nervous system dysfunction in the SOD-1G93A transgenic rat model of amyotrophic lateral sclerosis." J Neuropathol Exp Neurol 73(7): 658-670.
Rossi, C., M. Cusimano, et al. (2018). "Interleukin 4 modulates microglia homeostasis and attenuates the early slowly progressive phase of amyotrophic lateral sclerosis." Cell death & disease 9(2): 250.
Saxena, S., Cabuy, E. & Caroni, P. A role for motoneuron subtype-selective ER stress in disease manifestations of FALS mice. Nat Neurosci 12, 627-636, doi:10.1038/nn.2297 (2009)
Schreij, A. M., E. A. Fon, et al. (2016). "Endocytic membrane trafficking and neurodegenerative disease." Cellular and molecular life sciences : CMLS 73(8): 1529-1545.
Seaman, M.N., Marcusson, E.G., Cereghino, J.L., Emr, S.D., (1997). Endosome to Golgiretrieval of the vacuolar protein sorting receptor, Vps10p, requires thefunction of the VPS29, VPS30, and VPS35 gene products. J. Cell Biol. 137, 79-92.
Seaman, M. N. (2004). "Cargo-selective endosomal sorting for retrieval to the Golgi requires retromer." The Journal of cell biology 165(1): 111-122.
Seaman, M. N. (2005). "Recycle your receptors with retromer." Trends Cell Biol 15(2): 68-75.
Small, S. A., K. Kent, et al. (2005). "Model-guided microarray implicates the retromer complex in Alzheimer's disease." Ann Neurol 58(6): 909-919.
Small, S. A. and G. A. Petsko (2015). "Retromer in Alzheimer disease, Parkinson disease and other neurological disorders." Nature reviews. Neuroscience 16(3): 126-132.
Soo, K. Y., M. Halloran, et al. (2015). "Rab1-dependent ER-Golgi transport dysfunction is a common pathogenic mechanism in SOD1, TDP-43 and FUS-associated ALS." Acta neuropathologica 130(5): 679-697.
Stafa, K. et al. GTPase activity and neuronal toxicity of Parkinson's disease-associated LRRK2 is regulated by ArfGAP1. PLoS Genet 8, e1002526, doi:10.1371/journal.pgen.1002526 (2012).
Stevens, J. C., R. Chia, et al. (2010). "Modification of superoxide dismutase 1 (SOD1) properties by a GFP tag--implications for research into amyotrophic lateral sclerosis (ALS)." PLoS One 5(3): e9541.
Temkin, P., B. Lauffer, et al. (2011). "SNX27 mediates retromer tubule entry and endosome-to-plasma membrane trafficking of signalling receptors." Nature cell biology 13(6): 715-721.
van Dis, V., M. Kuijpers, et al. (2014). "Golgi fragmentation precedes neuromuscular denervation and is associated with endosome abnormalities in SOD1-ALS mouse motor neurons." Acta neuropathologica communications 2: 38.
Vilarino-Guell, C., C. Wider, et al. (2011). "VPS35 mutations in Parkinson disease." Am J Hum Genet 89(1): 162-167.
Walker, L. C., M. I. Diamond, et al. (2013). "Mechanisms of protein seeding in neurodegenerative diseases." JAMA neurology 70(3): 304-310.
Wen, L., F. L. Tang, et al. (2011). "VPS35 haploinsufficiency increases Alzheimer's disease neuropathology." J Cell Biol 195(5): 765-779.
Zavodszky, E., M. N. Seaman, et al. (2014). "Mutation in VPS35 associated with Parkinson's disease impairs WASH complex association and inhibits autophagy." Nat Commun 5: 3828.
Yuan M, Breitkopf SB, Yang X, Asara JM. A positive/negative ion-switching, targeted mass spectrometry-based metabolomics platform for bodily fluids, cells, and fresh and fixed tissue. Nat Protoc. 2012 Apr 12;7(5):872-81. doi: 10.1038/nprot.2012.024.

## Claims

1. A compound of Formula (I) 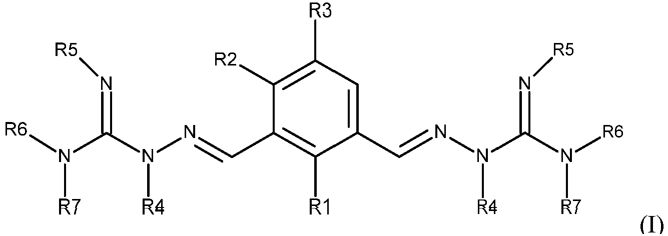 and its salts and solvates,
wherein:
R₁ is selected from hydrogen and halo;
R₂ is selected from hydrogen, halo, hydroxy, alkoxy and alkyl optionally substituted by a halogen atom, a hydroxy, a C₁-C₆ alkoxy, or an amino group;
R₃ is selected from hydrogen, halo, alkoxy and alkyl optionally substituted by a halogen atom, a hydroxy, a C₁-C₆ alkoxy, or an amino group, aralkyl, an optionally substituted aryl, which is optionally substituted by a halogen atom, a linear or branched C₁-C₆ alkyl, a hydroxy, a C₁-C₆ alkoxy, or an amino group, --NHCOR₈, - NHSO₂R₈, -COR₉, -NO₂, aminoguanidyl-hydrazone, -NHCOCH₃, and -NHSO₂CH₃;
R₄ is selected from hydrogen and alkyl optionally substituted by a halogen atom, a hydroxy, a C₁-C₆ alkoxy, or an amino group;
R₅ is selected from hydrogen and alkyl optionally substituted by a halogen atom, a hydroxy, a C₁-C₆ alkoxy, or an amino group;
R₆ is selected from hydrogen, alkyl optionally substituted by a halogen atom, a hydroxy, a C₁-C₆ alkoxy, or an amino group, an optionally substituted aryl, which is optionally substituted by a halogen atom, a linear or branched C₁-C₆ alkyl, a hydroxy, a C₁-C₆ alkoxy, or an amino group, and arylalkyl;
R₇ is selected from hydrogen and alkyl optionally substituted by a halogen atom, a hydroxy, a C₁-C₆ alkoxy, or an amino group;
or
R₅ and R₆ together with the two nitrogen atoms to which they are bound, form a cycle selected from imidazolino, tetrahydropyrimidino and benzimidazolino;
or
R₆ and R₇, each independently, together with the nitrogen atom to which they are bound, form a pyrrolidino or a piperidino group;
R₈ is selected from hydrogen, alkyl substituted by a halogen atom, a hydroxy, a C₁-C₆ alkoxy, or an amino group,and an optionally substituted aryl, which is optionally substituted by a halogen atom, a linear or branched C₁-C₆ alkyl, a hydroxy, a C₁-C₆ alkoxy, or an amino group;
R₉ is selected from groups of Formula (IIIa), (IIIb), (IIIc), (IIId) and (IIIe) 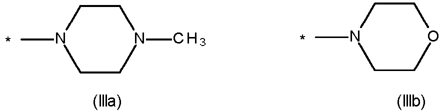 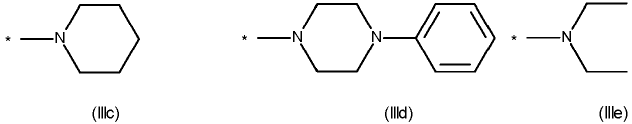
wherein the asterisk (*) indicates the linking bond;
as well as compounds of formula (I) wherein
- R₃ is acetamido and R₁, R₂, R₄, R₅, R₆, R₇ are all hydrogen (Compound 2l)
- R₃ is mesylamido and R₁, R₂, R₄, R₅, R₆, R₇ are all hydrogen (Compound 2n)
and
- R₃ is carbomethoxy and R₁, R₂, R₄, R₅, R₆, R₇ are all hydrogen (Compound 2p);
provided that said compound of formula (I) is not one of the following:
- R₁, R₂, R₃, R₄, R₅, R₆, R₇ are all hydrogen (Compound 2a)
- R₂ is hydroxyl and R₁, R₃, R₄, R₅, R₆, R₇ are all hydrogen (Compound 2d)
- R₃ is bromine and R₁, R₂, R₄, R₅, R₆, R₇ are all hydrogen (Compound 2h)
- R3 is guanidinoiminomethyl and R1, R2, R4, R5, R6, R7 are all hydrogen (Compound 2r)
- R5 and R6 together with the nitrogen atom to which they are bond form a imidazolidino group and R1, R2, R3, R4, R7 are all hydrogen (Compound 2x)
- R5 and R6 together with the nitrogen atoms to which they are bond form a benzimidazolidino group and R1, R2, R3, R4, R7 are all hydrogen (Compound 2z).

2. The compound of Formula (I) according to claim 1, **characterized in that**
R₁ is selected from hydrogen and bromine;
R₂ is selected from hydrogen, bromine, hydroxy, methoxy, methyl, n-butyl and n-butyloxy;
R₃ is selected from hydrogen, bromine, methoxy, -NO₂, phenyl, -NHCOCH₃, -NHCO-p-tolyl, -NHSO₂CH₃, -NHSO₂-p-tolyl, -CO-R₉ and aminoguanidyl-hydrazone;
R₄ is selected from hydrogen and methyl;
R₅ is hydrogen;
R₆ is selected from hydrogen, methyl, n-butyl and benzyl;
R₇ is hydrogen,
or
R₅ and R₆, together with the two nitrogen atoms to which they are bound, form a cycle selected from imidazolidino, tetrahydropyrimidino and benzimidazolino;
or
R₆ and R₇, together with the nitrogen atom to which they are bound, form a pyrrolidino group;
R₉ is selected from groups of Formula (IIIa), (IIIb), (IIIc), (IIId) and (IIIe) 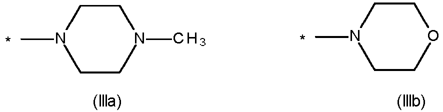 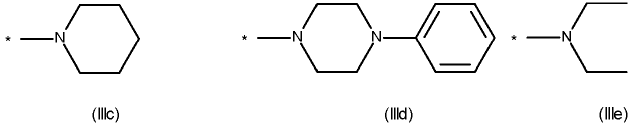
wherein the asterisk (*) indicates the linking bond,
or one of its salts or solvates.

3. The compound of Formula (I) according to any one of claims 1 or 2, selected from compounds of Formula (I) wherein
- R₁ is bromine and R₂, R₃, R₄, R₅, R₆, R₇ are all hydrogen (Compound 2b)
- R₂ is bromine and R₁, R₃, R₄, R₅, R₆, R₇ are all hydrogen (Compound 2c)
- R₂ is methoxy and R₁, R₃, R₄, R₅, R₆, R₇ are all hydrogen (Compound 2e)
- R₂ is n-butyloxy and R₁, R₃, R₄, R₅, R₆, R₇ are all hydrogen (Compound 2f)
- R₃ is methyl and R₁, R₂, R₄, R₅, R₆, R₇ are all hydrogen (Compound 2g)
- R₃ is methoxy and R₁, R₂, R₄, R₅, R₆, R₇ are all hydrogen (Compound 2i)
- R₃ is nitro and R₁, R₂, R₄, R₅, R₆, R₇ are all hydrogen (Compound 2j)
- R₃ is phenyl and R₁, R₂, R₄, R₅, R₆, R₇ are all hydrogen (Compound 2k)
- R₃ is acetamido and R₁, R₂, R₄, R₅, R₆, R₇ are all hydrogen (Compound 2l)
- R₃ is p-tolylamido and R₁, R₂, R₄, R₅, R₆, R₇ are all hydrogen (Compound 2m)
- R₃ is mesylamido and R₁, R₂, R₄, R₅, R₆, R₇ are all hydrogen (Compound 2n)
- R₃ is p-tosylamido and R₁, R₂, R₄, R₅, R₆, R₇ are all hydrogen (Compound 2o)
- R₃ is carbomethoxy and R₁, R₂, R₄, R₅, R₆, R₇ are all hydrogen (Compound 2p)
- R₃ is 1-(4'-phenylpiperazinyl)carbonyl group and R₁, R₂, R₄, R₅, R₆, R₇ are all hydrogen (Compound 2q)
- R₆ is n-butyl and R₁, R₂, R₃, R₄, R₅, R₇ are all hydrogen (Compound 2t)
- R₆ is benzyl and R₁, R₂, R₃, R₄, R₅, R₇ are all hydrogen (Compound 2u)
- R₆ and R₇ together with the nitrogen atom to which they are bond form a pyrrolidino group and R₁, R₂, R₃, R₄, R₅ are all hydrogen (Compound 2v)
- R₃ is p-tolylamido, R₆ is n-butyl and R₁, R₂, R₄, R₅, R₇ are all hydrogen (Compound 2w)
- R₄ is methyl, R₅ and R₆ together with the nitrogen atoms to which they are bond form a imidazolidino group and R₁, R₂, R₃, R₇ are all hydrogen (Compound 2y) or one of its salts or solvates.

4. The compound of of Formula (I) according to claim 3, selected from Compounds 2b, 2c, 2e, 2f, 2g, 2i, 2l, 2u and 2v.

5. The compound of Formula (I) as claimed in any one of claims 1 to 4, characterized that it is in the form of one of its salts.

6. A pharmaceutical composition comprising at least one compound of Formula (I) as claimed in any one of claims 1 to 5, or of a pharmacetically acceptable salt or solvate thereof, and at least one pharmacetically acceptable carrier or excipient.

7. A compound of Formula (I) as claimed in any one of claims 1 to 5, or of a pharmacetically acceptable salt or solvate thereof, for its use in therapy and/or diagnostics.

8. A compound of Formula (I) 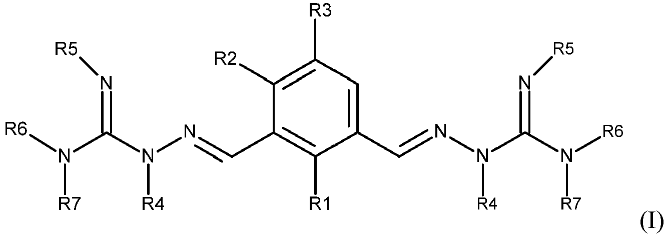 and its pharmaceutically acceptable salts and solvates,
wherein:
R₁ is selected from hydrogen and halo;
R₂ is selected from hydrogen, halo, hydroxy, alkoxy and alkyl;
R₃ is selected from hydrogen, halo, hydroxy, alkoxy and alkyl, aralkyl, aryl, -COOR₈, -NHCOR₈, -NHSO₂R₈, -COR₉, -NO₂, aminoguanidyl-hydrazone, -NHCOCH₃ and -NHSO₂CH₃;
R₄ is selected from hydrogen and alkyl;
R₅ is selected from hydrogen and alkyl;
R₆ is selected from hydrogen, alkyl, aryl and arylalkyl;
R₇ is selected from hydrogen and alkyl;
or
R₅ and R₆ together with the two nitrogen atoms to which they are bound, form a cycle selected from imidazolino, tetrahydropyrimidino and benzimidazolino;
or
R₆ and R₇, each independently, together with the nitrogen atom to which they are bound, form a pyrrolidino or a piperidino group;
R₈ is selected from hydrogen, alkyl substituted by a halogen atom, a hydroxy, a C₁-C₆ alkoxy, or an amino group, and an optionally substituted aryl, which is optionally substituted by a halogen atom, a linear or branched C₁-C₆ alkyl, a hydroxy, a C₁-C₆ alkoxy, or an amino group;
R₉ is selected from groups of Formula (IIIa), (IIIb), (IIIc), (IIId) and (IIIe) 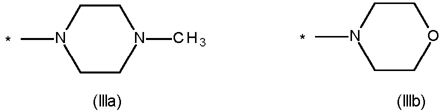 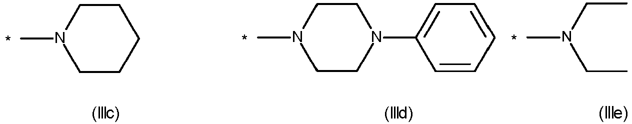
wherein the asterisk (*) indicates the linking bond;
as well as compounds of formula (I) wherein
- R₃ is acetamido and R₁, R₂, R₄, R₅, R₆, R₇ are all hydrogen (Compound 2l)
- R₃ is mesylamido and R₁, R₂, R₄, R₅, R₆, R₇ are all hydrogen (Compound 2n)
and
R₃ is carbomethoxy and R₁, R₂, R₄, R₅, R₆, R₇ are all hydrogen (Compound 2p) for its use in the treatment and/or prevention of neurological diseases, including amyotrophic lateral sclerosis (ALS), Charcot-Marie Tooth disease, Azheimer's disease (AD), Parkinson's disease (PD).

9. A process for the preparation of a compound of Formula (I) or one of its salts or solvates, according to any one of claims 1 to 5, which comprises reacting a compound of Formula (IV) 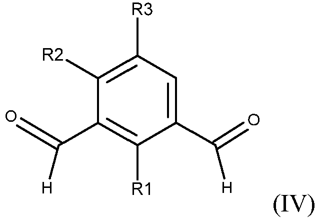 wherein R₁, R₂ and R₃ are as above defined, with a compound of Formula (V) 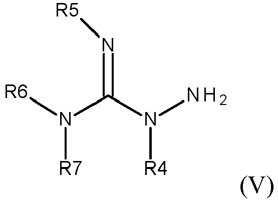 or a salt thereof, wherein R₄, R₅, R₆, and R₃ are as above defined, in a suitable solvent, and isolating the compound of Formula (I) thus obtained, optionally purifying and/o optionally converting it in a salt or solvate thereof.

## Patentansprüche

1. Eine Verbindung der Formel (I) 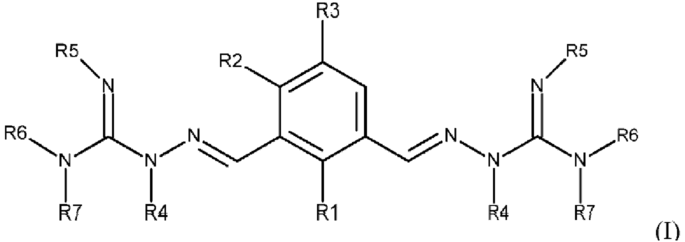 und ihre Salze und Solvate,
wobei:
R₁ aus Wasserstoff und Halogen ausgewählt ist;
R₂ aus Wasserstoff, Halogen, Hydroxy, Alkoxy und Alkyl, optional substituiert mit einem Halogenatom, einem Hydroxy, einem C₁-C₆-Alkoxy oder einer Aminogruppe, ausgewählt ist;
R₃ aus Wasserstoff, Halogen, Alkoxy und Alkyl, optional substituiert mit einem Halogenatom, einem Hydroxy, einem C₁-C₆-Alkoxy oder einer Aminogruppe, Aralkyl, einem optional substituierten Aryl, das optional durch ein Halogenatom, ein lineares oder verzweigtes C₁-C₆-Alkyl, ein Hydroxy, ein C₁-C₆-Alkoxy oder eine Aminogruppe substituiert ist, -NHCOR₈, -NHSO₂R₈, -COR₉, -NO₂, Aminoguanidylhydrazon, - NHCOCH₃ und -NHSO₂CH₃ ausgewählt ist;
R₄ aus Wasserstoff und Alkyl, optional substituiert mit einem Halogenatom, einem Hydroxy, einem C₁-C₆-Alkoxy oder einer Aminogruppe, ausgewählt ist;
R₅ aus Wasserstoff und Alkyl, optional substituiert mit einem Halogenatom, einem Hydroxy, einem C₁-C₆-Alkoxy oder einer Aminogruppe, ausgewählt ist;
R₆ aus Wasserstoff, Alkyl, optional substituiert mit einem Halogenatom, einem Hydroxy, einem C₁-C₆-Alkoxy oder einer Aminogruppe, einem optional substituierten Aryl, das optional durch ein Halogenatom, ein lineares oder verzweigtes C₁-C₆-Alkyl, ein Hydroxy, ein C₁-C₆-Alkoxy oder eine Aminogruppe substituiert ist, und Arylalkyl ausgewählt ist;
R₇ aus Wasserstoff und Alkyl, optional substituiert mit einem Halogenatom, einem Hydroxy, einem C₁-C₆-Alkoxy oder einer Aminogruppe, ausgewählt ist;
oder
R₅ und R₆ zusammen mit den beiden Stickstoffatomen, an die sie gebunden sind, einen Zyklus bilden, ausgewählt aus Imidazolino, Tetrahydropyrimidino und Benzim idazolino;
oder
R₆ und R₇ jeweils unabhängig voneinander zusammen mit dem Stickstoffatom, an das sie gebunden sind, eine Pyrrolidino- oder eine Piperidinogruppe bilden;
R₈ aus Wasserstoff, Alkyl, substituiert mit einem Halogenatom, einem Hydroxy, einem C₁-C₆-Alkoxy oder einer Aminogruppe, und einem optional substituierten Aryl, das optional durch ein Halogenatom, ein lineares oder verzweigtes C₁-C₆-Alkyl, ein Hydroxy, ein C₁-C₆-Alkoxy oder eine Aminogruppe substituiert ist, ausgewählt ist;
R₉ aus den Gruppen der Formel (IIIa), (IIIb), (IIIc), (IIId) und (IIIe) 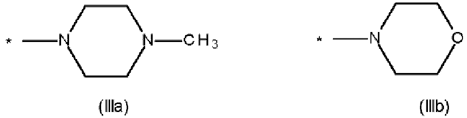 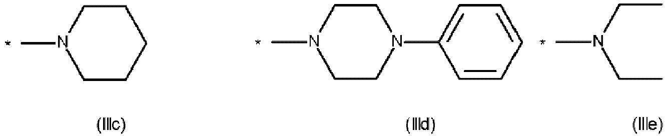
ausgewählt ist, wobei das Sternchen (*) die verbindende Bindung anzeigt;
und Verbindungen der Formel (I), wobei
- R₃ Acetamido ist und R₁, R₂, R₄, R₅, R₆, R₇ alle Wasserstoff sind (Verbindung 21)
- R₃ Mesylamido ist und R₁, R₂, R₄, R₅, R₆, R₇ alle Wasserstoff sind (Verbindung 2n)
und
- R₃ Carbomethoxy ist und R₁, R₂, R₄, R₅, R₆, R₇ alle Wasserstoff sind (Verbindung 2p);
unter der Bedingung, dass die Verbindung der Formel (I) keine der folgenden Verbindungen ist:
- R₁, R₂, R₃, R₄, R₅, R₆, R₇ sind alle Wasserstoff (Verbindung 2a)
- R₂ ist Hydroxyl und R₁, R₃, R₄, R₅, R₆, R₇ sind alle Wasserstoff (Verbindung 2d)
- R₃ ist Brom und R₁, R₂, R₄, R₅, R₆, R₇ sind alle Wasserstoff (Verbindung 2h)
- R₃ ist Guanidinoiminomethyl und R₁, R₂, R₄, R₅, R₆, R₇ sind alle Wasserstoff (Verbindung 2r)
- R₅ und R₆ bilden zusammen mit dem Stickstoffatom, an das sie gebunden sind, eine Imidazolidinogruppe und R₁, R₂, R₃, R₄, R₇ sind alle Wasserstoff (Verbindung 2x)
- R₅ und R₆ bilden zusammen mit den Stickstoffatomen, an die sie gebunden sind, eine Benzimidazolidinogruppe und R₁, R₂, R₃, R₄, R₇ sind alle Wasserstoff (Verbindung 2z).

2. Die Verbindung der Formel (I) gemäß Anspruch 1, **dadurch gekennzeichnet, dass**
R₁ aus Wasserstoff und Brom ausgewählt ist;
R₂ aus Wasserstoff, Brom, Hydroxy, Methoxy, Methyl, n-Butyl und n-Butyloxy ausgewählt ist;
R₃ aus Wasserstoff, Brom, Methoxy, -NO₂, Phenyl, -NHCOCH₃, -NHCO-p-tolyl, - NHSO₂CH₃, -NHSO₂-p-tolyl, -CO-R₉ und Aminoguanidylhydrazon ausgewählt ist;
R₄ aus Wasserstoff und Methyl ausgewählt ist;
R₅ Wasserstoff ist;
R₆ aus Wasserstoff, Methyl, n-Butyl und Benzyl ausgewählt ist;
R₇ Wasserstoff ist,
oder
R₅ und R₆ zusammen mit den beiden Stickstoffatomen, an die sie gebunden sind, einen Zyklus bilden, ausgewählt aus Imidazolidino, Tetrahydropyrimidino und Benzim idazolino;
oder
R₆ und R₇ zusammen mit dem Stickstoffatom, an das sie gebunden sind, eine Pyrrolidinogruppe bilden;
R₉ aus den Gruppen der Formel (IIIa), (Illb), (IIIc), (IIId) und (IIIe) 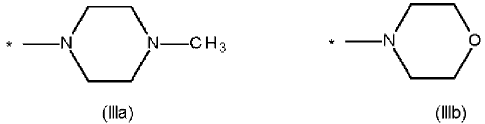 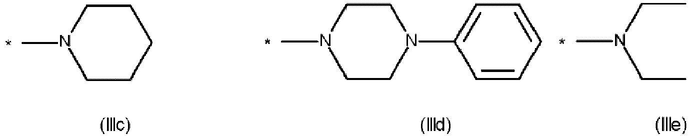
ausgewählt ist,
wobei das Sternchen (*) die verbindende Bindung anzeigt,
oder eines ihrer Salze oder Solvate.

3. Die Verbindung der Formel (I) gemäß einem der Ansprüche 1 oder 2, ausgewählt aus Verbindungen der Formel (I), wobei
- R₁ Brom ist und R₂, R₃, R₄, R₅, R₆, R₇ alle Wasserstoff sind (Verbindung 2b)
- R₂ Brom ist und R₁, R₃, R₄, R₅, R₆, R₇ alle Wasserstoff sind (Verbindung 2c)
- R₂ Methoxy ist und R₁, R₃, R₄, R₅, R₆, R₇ alle Wasserstoff sind (Verbindung 2e)
- R₂ n-Butyloxy ist und R₁, R₃, R₄, R₅, R₆, R₇ alle Wasserstoff sind (Verbindung 2f)
- R₃ Methyl ist und R₁, R₂, R₄, R₅, R₆, R₇ alle Wasserstoff sind (Verbindung 2g)
- R₃ Methoxy ist und R₁, R₂, R₄, R₅, R₆, R₇ alle Wasserstoff sind (Verbindung 2i)
- R₃ Nitro ist und R₁, R₂, R₄, R₅, R₆, R₇ alle Wasserstoff sind (Verbindung 2j)
- R₃ Phenyl ist und R₁, R₂, R₄, R₅, R₆, R₇ alle Wasserstoff sind (Verbindung 2k)
- R₃ Acetamido ist und R₁, R₂, R₄, R₅, R₆, R₇ alle Wasserstoff sind (Verbindung 21)
- R₃ p-Tolylamido ist und R₁, R₂, R₄, R₅, R₆, R₇ alle Wasserstoff sind (Verbindung 2m)
- R₃ Mesylamido ist und R₁, R₂, R₄, R₅, R₆, R₇ alle Wasserstoff sind (Verbindung 2n)
- R₃ p-Tosylamido ist und R₁, R₂, R₄, R₅, R₆, R₇ alle Wasserstoff sind (Verbindung 2o)
- R₃ Carbomethoxy ist und R₁, R₂, R₄, R₅, R₆, R₇ alle Wasserstoff sind (Verbindung 2p)
- R₃ eine 1-(4'-Phenylpiperazinyl)carbonylgruppe ist und R₁, R₂, R₄, R₅, R₆, R₇ alle Wasserstoff sind (Verbindung 2q)
- R₆ n-Butyl ist und R₁, R₂, R₃, R₄, R₅, R₇ alle Wasserstoff sind (Verbindung 2t)
- R₆ Benzyl ist und R₁, R₂, R₃, R₄, R₅, R₇ alle Wasserstoff sind (Verbindung 2u)
- R₆ und R₇ zusammen mit dem Stickstoffatom, an das sie gebunden sind, eine Pyrrolidinogruppe bilden und R₁, R₂, R₃, R₄, R₅ alle Wasserstoff sind (Verbindung 2v)
- R₃ Tosylamido ist, R₆ n-Butyl ist und R₁, R₂, R₄, R₅, R₇ alle Wasserstoff sind (Verbindung 2w)
- R₄ Methyl ist, R₅ und R₆ zusammen mit den Stickstoffatomen, an die sie gebunden sind, eine Imidazolidinogruppe bilden und R₁, R₂, R₃, R₇ alle Wasserstoff sind (Verbindung 2y)
oder eines ihrer Salze oder Solvate.

4. Die Verbindung der Formel (I) gemäß Anspruch 3, ausgewählt aus den Verbindungen 2b, 2c, 2e, 2f, 2g, 2i, 2l, 2u und 2v.

5. Die Verbindung der Formel (I) gemäß einem der Ansprüche 1 bis 4, **dadurch gekennzeichnet, dass** sie in Form eines ihrer Salze vorliegt.

6. Eine pharmazeutische Zusammensetzung, umfassend mindestens eine Verbindung der Formel (I) gemäß einem der Ansprüche 1 bis 5 oder ein pharmazeutisch verträgliches Salz oder Solvat davon und mindestens einen pharmazeutisch verträglichen Träger oder Hilfsstoff.

7. Eine Verbindung der Formel (I) gemäß einem der Ansprüche 1 bis 5 oder ein pharmazeutisch verträgliches Salz oder Solvat davon zur Verwendung in der Therapie und/oder Diagnostik.

8. Eine Verbindung der Formel (I) 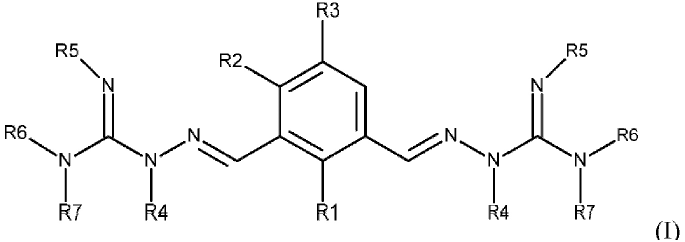 und ihre pharmazeutisch verträglichen Salze und Solvate,
wobei:
R₁ aus Wasserstoff und Halogen ausgewählt ist;
R₂ aus Wasserstoff, Halogen, Hydroxy, Alkoxy und Alkyl ausgewählt ist;
R₃ aus Wasserstoff, Halogen, Hydroxy, Alkoxy und Alkyl, Aralkyl, Aryl, -COOR₈, - NHCOR₈, -NHSO₂R₈, -COR₉, -NO₂ und Aminoguanidylhydrazon, -NHCOCH₃ und - NHSO₂CH₃ ausgewählt ist;
R₄ aus Wasserstoff und Alkyl ausgewählt ist;
R₅ aus Wasserstoff und Alkyl ausgewählt ist;
R₆ aus Wasserstoff, Alkyl, Aryl und Arylalkyl ausgewählt ist;
R₇ ist aus Wasserstoff und Alkyl ausgewählt;
oder
R₅ und R₆ zusammen mit den beiden Stickstoffatomen, an die sie gebunden sind, einen Zyklus bilden, ausgewählt aus Imidazolino, Tetrahydropyrimidino und Benzim idazolino;
oder
R₆ und R₇ jeweils unabhängig voneinander zusammen mit dem Stickstoffatom, an das sie gebunden sind, eine Pyrrolidino- oder eine Piperidinogruppe bilden;
R₈ aus Wasserstoff, Alkyl, substituiert mit einem Halogenatom, einem Hydroxy, einem C₁-C₆-Alkoxy oder einer Aminogruppe, und einem optional substituierten Aryl, das optional durch ein Halogenatom, ein lineares oder verzweigtes C₁-C₆-Alkyl, ein Hydroxy, ein C₁-C₆-Alkoxy oder eine Aminogruppe substituiert ist, ausgewählt ist;
R₉ aus den Gruppen der Formel (IIIa), (Illb), (IIIc), (IIId) und (IIIe) 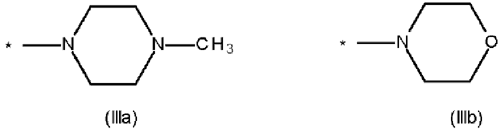 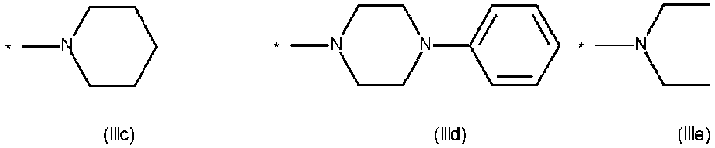 ausgewählt ist, wobei das Sternchen (*) die verbindende Bindung anzeigt;
und Verbindungen der Formel (I), wobei
- R₃ Acetamido ist und R₁, R₂, R₄, R₅, R₆, R₇ alle Wasserstoff sind (Verbindung 21)
- R₃ Mesylamido ist und R₁, R₂, R₄, R₅, R₆, R₇ alle Wasserstoff sind (Verbindung 2n)
und
- R₃ Carbomethoxy ist und R₁, R₂, R₄, R₅, R₆, R₇ alle Wasserstoff sind (Verbindung 2p)
zur Verwendung in der Behandlung und/oder Vorbeugung neurologischer Erkrankungen, einschließlich amyotropher Lateralsklerose (ALS), Charcot-Marie-Tooth-Krankheit, Alzheimer-Krankheit (AD), Parkinson-Krankheit (PD).

9. Verfahren zur Herstellung einer Verbindung der Formel (I) oder eines ihrer Salze oder Solvate gemäß einem der Ansprüche 1 bis 5, umfassend die Umsetzung einer Verbindung der Formel (IV) 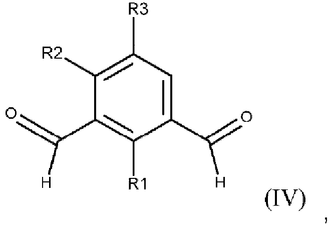 wobei R₁, R₂, und R₃ wie oben definiert sind, mit einer Verbindung der Formel (V) 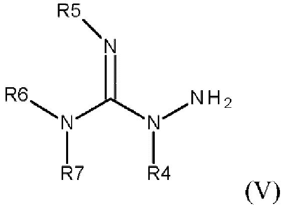 oder eines Salzes davon, wobei R₄, R₅, R₆ und R₃ wie oben definiert sind, in einem geeigneten Lösungsmittel und Isolieren der so erhaltenen Verbindung der Formel (I), optionales Reinigen und/oder optionales Umwandeln in ein Salz oder Solvat davon.

## Revendications

1. Composé de formule (I) 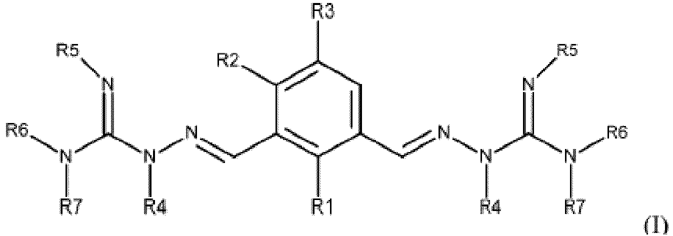 et ses sels et solvates, où :
R₁ est choisi parmi l'hydrogène et l'halo ;
R₂ est choisi parmi l'hydrogène, l'halo, l'hydroxy, l'alcoxy et l'alkyle éventuellement substitué par un atome d'halogène, un hydroxy, un alcoxy en C₁-C₆ ou un groupe amino ;
R₃ est choisi parmi l'hydrogène, l'halo, l'alcoxy et l'alkyle éventuellement substitué par un atome d'halogène, un hydroxy, un alcoxy en C₁-C₆ ou un groupe amino, un aralkyle, un aryle éventuellement substitué, qui est éventuellement substitué par un atome d'halogène, un alkyle C₁-C₆ linéaire ou ramifié, un hydroxy, un alcoxy en C₁-C₆ ou un groupe amino, --NHCOR₈, - NHSO₂R₈, -COR₉, -NO₂, l'aminoguanidyl-hydrazone, -NHCOCH₃, et -NHSO₂CH₃ ;
R₄ est choisi parmi l'hydrogène et l'alkyle éventuellement substitué par un atome d'halogène, un hydroxy, un alcoxy en C₁-C₆ ou un groupe amino ;
R₅ est choisi parmi l'hydrogène et l'alkyle éventuellement substitué par un atome d'halogène, un hydroxy, un alcoxy en C₁-C₆ ou un groupe amino ;
R₆ est choisi parmi l'hydrogène, l'alkyle éventuellement substitué par un atome d'halogène, un hydroxy, un alcoxy en C₁-C₆ ou un groupe amino, un aryle éventuellement substitué, qui est éventuellement substitué par un atome d'halogène, un alkyle en C₁-C₆ linéaire ou ramifié, un hydroxy, un alcoxy en C₁-C₆ ou un groupe amino, et l'arylalkyle ;
R₇ est choisi parmi l'hydrogène et l'alkyle éventuellement substitué par un atome d'halogène, un hydroxy, un alcoxy en C₁-C₆ ou un groupe amino ;
ou
R₅ et R₆ forment, avec les deux atomes d'azote auxquels ils sont liés, un cycle choisi parmi les imidazolino, les tétrahydropyrimidino et les benzimidazolino ;
ou
R₆ et R₇, chacun indépendamment, forment, avec l'atome d'azote auquel ils sont liés, un groupe pyrrolidino ou piperidino ;
R₈ est choisi parmi l'hydrogène, l'alkyle substitué par un atome d'halogène, un hydroxy, un alcoxy en C₁-C₆ ou un groupe amino, et un aryle éventuellement substitué, qui est éventuellement substitué par un atome d'halogène, un alkyle en C₁-C₆ linéaire ou ramifié, un hydroxy, un alcoxy en C₁-C₆ ou un groupe amino ;
R₉ est choisi parmi les groupes de formule (IIIa), (IIIb), (IIIc), (IIId) et (IIIe) 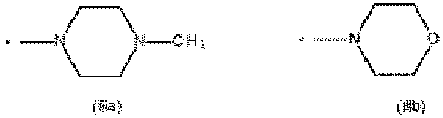 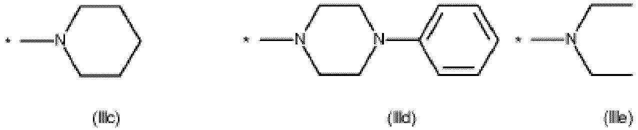
dans lequel l'astérisque (*) indique le lien de liaison ;
ainsi que les composés de formule (I) dans lesquels
- R₃ est de l'acétamido et R₁, R₂, R₄, R₅, R₆, R₇ sont tous de l'hydrogène (composé 2l)
- R₃ est du mésylamido et R₁, R₂, R₄, R₅, R₆, R₇ sont tous de l'hydrogène (composé 2n) et
- R₃ est du carbométhoxy et R₁, R₂, R₄, R₅, R₆, R₇ sont tous de l'hydrogène (composé 2p) ; à condition que ladite composé de formule (I) ne soit pas l'une des suivants :
- R₁, R₂, R₃, R₄, R₅, R₆, R₇ sont tous de l'hydrogène (composé 2a)
- R₂ est du hydroxyle et R₁, R₃, R₄, R₅, R₆, R₇ sont tous de l'hydrogène (composé 2d)
- R₃ est du brome et R₁, R₂, R₄, R₅, R₆, R₇ sont tous de l'hydrogène (composé 2h)
- R3 est du guanidinoiminométhyle et R1, R2, R4, R5, R6, R7 sont tous de l'hydrogène (composé 2r)
- R5 et R6 forment, avec l'atome d'azote auquel ils sont liés, un groupe imidazolidino et R1, R2, R3, R4, R7 sont tous de l'hydrogène (composé 2x)
- R5 et R6 forment, avec les atomes d'azote auxquels ils sont liés, un groupe benzimidazolidino et R1, R2, R3, R4, R7 sont tous de l'hydrogène (composé 2z).

2. Composé de formule (I) selon la revendication 1, **caractérisée en ce que**
R₁ est choisi parmi l'hydrogène et le brome ;
R₂ est choisi parmi l'hydrogène, le brome, l'hydroxy, le méthoxy, le méthyle, le n-butyle et le n-butyloxy ;
R₃ est choisi parmi l'hydrogène, le brome, le méthoxy, -NO₂, le phényle, -NHCOCH₃, -NHCO-p-tolyle, -NHSO₂CH₃, -NHSO₂-p-tolyle, -CO-R₉ et l'aminoguanidyl-hydrazone ;
R₄ est choisi parmi l'hydrogène et le méthyle ;
R₅ est l'hydrogène ;
R₆ est choisi parmi l'hydrogène, le méthyle, le n-butyle et le benzyle ;
R₇ est l'hydrogène,
ou
R₅ et R₆ forment, avec les deux atomes d'azote auxquels ils sont liés, un cycle choisi parmi les imidazolidino, les tétrahydropyrimidino et les benzimidazolino ;
ou
R₆ et R₇ forment, avec l'atome d'azote auquel ils sont liés, un groupe pyrrolidino ;
R₉ est choisi parmi les groupes de formule (IIIa), (IIIb), (IIIc), (IIId) et (IIIe) 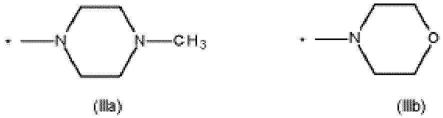 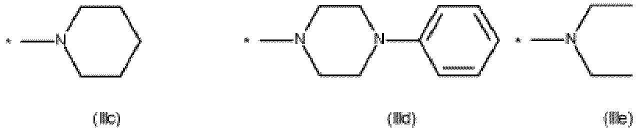
où l'astérisque (*) indique le lien de liaison,
ou l'un de ses sels ou solvates.

3. Composé de formule (I) selon l'une quelconque des revendications 1 ou 2, choisi parmi les composés de formule (I) où
- R₁ est du brome et R₂, R₃, R₄, R₅, R₆, R₇ sont tous de l'hydrogène (composé 2b)
- R₂ est du brome et R₁, R₃, R₄, R₅, R₆, R₇ sont tous de l'hydrogène (composé 2c)
- R₂ est du méthoxy et R₁, R₃, R₄, R₅, R₆, R₇ sont tous de l'hydrogène (composé 2e)
- R₂ est du n-butyloxy et R₁, R₃, R₄, R₅, R₆, R₇ sont tous de l'hydrogène (composé 2f)
- R₃ est du méthyle et R₁, R₂, R₄, R₅, R₆, R₇ sont tous de l'hydrogène (composé 2g)
- R₃ est du méthoxy et R₁, R₂, R₄, R₅, R₆, R₇ sont tous de l'hydrogène (composé 2i)
- R₃ est du nitro et R₁, R₂, R₄, R₅, R₆, R₇ sont tous de l'hydrogène (composé 2j)
- R₃ est du phényle et R₁, R₂, R₄, R₅, R₆, R₇ sont tous de l'hydrogène (composé 2k)
- R₃ est de l'acétamido et R₁, R₂, R₄, R₅, R₆, R₇ sont tous de l'hydrogène (composé 2l)
- R₃ est du p-tolylamido et R₁, R₂, R₄, R₅, R₆, R₇ sont tous de l'hydrogène (composé 2m)
- R₃ est du mésylamido et R₁, R₂, R₄, R₅, R₆, R₇ sont tous de l'hydrogène (composé 2n)
- R₃ est du p-tosylamido et R₁, R₂, R₄, R₅, R₆, R₇ sont tous de l'hydrogène (composé 2o)
- R₃ est du carbométhoxy et R₁, R₂, R₄, R₅, R₆, R₇ sont tous de l'hydrogène (composé 2p)
- R₃ est un groupe 1-(4'-phénylpipérazinyl)carbonyle et R₁, R₂, R₄, R₅, R₆, R₇ sont tous de l'hydrogène (composé 2q)
- R₆ est du n-butyle et R₁, R₂, R₃, R₄, R₅, R₇ sont tous de l'hydrogène (composé 2t)
- R₆ est du benzyle et R₁, R₂, R₃, R₄, R₅, R₇ sont tous de l'hydrogène (composé 2u)
- R₆ et R₇ forment, avec l'atome d'azote auquel ils sont liés, un groupe pyrrolidino et R₁, R₂, R₃, R₄, R₅ sont tous de l'hydrogène (composé 2v)
- R₃ est du p-tolylamido, R₆ est n-butyle et R₁, R₂, R₄, R₅, R₇ sont tous de l'hydrogène (composé 2w)
- R₄ est du méthyle, R₅ et R₆ forment, avec les atomes d'azote auxquels ils sont liés, un groupe imidazolidino et R₁, R₂, R₃, R₇ sont tous de l'hydrogène (composé 2y) ou l'un de ses sels ou solvates.

4. Composé de formule (I) selon la revendication 3, choisie parmi les composés 2b, 2c, 2e, 2f, 2g, 2i, 2l, 2u et 2v.

5. Composé de formule (I) selon l'une quelconque des revendications 1 à 4, **caractérisée en ce qu'**elle se présente sous la forme d'un de ses sels.

6. Composition pharmaceutique comprenant au moins un composé de formule (I) selon l'une quelconque des revendications 1 à 5, ou l'un de ses sels ou solvates pharmaceutiquement acceptable, et au moins un support ou excipient pharmaceutiquement acceptable.

7. Composé de formule (I) selon l'une quelconque des revendications 1 à 5, ou du sel ou solvate pharmaceutiquement acceptable de celui-ci, pour son utilisation dans la thérapie et/ou le diagnostic.

8. Composé de formule (I) 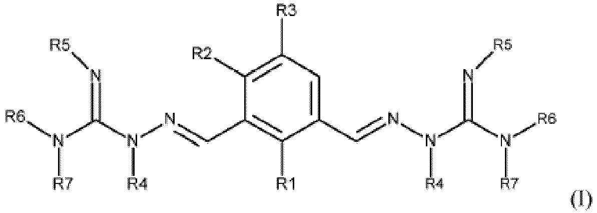
et ses sels et solvates pharmaceutiquement acceptables,
où :
R₁ est choisi parmi l'hydrogène et l'halo ;
R₂ est choisi parmi l'hydrogène, l'halo, l'hydroxy, l'alcoxy et l'alkyle ;
R₃ est choisi parmi l'hydrogène, l'halo, l'hydroxy, l'alcoxy et l'alkyle, l'aralkyle, l'aryle, -COOR₈, -NHCOR₈, -NHSO₂R₈, -COR₉, -NO₂, l'aminoguanidyl-hydrazone, -NHCOCH₃ et - NHSO₂CH₃ ;
R₄ est choisi parmi l'hydrogène et l'alkyle ;
R₅ est choisi parmi l'hydrogène et l'alkyle ;
R₆ est choisi parmi l'hydrogène, l'alkyle, l'aryle et l'arylalkyle ;
R₇ est choisi parmi l'hydrogène et l'alkyle ;
ou
R₅ et R₆ forment, avec les deux atomes d'azote auxquels ils sont liés, un cycle choisi parmi les imidazolino, les tétrahydropyrimidino et les benzimidazolino ;
ou
R₆ et R₇, chacun indépendamment, forment, avec l'atome d'azote auquel ils sont liés, un groupe pyrrolidino ou piperidino ;
R₈ est choisi parmi l'hydrogène, l'alkyle substitué par un atome d'halogène, un hydroxy, un alcoxy en C₁-C₆ ou un groupe amino, et un aryle éventuellement substitué, qui est éventuellement substitué par un atome d'halogène, un alkyle en C₁-C₆ linéaire ou ramifié, un hydroxy, un alcoxy en C₁-C₆ ou un groupe amino ;
R₉ est choisi parmi les groupes de formule (IIIa), (IIIb), (IIIc), (IIId) et (IIIe) 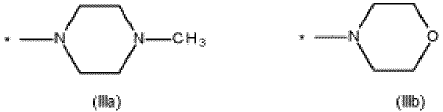 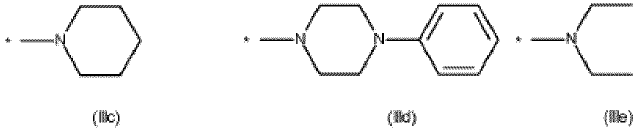
dans lequel l'astérisque (*) indique le lien de liaison ;
ainsi que les composés de formule (I) où
- R₃ est de l'acétamido et R₁, R₂, R₄, R₅, R₆, R₇ sont tous de l'hydrogène (composé 2l)
- R₃ est du mésylamido et R₁, R₂, R₄, R₅, R₆, R₇ sont tous de l'hydrogène (composé 2n)
et
R₃ est du carbométhoxy et R₁, R₂, R₄, R₅, R₆, R₇ sont tous de l'hydrogène (composé 2p) pour son utilisation dans le traitement et/ou la prévention des maladies neurologiques, comprenant la sclérose latérale amyotrophique (SLA), la maladie de Charcot-Marie, la maladie d'Alzheimer (MA), la maladie de Parkinson (MP).

9. Procédé de préparation d'un composé de formule (I) ou de l'un de ses sels ou solvates, selon l'une quelconque des revendications 1 à 5, comprenant la réaction d'un composé de formule (IV) 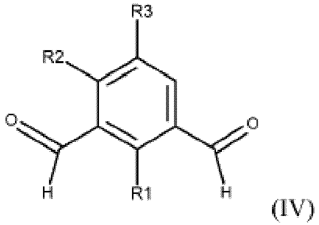 où R₁, R₂ et R₃ sont tels que définis ci-dessus, avec un composé de formule (V) 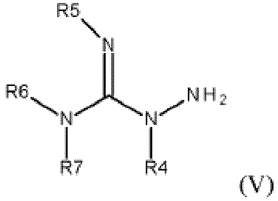 ou un de ses sels, où R₄, R₅, R₆ et R₃ sont tels que définis ci-dessus, dans un solvant approprié, et en isolant le composé de formule (I) ainsi obtenue, en la purifiant éventuellement et/ou en la convertissant éventuellement en un de ses sels ou de ses solvates.
